(19) Europäisches Patentamt
European Patent Office
Office européen des brevets

(11) Veröffentlichungsnummer: **0 173 957**
**B1**

(12) **EUROPÄISCHE PATENTSCHRIFT**

(45) Veröffentlichungstag der Patentschrift: **18.01.89**

(21) Anmeldenummer: **85110833.2**

(22) Anmeldetag: **19.08.85**

(51) Int. Cl.[4]: **C 07 D 239/42**, C 07 D 251/46, C 07 D 251/22, C 07 D 251/18, C 07 D 251/52, C 07 D 253/06, C 07 D 401/12, C 07 D 403/12, A 01 N 47/42

(54) **Sulfonyliso(thio)harnstoff- Derivate.**

(30) Priorität: **30.08.84 DE 3431921**
**17.05.85 DE 3517844**

(43) Veröffentlichungstag der Anmeldung:
**12.03.86 Patentblatt 86/11**

(45) Bekanntmachung des Hinweises auf die Patenterteilung:
**18.01.89 Patentblatt 89/3**

(84) Benannte Vertragsstaaten:
**AT BE DE FR GB IT NL SE**

(56) Entgegenhaltungen:
**EP-A- 0 151 554**
**EP-A- 1 170 014**
**DE-A- 3 243 533**
**US-A- 4 310 346**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Koeln 80 (DE)**
Erfinder: **Schmidt, Robert R. Dr., Im Waldwinkel 110, D-5060 Bergisch-Gladbach 2 (DE)**
Erfinder: **Eue, Ludwig, Dr., Paul-Klee-Strasse 36, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue Sulfonyliso(thio)-harnstoff-Derivate, ein erfinderisches Verfahren zu ihrer Herstellung sowie ihre Verwendung als Herbizide.

Verschiedene Isoharnstoffe bzw. Isothioharnstoffe sind als potentielle Herbizide bekannt geworden, haben jedoch bisher als Mittel zur Unkrautbekämpfung und/oder Regulierung des Pflanzenwachstums keine grössere Bedeutung erlangt (vergl. DE-AS 1 138 039 und GB-PS 1 202 736).

In EP-A-O 0 117 014 werden bestimmte S-Alkyl-N-arylsulfonyl-N'-heterocyclyl-isothioharnstoffe beschrieben, welche herbizide Eigenschaften besitzen.

Aus DE-A-3 243 533 sind bestimmte Aminosulfonylharnstoffe aus der Reihe 1-Diazinyl- oder 1-Triazinyl-3-aminosulfonylharnstoffe, 1-Diazinyl- oder 1-Triazinyl-2-alkyl-3-aminosulfonylisoharnstoffe und die entsprechenden Isothioharnstoffe sowie herbizide Zubereitungen, die solche Verbindungen enthalten, bekannt.

In US-A-4 310 346 werden weitere S-Alkyl- und S-Benzyl-N-phenylsulfonyl-N'-(diazinyl- und -triazinyl)-isothioharnstoffe mit herbiziden Eigenschaften beschrieben.

Eine weitere Gruppe von herbizid wirksamen, im Phenylring ortho-substituierter N-Phenylsulfonyl-N'-(diazinyl- und -triazinyl)-thioharnstoffe und -isoharnstoffe ist aus EP-A-0 151 554 bekannt.

Es wurden nun neue Sulfonyliso(thio)harnstoff-Derivate der allgemeinen Formel (I) gefunden,

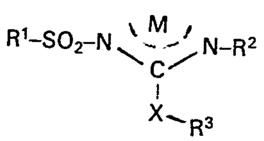

(I),

in welcher

$R^1$ für den Rest 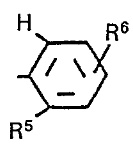 steht, worin

$R^5$ und $R^6$ gleich oder verschieden sind und für Wasserstoff, Halogen [wie insbesondere Fluor, Chlor, Brom und/oder Jod], Cyano, Nitro, $C_1$–$C_6$-Alkyl [welches gegebenenfalls durch Fluor, Chlor, Brom, Cyano, Carboxy, $C_1$–$C_4$-Alkoxycarbonyl, $C_1$–$C_4$-Alkylamino-carbonyl, Di-($C_1$–$C_4$-alkyl)-amino-carbonyl, Hydroxy, $C_1$–$C_4$-Alkoxy, Formyloxy, $C_1$–$C_4$-Alkyl-carbonyloxy, $C_1$–$C_4$-Alkoxy-carbonyloxy, $C_1$–$C_4$-Alkylamino-carbonyloxy, $C_1$–$C_4$-Alkylthio, $C_1$–$C_4$-Alkylsulfinyl, $C_1$–$C_4$-Alkylsulfonyl, Di-($C_1$–$C_4$-alkyl)-aminosulfonyl, $C_3$–$C_6$-Cycloalkyl oder Phenyl substituiert ist], für $C_2$–$C_6$-Alkenyl [welches gegebenenfalls durch Fluor, Chlor, Brom, Cyano, $C_1$–$C_4$-Alkoxycarbonyl, Carboxy oder Phenyl substituiert ist], für $C_2$–$C_6$-Alkinyl [welches gegebenenfalls durch Fluor, Chlor, Brom, Cyano, $C_1$–$C_4$-Alkoxy-carbonyl, Carboxy oder Phenyl substituiert ist], für $C_1$–$C_4$-Alkoxy [welches gegebenenfalls durch Fluor, Chlor, Brom, Cyano, Carboxy, $C_1$–$C_4$-Alkoxy-carbonyl, $C_1$–$C_4$-Alkoxy, $C_1$–$C_4$-Alkylthio, $C_1$–$C_4$-Alkylsulfinyl oder $C_1$–$C_4$-Alkylsulfonyl substituiert ist], für $C_1$–$C_4$-Alkylthio [welches gegebenenfalls durch Fluor, Chlor, Brom, Cyano, Carboxy, $C_1$–$C_4$-Alkoxycarbonyl, $C_1$–$C_4$-Alkylthio, $C_1$–$C_4$-Alkylsulfinyl oder $C_1$–$C_4$-Alkylsulfonyl substituiert ist], für $C_3$–$C_6$-Alkenyloxy [welches gegebenenfalls durch Fluor, Chlor, Brom, Cyano oder $C_1$–$C_4$-Alkoxy-carbonyl substituiert ist], für $C_2$–$C_6$-Alkenylthio [welches gegebenenfalls durch Fluor, Chlor, Brom, Cyano, Nitro, $C_1$–$C_3$-Alkylthio oder $C_1$–$C_4$-Alkoxycarbonyl substituiert ist], $C_3$–$C_6$-Alkinyloxy, $C_3$–$C_6$-Alkinylthio oder für den Rest $-S(O)_p-R^7$ stehen, wobei

p für die Zahlen 1 oder 2 steht und

$R^7$ für $C_1$–$C_4$-Alkyl [welches gegebenenfalls durch Fluor, Chlor, Brom, Cyano oder $C_1$–$C_4$-Alkoxy-carbonyl substituiert ist], $C_3$–$C_6$-Alkenyl, $C_3$–$C_6$-Alkinyl, $C_1$–$C_4$-Alkoxy, $C_1$–$C_4$-Alkoxy-amino, $C_1$–$C_4$-Alkoxy-$C_1$–$C_4$-alkylamino, $C_1$–$C_4$-Alkylamino oder Di-($C_1$–$C_4$-alkyl)-amino steht,

$R^5$ und $R^6$ weiterhin für Phenoxy, für $C_1$–$C_4$-Alkylcarbonylamino, $C_1$–$C_4$-Alkoxy-carbonylamino, $C_1$–$C_4$-Alkylamino-carbonyl-amino, Di-($C_1$–$C_4$-alkyl)-amino-carbonylamino oder für den Rest $-CO-R^8$ stehen, wobei

$R^8$ für $C_1$–$C_6$-Alkyl, $C_1$–$C_6$-Alkoxy, $C_3$–$C_6$-Cycloalkoxy, $C_3$–$C_6$-Alkenyloxy, $C_1$–$C_4$-Alkylthio, $C_1$–$C_4$-Alkylamino, $C_1$–$C_4$-Alkoxyamino, $C_1$–$C_4$-Alkoxy-$C_1$–$C_4$-alkyl-amino oder Di-($C_1$–$C_4$-alkyl)-amino steht [welche gegebenenfalls durch Fluor und/oder Chlor substituiert sind],

$R^5$ und $R^6$ weiterhin für $C_1$–$C_4$-Alkylsulfonyloxy, Di-($C_1$–$C_4$-alkyl)-aminosulfonylamino oder für den Rest $-CH=N-R^9$ stehen, wobei

$R^9$ für gegebenenfalls durch Fluor, Chlor, Cyano, Carboxy, $C_1$–$C_4$-Alkoxy, Carbonyl, $C_1$–$C_4$-Alkylthio, $C_1$–$C_4$-Alkylsulfinyl oder $C_1$–$C_4$-Alkylsulfonyl substituiertes $C_1$–$C_6$-Alkyl, für gegebenenfalls durch Fluor oder Chlor substituiertes Benzyl, für gegebenenfalls durch Fluor oder Chlor substituiertes $C_3$–$C_6$-Alkenyl oder $C_3$–$C_6$-Alkinyl, für gegebenenfalls durch Fluor, Chlor, Brom, $C_1$–$C_4$-Alkyl, $C_1$–$C_4$-Alkoxy, Trifluormethyl, Trifluormethoxy oder Trifluormethylthio substituiertes Phenyl, für gegebenenfalls durch Fluor und/oder Chlor substituiertes $C_1$–$C_6$-Alkoxy, $C_3$–$C_6$-Alkenoxy, $C_3$–$C_6$-Alkinoxy oder Benzyloxy, für Amino, $C_1$–$C_4$-Alkylamino, Di-($C_1$–$C_4$-alkyl)amino, Phenylamino, $C_1$–$C_4$-Alkyl-carbonyl-amino, $C_1$–$C_4$-Alkoxy-carbonylamino, $C_1$–$C_4$-Alkyl-sulfonylamino oder für gegebenenfalls durch Fluor, Chlor, Brom oder Methyl substituiertes Phenylsulfonylamino steht, worin weiter

$R^1$ für den Rest 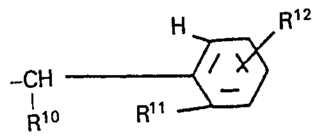 steht, worin

$R^{10}$ für Wasserstoff oder $C_1$–$C_4$-Alkyl steht,

$R^{11}$ und $R^{12}$ gleich oder verschieden sind und für Wasserstoff, Fluor, Chlor, Brom, Nitro, Cyano, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], Carboxy, $C_1$–$C_4$-Alkoxy-carbonyl, $C_1$–$C_4$-Alkylsulfonyl oder Di-($C_1$–$C_4$-alkyl)-aminosulfonyl stehen; worin weiter

$R^1$ für den Rest 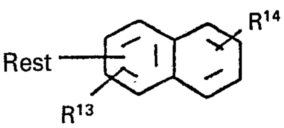 steht, worin

$R^{13}$ und $R^{14}$ gleich oder verschieden sind und für Wasserstoff, Fluor, Chlor, Brom, Nitro, Cyano, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist], stehen; worin weiter

$R^1$ für den Rest 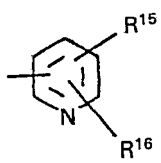 steht, worin

$R^{15}$ und $R^{16}$ gleich oder verschieden sind und für Wasserstoff, Fluor, Chlor, Brom, Nitro, Cyano, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], für $C_1$–$C_4$-Alkylthio, $C_1$–$C_4$-Alkylsulfinyl oder $C_1$–$C_4$-Alkylsulfonyl [welche gegebenenfalls durch Fluor und/oder Chlor substituiert sind], sowie für Di-($C_1$–$C_4$-alkyl)-aminosulfonyl oder $C_1$–$C_4$-Alkoxy-carbonyl stehen; worin weiter

$R^1$ für den Rest 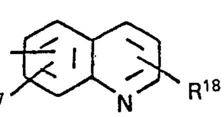 steht, worin

$R^{17}$ und $R^{18}$ gleich oder verschieden sind und für Wasserstoff, Fluor, Chlor, Brom, $C_1$–$C_4$-Alkyl [welches gegebenenfalls durch Fluor und/oder Brom substituiert ist], $C_1$–$C_4$-Alkoxy [welches gegebenenfalls durch Fluor und/oder Chlor substituiert ist], für $C_1$–$C_4$-Alkylthio, $C_1$–$C_4$-Alkylsulfinyl oder $C_1$–$C_4$-Alkylsulfonyl [welche gegebenenfalls durch Fluor und/oder Chlor substituiert sind], oder für Di-($C_1$–$C_4$-alkyl)-aminosulfonyl stehen; worin weiter

$R^1$ für den Rest 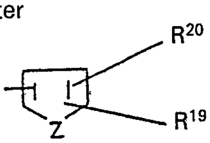 steht, worin

$R^{19}$ und $R^{20}$ gleich oder verschieden sind und für Wasserstoff, Fluor, Chlor, Brom, Cyano, Nitro, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylthio, $C_1$–$C_4$-Alkylsulfinyl oder $C_1$–$C_4$-Alkylsulfonyl [welches gegebenenfalls durch Fluor und/oder Chlor substituiert ist], Di-($C_1$–$C_4$-alkyl)-amino-sulfonyl oder $C_1$–$C_4$-Alkoxy-carbonyl stehen, und

Z für Sauerstoff, Schwefel oder die Gruppierung N–$Z^1$ steht, wobei

$Z^1$ für Wasserstoff, $C_1$–$C_4$-Alkyl [welches gegebenenfalls durch Fluor, Chlor, Brom oder Cyano substituiert ist], $C_3$–$C_6$-Cycloalkyl, Benzyl, Phenyl [welches gegebenenfalls durch Fluor, Chlor, Brom oder Nitro substituiert ist], $C_1$–$C_4$-Alkylcarbonyl, $C_1$–$C_4$-Alkoxy-carbonyl oder Di-($C_1$–$C_4$-alkyl)-aminocarbonyl steht; worin weiter

$R^1$ für den Rest 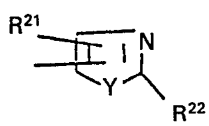 steht, worin

$R^{21}$ für Wasserstoff, $C_1$–$C_5$-Alkyl oder Halogen steht,

$R^{22}$ für Wasserstoff oder $C_1$–$C_5$-Alkyl steht und

Y für Schwefel oder die Gruppierung N–$R^{23}$ steht, wobei

$R^{23}$ für Wasserstoff oder $C_1$–$C_5$-Alkyl steht; worin weiter

$R^2$ für den Rest 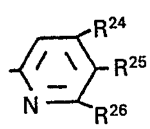 steht, worin

$R^{24}$ und $R^{26}$ gleich oder verschieden sind und für Wasserstoff, Fluor, Chlor, Brom, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist] stehen mit der Massgabe, dass wenigstens einer der Reste $R^{24}$ und $R^{26}$ von Wasserstoff verschieden ist, und

$R^{25}$ für Wasserstoff, Fluor, Chlor, Brom, Cyano oder $C_1$–$C_4$-Alkyl [welches gegebenenfalls durch Fluor und/oder Chlor substituiert ist] steht; worin weiter

$R^2$ für den Rest 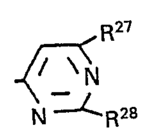 steht, worin

$R^{27}$ und $R^{28}$ gleich oder verschieden sind und für Wasserstoff, Fluor, Chlor, Brom, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylamino oder Di-($C_1$–$C_4$-

alkyl)-amino stehen mit der Massgabe, dass wenigstens einer der Reste $R^{27}$ und $R^{28}$ von Wasserstoff verschieden ist; worin weiter

$R^2$ für den Rest 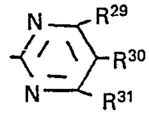 steht, worin

$R^{29}$ für Wasserstoff, Fluor, Chlor, Brom, Hydroxy, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist] steht,

$R^{30}$ für Wasserstoff, Fluor, Chlor, Brom, $C_1$–$C_4$-Alkyl [welches gegebenenfalls durch Fluor und/oder Chlor substituiert ist], Cyano, Formyl, $C_1$–$C_4$-Alkyl-carbonyl oder $C_1$–$C_4$-Alkoxycarbonyl steht und

$R^{31}$ für Wasserstoff, Fluor, Chlor, Brom, Hydroxy, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], Amino, $C_1$–$C_4$-Alkyl-amino oder Di-($C_1$–$C_4$-alkyl)-amino steht, oder

$R^{30}$ und $R^{31}$ gemeinsam für $C_3$–$C_4$-Alkandiyl stehen; worin weiter

$R^2$ für den Rest 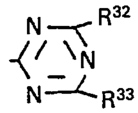 steht, worin

$R^{32}$ und $R^{33}$ gleich oder verschieden sind und für Fluor, Chlor, Brom, Hydroxy, $C_1$–$C_4$-Alkyl [welches gegebenenfalls durch Fluor und/oder Chlor substituiert ist], $C_3$–$C_5$-Cycloalkyl, $C_1$–$C_4$-Alkoxy [welches gegebenenfalls durch Fluor und/oder Chlor substituiert ist], $C_1$–$C_4$-Alkylthio oder für $C_1$–$C_4$-Alkylamino bzw. Di-($C_1$–$C_4$-alkyl)-amino stehen; worin weiter

$R^2$ für den Rest 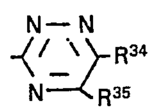 steht, worin

$R^{34}$ und $R^{35}$ gleich oder verschieden sind und für Wasserstoff, Methyl oder Methoxy stehen; worin weiter

$R^3$ für einen Phenylrest steht, welcher gegebenenfalls substituiert ist durch einen oder mehrere Reste aus der Reihe Halogen [wie insbesondere Fluor, Chlor, Brom und Iod], Cyano, Nitro, Hydroxy, Carboxy, $C_1$–$C_6$-Alkyl [welches gegebenenfalls durch Fluor, Chlor, Brom, Nitro, Cyano, Hydroxy, Carboxy, $C_1$–$C_4$-Alkoxy-carbonyl, $C_1$–$C_4$-Alkoxy, $C_1$–$C_4$-Alkylthio oder Phenyl substituiert ist], $C_3$–$C_6$-Cycloalkyl, $C_1$–$C_4$-Alkoxy [welches gegebenenfalls durch Fluor, Chlor, Brom, Cyano, Carboxy, $C_1$–$C_4$-Alkoxy, $C_1$–$C_4$-Alkylthio oder $C_1$–$C_4$-Alkoxy-carbonyl substituiert ist], $C_1$–$C_4$-Alkylthio [welches gegebenenfalls durch Fluor, Chlor, Brom, Cyano, Carboxy, $C_1$–$C_4$-Alkoxy-carbonyl substituiert ist], Amino, $C_1$–$C_4$-Alkyl-amino bzw. Di-($C_1$–$C_4$-alkyl)-amino [welche gegebenenfalls durch Fluor, Chlor, Brom, Cyano, Carboxy, $C_1$–$C_4$-Alkoxy oder $C_1$–$C_4$-Alkoxy-carbonyl substituiert sind], $C_1$–$C_4$-Alkyl-carbonylamino, $C_1$–$C_4$-Alkoxy-carbonylamino, (Di)-$C_1$–$C_4$-Alkylamino-carbonyl-amino, Formyl, $C_1$–$C_4$-Alkyl-carbonyl, Benzoyl, $C_1$–$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$–$C_4$-Alkyl-carbonyloxy, $C_1$–$C_4$-Alkoxy-carbonyloxy, $C_1$–$C_4$-Alkyl-amino-carbonyloxy und Di-($C_1$–$C_4$-alkyl)-amino-carbonyloxy, oder welcher gegebenenfalls durch einen Benzorest [welcher gegebenenfalls durch Fluor, Chlor, Brom, Cyano, Nitro, Methyl und/oder Trifluormethyl substituiert ist] anelliert ist; worin weiter

$R^3$ für einen fünf- oder sechsgliedrigen heteroaromatischen Ring steht, welcher 1 bis 3 Stickstoffatome und/oder ein Sauerstoff- oder Schwefelatom enthält und welcher gegebenenfalls benzanelliert ist und/oder durch Fluor, Chlor, Brom, Cyano, Nitro, $C_1$–$C_3$-Alkyl oder $C_1$–$C_3$-Alkoxy [wobei letztere gegebenenfalls durch Fluor und/oder Chlor substituiert sind] substituiert ist; worin weiter

X für Sauerstoff oder Schwefel steht und

M für Wasserstoff, ein Natrium-, Kalium-, Magnesium-, Calcium-, Aluminium-, Mangan-, Eisen-, Cobalt-, oder Nickel-Äquivalent steht, sowie Addukte von Verbindungen der Formel (I) mit Halogenwasserstoffsäuren, mit Schwefelsäure, mit gegebenenfalls durch Fluor und/oder Chlor substituierten Alkansulfonsäuren mit 1 bis 4 Kohlenstoffatomen oder mit Benzol- oder Naphthalinsulfonsäuren, welche gegebenenfalls durch Fluor, Chlor, Brom oder Methyl substituiert sind.

Die allgemeine Formel (I) steht – wenn M für Wasserstoff steht – für die einzelnen Tautomeren der Formel (Ia) und (Ib)

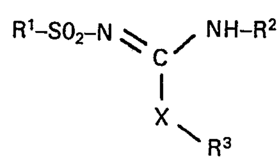
(Ia)

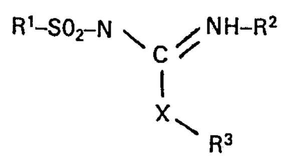
(Ib)

in welcher

$R^1$, $R^2$, $R^3$ und X die oben angegebenen Bedeutungen haben, sowie für Gemische der Tautomeren (Ia) und (Ib).

Das Mischungsverhältnis hängt von aggregationsbestimmenden Faktoren, wie z.B. Temperatur, Lösungsmittel und Konzentration ab.

Man erhält die neuen Sulfonyliso(thio)harnstoff-Derivate der Formel (I), nach einem erfinderischen Verfahren, indem man Sulfonylguanidin-Derivate der Formel (II)

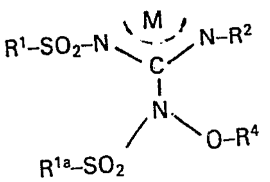

(II)

in welcher

$R^1$, $R^2$ und M die oben angegebenen Bedeutungen haben,

$R^{1a}$ die gleiche Bedeutung wie $R^1$ hat, aber nicht in jedem Einzelfall mit $R^1$ identisch sein muss, und

$R^4$ für $C_1$–$C_4$-Alkyl oder Benzyl steht, mit Verbindungen der Formel (III),

$M^1$–X–$R^3$ (III)

in welcher

X und $R^3$ die oben angegebenen Bedeutungen haben und

$M^1$ für Wasserstoff, Natrium oder Kalium steht, gegebenenfalls in Gegenwart von Basen und in Gegenwart von Verdünnungsmitteln umsetzt und gegebenenfalls die hierbei erhaltenen Produkte der Formel (I) mit Säuren behandelt.

Die neuen Sulfonyliso(thio)harnstoff-Derivate der Formel (I) und ihre Addukte mit starken Säuren zeichnen sich durch starke herbizide Wirksamkeit aus.

Überraschenderweise zeigen die neuen Verbindungen der Formel (I) erheblich bessere herbizide Wirkung als vorbekannte Isoharnstoff- bzw. Isothioharnstoff-Derivate gleicher Wirkungsrichtung.

Es ist weiter als überraschend anzusehen, dass die erfindungsgemässen Verbindungen der Formel (I) durch selektive Spaltung von Sulfonylguanidin-Derivaten der Formel (II) hergestellt werden können, da neben dieser neuartigen Reaktion auch andere Spaltungsreaktionen, beispielsweise durch Angriff an den Sulfonyl-Gruppierungen, zu erwarten gewesen wären.

Gegenstand der Erfindung sind vorzugsweise Verbindungen der Formel (I), in welcher

(A) $R^1$ für den Rest 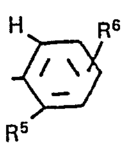 steht worin

$R^5$ für Fluor, Chlor, Brom, Methyl, Trifluormethyl, Methoxy, Difluormethoxy, Trifluormethoxy, $C_1$–$C_3$-Alkylthio, Difluormethylthio, Trifluormethylthio, $C_1$–$C_3$-Alkylsulfinyl, $C_1$–$C_3$-Alkylsulfonyl, Dimethylaminosulfonyl, Diethylaminosulfonyl, N-Methoxy-N-methylaminosulfonyl, Phenoxy, $C_1$–$C_3$-Alkoxy-carbonyl oder $C_1$–$C_3$-Alkyl-aminocarbonyl steht und

$R^6$ für Wasserstoff steht; worin weiter

$R^2$ für den Rest 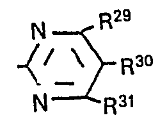 steht, worin

$R^{29}$ für Wasserstoff, Fluor, Chlor, Brom, Hydroxy, $C_1$–$C_3$-Alkyl, $C_1$–$C_3$-Alkoxy oder Difluormethoxy steht,

$R^{30}$ für Wasserstoff, Chlor, Brom oder Methyl steht und

$R^{31}$ für $C_1$–$C_3$-Alkyl, Hydroxy, Fluor, Chlor, Brom oder $C_1$–$C_3$-Alkoxy steht; worin weiter

$R^3$ für einen Phenylrest steht, welcher gegebenenfalls substituiert ist durch einen oder zwei Reste aus der Reihe Fluor, Chlor, Brom, Jod, Cyano, Nitro, Hydroxy, Carboxy, $C_1$–$C_3$-Alkoxy-carbonyl, $C_1$–$C_4$-Alkyl, Trifluormethyl, Hydroxymethyl, Methoxycarbonylmethyl, Phenyl-$C_1$–$C_3$-alkyl, Cyclohexyl, $C_1$–$C_3$-Alkoxy, Trifluormethoxy, $C_1$–$C_3$-Alkylthio, Trifluormethylthio, Dimethylamino, Amino, Acetylamino, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pyridoxy [welches gegebenenfalls durch Chlor und/oder Trifluormethyl substituiert ist], oder welcher gegebenenfalls benzanelliert ist; worin weiter

X für Sauerstoff oder Schwefel steht und

M für Wasserstoff, ein Natrium-, Kalium-, oder Calcium-äquivalent steht; worin weiter

(B) $R^1$, $R^3$, X und M die oben unter (A) angegebene Bedeutung haben und

$R^2$ für den Rest 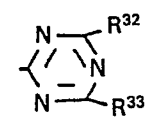 steht, worin

$R^{32}$ für Fluor, Chlor, Cyclopropyl, $C_1$–$C_2$-Alkyl, $C_1$–$C_2$-Alkoxy oder $C_1$–$C_2$-Alkylthio steht und

$R^{33}$ für Fluor, Chlor, Cyclopropyl, $C_1$–$C_2$-Alkyl, $C_1$–$C_2$-Alkoxy, $C_1$–$C_2$-Alkylamino oder Di-($C_1$–$C_2$-alkyl)-amino steht.

Verwendet man beim erfindungsgemässen Herstellungsverfahren beispielsweise N'-(4,6-Dimethyl-pyrimidin-2-yl)-N''-methoxy-N'',N'''-bis-(2-methoxycarbonylbenzolsulfonyl)-guanidin und Natriumphenolat als Ausgangsstoffe, so kann der Reaktionsablauf durch folgendes Formelschema skizziert werden:

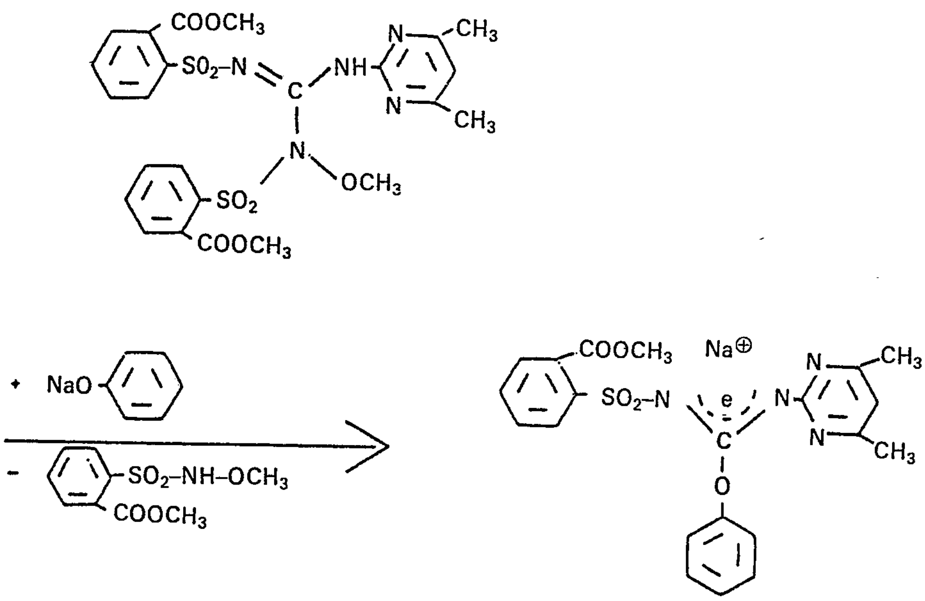

Die beim erfindungsgemässen Herstellungsverfahren als Ausgangsstoffe zu verwendenden Sulfonylguanidin-Derivate sind durch die Formel (II) allgemein definiert. In dieser Formel haben vorzugsweise die Reste

$R^1$, $R^2$ und M die gleichen Bedeutungen, wie sie oben im Rahmen der Definition der entsprechenden Reste in Formel (I) als bevorzugt genannt sind,

$R^{1a}$ hat vorzugsweise die gleiche Bedeutung, wie sie oben im Rahmen der Substituentendefinition in Formel (I) als bevorzugt für $R^1$ angegeben ist, und

$R^4$ steht vorzugsweise für Methyl.

Als Beispiele für die Ausgangsstoffe der Formel (II) seien genannt:
N'-(4,6-Dimethyl-pyrimidin-2-yl)-N''-methoxy-
-N'',N'''-bis-(2-chlor-benzolsulfonyl)-,
-N'',N'''-bis-(2-brom-benzolsulfonyl)-,
-N'',N'''-bis-(2-fluor-benzolsulfonyl)-,
-N'',N'''-bis-(2-methyl-benzolsulfonyl)-,
-N'',N'''-bis-(2-trifluormethyl-benzolsulfonyl)-,
-N'',N'''-bis-(2-methoxy-benzolsulfonyl)- und
-N'',N'''-bis-(2-methoxycarbonyl-benzolsulfonyl)-guanidin,

N'-(4-Methyl-pyrimidin-2-yl)-N''-methoxy-
-N'',N'''-bis-(2-chlor-benzolsulfonyl)-,
-N'',N'''-bis-(2-methoxycarbonyl-benzolsulfonyl)-,
-N'',N'''-bis-(2-difluormethoxy-benzolsulfonyl)-,
-N'',N'''-bis-(2-diethylaminosulfonyl-benzolsulfonyl)-,
-N'',N'''-bis-(2-brom-benzolsulfonyl)-,
-N'',N'''-bis-(2-trifluormethylthio-benzolsulfonyl-,
-N'',N'''-bis-(2-phenoxy-benzolsulfonyl)- und
-N'',N'''-bis-(2-methylsulfonyl-benzolsulfonyl)-guanidin,

N'-(4,6-Dimehtoxy-pyrimidin-2-yl)-N''-methoxy-
-N'',N'''-bis-(2-chlor-benzolsulfonyl)-,
-N'',N'''-bis-(2-methoxycarbonyl-benzolsulfonyl)-,
-N'',N'''-bis-(2-difluormethoxy-benzolsulfonyl)-,
-N'',N'''-bis-(2-trifluormethoxy-benzolsulfonyl)-,
-N'',N'''-bis-(2-dimethylaminosulfonyl-benzolsulfonyl)-,
-N'',N'''-bis-(2-methylthio-benzolsulfonyl)-,
-N'',N'''-bis-(2-ethoxycarbonyl-benzolsulfonyl)-,
-N'',N'''-bis-(2-cyclopropyloxycarbonyl-benzolsulfonyl)-,
-N'',N'''-bis-(2-trifluormethylsulfonyl-benzolsulfonyl)-,
-N'',N'''-bis-(2-nitro-benzolsulfonyl)- und
-N'',N'''-bis-(2-cyano-benzolsulfonyl-guanidin,

N'-(4-Methoxy-6-methyl-pyrimidin-2-yl)-N''-methoxy-
-N'',N'''-bis-(2-chlor-benzolsulfonyl)-,
-N'',N'''-bis-(2-methoxycarbonyl-benzolsulfonyl)-,
-N'',N'''-bis-(2-methylsulfonyl-benzolsulfonyl)-,
-N'',N'''-bis-(2-methylthio-benzolsulfonyl)-,
-N'',N'''-bis-(2-trifluormethoxy-benzolsulfonyl)-,
-N'',N'''-bis-(2-ethoxycarbonyl-benzolsulfonyl)-,
-N'',N'''-bis-(2-trifluormethyl-benzolsulfonyl)-,
-N'',N'''-bis-(2-isopropylthio-benzolsulfonyl)-,
-N'',N'''-bis-(2-N-methoxy-N-methylaminosulfonyl-benzolsulfonyl)-,
-N'',N'''-bis-(2-trifluormethylthio-benzolsulfonyl)-,
-N'',N'''-bis-(2,5-dichlor-benzolsulfonyl)- und
-N'',N'''-bis-(2-phenoxy-benzolsulfonyl)-guanidin,

N'-(4-Difluormethoxy-6-methyl-pyrimidin-2-yl)-N''-methoxy-
-N'',N'''-bis-(2-ethoxycarbonyl-benzolsulfonyl)-,
-N'',N'''-bis-(2-brom-benzolsulfonyl)-,
-N'',N'''-bis-(2-methylsulfonylmethyl-benzolsulfonyl)-,
-N'',N'''-bis-(2-trifluormethylthio-benzolsulfonyl)-,
-N'',N'''-bis-(2-methoxy-benzolsulfonyl)-,
-N'',N'''-bis-(2-methoxycarbonyl-benzolsulfonyl)- und
-N'',N'''-bis-(2-chlor-benzolsulfonyl)-guanidin,

N'-(4,6-Dimethyl-s-triazin-2-yl)-N''-methoxy-
-N'',N'''-bis-(2-methyl-benzolsulfonyl)-,
-N'',N'''-bis-(2-methoxycarbonyl-benzolsulfonyl)-,
-N'',N'''-bis-(2-methylthio-benzolsulfonyl)-,
-N'',N'''-bis-(2-trifluormethoxy-benzolsulfonyl)-,

-N'',N'''-bis-(2-dimethylaminosulfonyl-benzolsulfonyl)-,
-N'',N'''-bis-(2-chlor-benzolsulfonyl)-,
-N'',N'''-bis-(2-propoxycarbonyl-benzolsulfonyl)- und
-N'',N'''-bis-(2-difluormethylthio-benzolsulfonyl)-guanidin,

N'-(4-Methoxy-6-methyl-s-triazin-2-yl)-N''-methoxy-
-N'',N'''-bis-(2-chlor-benzolsulfonyl)-,
-N'',N'''-bis-(2-cyano-benzolsulfonyl)-,
-N'',N'''-bis-(2-methoxycarbonyl-benzolsulfonyl)-,
-N'',N'''-bis-(2-isopropylthio-benzolsulfonyl)-,
-N'',N'''-bis-(2-phenoxy-benzolsulfonyl)- und
-N'',N'''-bis-(2-trifluormethoxy-benzolsulfonyl)-guanidin,

N'-(4,6-Dimethoxy-s-triazin-2-yl)-N''-methoxy-
-N'',N'''-bis-(2-phenoxy-benzolsulfonyl)-,
-N'',N'''-bis-(2-chlor-benzolsulfonyl)-,
-N'',N'''-bis-(2-methoxycarbonyl-benzolsulfonyl)-,
-N'',N'''-bis-(2-trifluormethylthio-benzolsulfonyl)-,
-N'',N'''-bis-(2-cyano-benzolsulfonyl)- und
-N'',N'''-bis-(2-isopropoxy-benzolsulfonyl)-guanidin,

N'-(4-Ethyl-6-methoxy-s-triazin-2-yl)-N''-methoxy-
-N'',N'''-bis-(2-chlor-benzolsulfonyl)-,
-N'',N'''-bis-(2-methoxycarbonyl-benzolsulfonyl)-,
-N'',N'''-bis-(2-trifluormethyl-benzolsulfonyl)-,
-N'',N'''-bis-(2-difluormethylthio-benzolsulfonyl)-,
-N'',N'''-bis-(2-dimethylaminosulfonyl-benzolsulfonyl)- und
-N'',N'''-bis-(2-cyano-benzolsulfonyl)-guanidin,

N'-(4-Dimethylamino-6-methyl-s-triazin-2-yl)-N''-methoxy
-N'',N'''-bis-(2-chlor-benzolsulfonyl)-,
-N'',N'''-bis-(2-ethoxycarbonyl-benzolsulfonyl)-,
-N'',N'''-bis-(2-chlormethyl-benzolsulfonyl)-,
-N'',N'''-bis-(2-fluormethyl-benzolsulfonyl)-,
-N'',N'''-bis-(2-iod-benzolsulfonyl)-,
-N'',N'''-bis-(2-methylthiomethyl-benzolsulfonyl)- und
-N'',N'''-bis-(2-trifluormethoxy-benzolsulfonyl)-guanidin,

N'-(5,6-Dimethyl-1,2,4-triazin-3-yl)-N''-methoxy-
-N'',N'''-bis-(2-chlor-benzolsulfonyl)-,
-N'',N'''-bis-(2-difluormethoxy-benzolsulfonyl)-,
-N'',N'''-bis-(2-methylsulfonylmethyl-benzolsulfonyl)-,
-N'',N'''-bis-(2-methoxycarbonyl-benzolsulfonyl)- und
-N'',N'''-bis-(2-cyano-benzolsulfonyl)-guanidin,

N'-(5-Methyl-1,2,4-triazin-3-yl)-N''-methoxy-
-N'',N'''-bis-(2-chlor-benzolsulfonyl)-,
-N'',N'''-bis-(2-phenoxy-benzolsulfonyl)-,
-N'',N'''-bis-(2-propoxycarbonyl-benzolsulfonyl)-,
-N'',N'''-bis-(2-dimethylaminocarbonyl-benzolsulfonyl)-,
-N'',N'''-bis-(2-dimethylaminosulfonyl-benzolsulfonyl)-,
-N'',N'''-bis-(2-brom-benzolsulfonyl)- und
-N'',N'''-bis-(2-trifluormethylthio-benzolsulfonyl)-guanidin,

N'-(2,6-Dimethyl-pyrimidin-4-yl)-N''-methoxy-
-N'',N'''-bis-(2-fluor-benzolsulfonyl)-,
-N'',N'''-bis-(2-ethoxycarbonyl-benzolsulfonyl)-,
-N'',N'''-bis-(2-difluormethoxy-benzolsulfonyl)-,
-N'',N'''-bis-(2-fluormethyl-benzolsulfonyl)-,
-N'',N'''-bis-(2-cyclopropyloxycarbonyl-benzolsulfonyl)- und
-N'',N'''-bis-(2-phenoxy-benzolsulfonyl)-guanidin,

N'-(2,6-Dimethoxy-pyrimidin-4-yl)-N''-methoxy-
-N'',N'''-bis-(2-fluor-benzolsulfonyl)-,
-N'',N'''-bis-(2-methoxycarbonyl-benzolsulfonyl)-,
-N'',N'''-bis-(2-trifluormethoxy-benzolsulfonyl)-,
-N'',N'''-bis-(2-phenoxy-benzolsulfonyl)-,
-N'',N'''-bis-(2-methylthio-benzolsulfonyl)-,
-N'',N'''-bis-(2-dimethylaminocarbonyl-benzolsulfonyl)-,
-N'',N'''-bis-(2-cyano-benzolsulfonyl)- und
-N'',N'''-bis-(2-methoxymethyl-benzolsulfonyl)-guanidin.

Die als Ausgangsstoffe zu verwendenden Sulfonyl-guanidin-Derivate der Formel (II) sind Gegenstand einer vorgängigen Patentanmeldung (vergl. DE-OS 33 34 455; EP-A-121 082).

Man erhält die Verbindungen der Formel (II) – für den Fall, dass $R^1$ und $R^{1a}$ identisch sind – wenn man Guanidin-Derivate der Formel (IV),

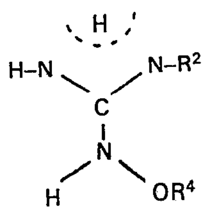

(IV)

in welcher

$R^2$ und $R^4$ die oben angegebenen Bedeutungen haben, mit wenigstens zwei Moläquivalenten von Sulfonsäurechloriden der Formel (V)

$$R^1\text{–}SO_2\text{–}Cl \quad (V)$$

in welcher

$R^1$ die oben angegebene Bedeutung hat, in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid oder Chloroform, bei Temperaturen zwischen −20 °C und +50 °C, vorzugsweise bei 0 °C bis 30 °C umsetzt. Die Aufarbeitung kann auf übliche Weise durchgeführt werden, beispielsweise durch Versetzen mit Wasser, gegebenenfalls Ansäuern z.B. mit Salzsäure, Extrahieren mit einem mit Wasser praktisch nicht mischbaren Lösungsmittel, wie z.B. Methylenchlorid oder Chloroform, Waschen der organischen Phase mit Wasser, Trocknen, Filtrieren und Einengen. Die dabei im Rückstand verbleibenden Produkte der Formel (II) können im allgemeinen mit organischen Lösungsmitteln zur Kristallisation gebracht und gegebenenfalls durch Umkristallisieren gereinigt werden. Die so erhaltenen Verbindungen der Formel (II), in welcher M für Wasserstoff steht, können auf übliche Weise durch Umsetzung mit Metallverbindungen in geeigneten Verdünnungsmitteln, z.B. mit Natriummethylat in Methanol, in entsprechende Salze der Formel (II) – M: Metalläquivalent – umgewandelt werden.

Die als Zwischenprodukte benötigten Guanidin-Derivate der Formel (IV) sind ebenfalls Gegenstand der oben genannten vorgängigen Patentanmeldung (vergl. DE-OS 3 334 455).

Man erhält diese Guanidin-Derivate, wenn man Cyanaminoverbindungen der Formel (VI)

$$NC-NH-R^2 \qquad (VI)$$

in welcher

$R^2$ die oben angegebene Bedeutung hat, mit Hydroxylamin-Derivaten der Formel (VII)

$$H_2N-OR^4 \qquad (VII)$$

in welcher

$R^4$ die oben angegebene Bedeutung hat, bzw. deren Hydrochloriden gegebenenfalls in Gegenwart von Verdünnungsmitteln, wie z.B. Ethanol oder Butanol, bei Temperaturen zwischen 20 °C und 150 °C, vorzugsweise zwischen 50 °C und 120 °C, umsetzt und gegebenenfalls anschliessend mit Säureakzeptoren, wie z.B. (wässr.) Ammoniak, Natriumhydroxid oder Kaliumcarbonat, behandelt. Die Guanidin-Derivate (IV) fallen hierbei im allgemeinen kristallin an.

Die Cyanaminoverbindungen der Formel (VI) sind zum Teil bekannt (vergl. J. Chem. Soc. 1953, 1725–1730). Man erhält diese Verbindungen im wesentlichen nach folgenden Synthesewegen:

(1) allgemein durch Umsetzung von Alkalimetall- oder Erdalkalimetall-Salzen von Cyanamid – wie z.B. Natriumcyanamid oder Calciumcyanamid – mit Halogenverbindungen der Formel (VIII)

$$Hal^1-R^2 \qquad (VIII)$$

in welcher

$R^2$ die oben angegebene Bedeutung hat und

$Hal^1$ für Fluor, Chlor, Brom oder Iod, insbesondere für Chlor, steht, gegebenenfalls in Gegenwart von inerten Verdünnungsmitteln, wie z.B. Aceton, Acetonitril oder Dimethylformamid, bei Temperaturen zwischen 0 °C und 150 °C, vorzugsweise zwischen 10 °C und 100 °C; nach Abdestillieren der flüchtigen Komponente und Auflösen des Rückstandes in Wasser können die Cyanaminoverbindungen der Formel (VI) durch Ansäuern, z.B. mit Salzsäure, ausgefällt und durch Absaugen isoliert werden; oder

(2) für den Fall, dass $R^2$ für einen substituierten Pyrimidinylrest steht, durch Umsetzung von Cyanoguanidin ('Dicyandiamid') mit β-Dicarbonylverbindungen oder gegebenenfalls Acetalen oder Enolethern hiervon, wie z.B. Acetylaceton (vergl. J. Chem. Soc. 1953, 1725–1730), Acetessigsäureester (vergl. J. Prakt. Chem. 77 (1908), 542 und J. Chem. Soc. 1948, 586) oder Malonsäureester (vergl. DE-PS 158 591). Die aus Acetessigsäureestern bzw. Malonsäureestern erhaltenen 2-Cyan-amino-4-hydroxy-6-methyl- bzw. -4,6-dihydroxy-pyrimidine können auf bekannte Weise durch Umsetzung mit Alkylierungsmitteln, wie z.B. Dimethyl- oder Diethylsulfat, gegebenenfalls in Gegenwart von Verdünnungsmitteln, wie z.B. Wasser, Methanol, Ethanol, n- und iso-Propanol, Aceton, Dioxan oder Dimethylformamid, und in Gegenwart von Säurebindemitteln, wie z.B. Natrium- oder Kaliumhydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z.B. Acetanhydrid oder Acetylchlorid acyliert und nach der Alkylierung wird mit wässrigen Säuren oder Basen wieder entacyliert.

In einem weiteren Alternativverfahren erhält man die Cyanaminoverbindungen der Formel (VI), wenn man

(3) Aminoverbindungen der Formel (IX)

$$H_2N-R^2 \qquad (IX)$$

in welcher

$R^2$ die oben angegebene Bedeutung hat, mit Carbonylisothiocyanaten der Formel (X)

$$R^{36}-\overset{\displaystyle O}{\overset{\|}{C}}-N=C=S \qquad (X)$$

in welcher

$R^{36}$ für Ethoxy oder Phenyl steht, gegebenenfalls in Gegenwart eines inerten Verdünnungsmittels, wie z.B. Aceton, Acetonitril oder Toluol, bei Temperaturen zwischen 0 °C und 100 °C umsetzt, die hierbei gebildeten Carbonylthioharnstoffe der Formel (XI)

$$R^{36}-\overset{\displaystyle O}{\overset{\|}{C}}-NH-\overset{\displaystyle S}{\overset{\|}{C}}-NH-R^2 \qquad (XI)$$

in welcher

$R^{36}$ und $R^2$ die oben angegebenen Bedeutungen haben, gegebenenfalls nach Einengen durch Absaugen isoliert und mit wässrigen Alkalimetall- oder Erdalkalimetall-hydroxidlösungen, wie z.B. Natronlauge, gegebenenfalls in Gegenwart eines organischen Lösungsmittels, wie z.B. Tetrahydrofuran oder Dioxan, bei Temperaturen zwischen 0 °C und 120 °C umsetzt und die nach Ansäuern, z.B. mit Salzsäure, kristallin erhaltenen Thioharnstoffe der Formel (XII)

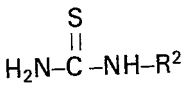

(XII)

in welcher

$R^2$ die oben angegebene Bedeutung hat, durch Absaugen isoliert und mit Metallverbindungen, welche Schwefelwasserstoff binden können, wie z.B. mit Blei(II)-acetat, Kupfer(II)-acetat, Quecksilber(II)-acetat oder Eisen(II)-acetat, in Gegenwart von wässrigen Alkalimetall- oder Erdalkalimetallhydroxidlösungen, wie z.B. Natronlauge, bei Temperaturen zwischen 20 °C und 100 °C umsetzt, nach Ende der Umsetzung filtriert und das Filtrat mit einer Säure, wie z.B. Essigsäure, ansäuert. Die hierbei kristallin anfallenden Produkte der Formel (VI) können durch Absaugen isoliert werden.

Die Ausgangsstoffe für die vorausgehend unter (1), (2) und (3) beschriebenen Herstellungsverfahren für die Cyanaminoverbindungen der Formel (VI) sind bekannt und/oder können nach an sich bekannten Verfahren hergestellt werden.

Hierzu gehören die Halogenverbindungen der Formel (VIII) (vergl. J. Chem. Soc. (C) 1966, 2031; Chem. Pharm. Bull. 11 (1963), 1382–1388; Arch. Pharm. 295 (1962), 649–657), die Aminoverbindungen der Formel (IX) (vergl. Chem. Pharm. Bull. 11 (1963), 1382–1388; J. Chem. Soc. 1946, 81 und US-PS 4 299 960) sowie die Carbonylisothiocyanate der Formel (X) (vergl. J. Heterocycl. Chem. 5 (1968), 837 und US-PS 4 160 037).

Die Hydroxylamin-Derivate der Formel (VII) sind bekannt oder können nach an sich bekannten Verfahren hergestellt werden (vergl. Chem. Pharm. Bull. 15 (1967), 345–349; Bull. Soc. Chim. France 1958, 664; Synthesis 1976, 682).

Die Sulfonsäurechloride der Formel (V) sind zum Teil bekannt (vergl. Chemistry Lett. 1978, 951; EP-PA 23 422, 35 893, 42 731, 44 808, 44 809, 51 466, 64 804 und 70 041; US-PS 2 929 820, 4 282 242 und 4 372 778; J. Org. Chem. 33 (1968), 2104; J. General Chem. 39 (1969), 2011).

Man erhält diese Verbindungen im wesentlichen nach folgenden zwei Synthesewegen:

(1) durch Umsetzung der entsprechenden Sulfonsäuren $R^1SO_3H$ bzw. von deren Alkalimetall- oder Erdalkalimetall-Salzen mit Halogenierungsmitteln, wie z.B. Phosphor(V)chlorid (Phosphorpentachlorid), Phosphorylchlorid (Phosphoroxychlorid), Thionylchlorid, Phosgen oder Benzotrichlorid, gegebenenfalls in Gegenwart von Katalysatoren, wie z.B. Pyridin oder Dimethylformamid, und gegebenenfalls unter Verwendung von inerten Verdünnungsmitteln, wie z.B. Methylenchlorid, Chloroform, Acetonitril, Chlorbenzol und/oder Sulfolan bei Temperaturen zwischen −20 °C und +150 °C, vorzugsweise zwischen 0 °C und +100 °C; nach Verdünnen mit Wasser können die Sulfonsäurechloride – soweit sie kristallin anfallen – durch Absaugen isoliert werden oder durch Extrahieren mit einem mit Wasser nicht mischbaren Lösungsmittel, wie z.B. Methylenchlorid, Diethylether oder Hexan, Waschen und Trocknen der Extrakte, Einengen und Umkristallisieren bzw. Destillieren gereinigt werden; oder auch

(2) auf an sich bekannte Weise (vergl. J. Org. Chem. 25(1960), 1824; DE-OS 2 308 262 und EP-PA 59 241) durch Umsetzung entsprechender Aminoverbindungen $R^1-NH_2$ mit Natriumnitrit und Salzsäure, gegebenenfalls in Gegenwart von Essigsäure, bei Temperaturen zwischen −10 °C und +20 °C, vorzugsweise zwischen −5 °C und +10 °C, und anschliessend (in situ) mit Schwefeldioxid oder einem Salz der schwefeligen Säure, wie z.B. Natriumsulfit oder Natriumhydrogensulfit in Gegenwart einer Kupferverbindung, wie z.B. Kupferchlorid oder Kupfersulfat, als Katalysator bei Temperaturen zwischen 0 °C und 80 °C, vorzugsweise zwischen 10 °C und 60 °C.

Die Aufarbeitung kann auf übliche Weise erfolgen: Bei Verdünnen mit Wasser fallen die Sulfonsäurechloride im allgemeinen kristallin an und können durch Absaugen isoliert werden. Sie können aber auch aus der wässrigen Dispersion mit einem mit Wasser praktisch nicht mischbaren Lösungsmittel, wie z.B. Methylenchlorid oder Diethylether, extrahiert, getrocknet und durch Vakuumdestillation gereinigt werden.

Man erhält die Ausgangsstoffe der Formel (II) auch, wenn man Monosulfonylguanidine der Formel (XIII)

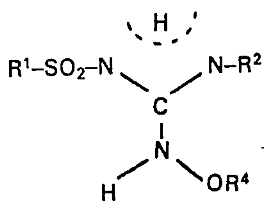

(XIII)

in welcher

$R^1$, $R^2$ und $R^4$ die oben angegebenen Bedeutungen haben, mit Sulfonsäurechloriden der Formel (XIV)

$R^{1a}-SO_2-Cl$ (XIV)

in welcher

$R^{1a}$ die oben angegebene Bedeutung hat, in Gegenwart von Säureakzeptoren, wie z.B. Pyridin oder Diazabicylooctan (DABCO), und gegebenenfalls in Gegenwart von Verdünnungsmitteln, wie z.B. Methylenchlorid oder Chloroform, bei Temperaturen zwischen −20 °C und +50 °C, vorzugsweise bei 0 °C bis +30 °C umsetzt. Die Aufarbeitung und gegebenenfalls die Umwandlung in Metallverbindungen kann wie oben beschrieben durchgeführt werden.

Die gegebenenfalls als Ausgangsstoffe zu verwendenden Monosulfonylguanidine der Formel (XIII) können nach folgenden Verfahren hergestellt werden:

(a) Durch Umsetzung von Guanidin-Derivaten der Formel (IV) – oben – mit angenähert stöchiometrischen Mengen von Sulfonsäurechloriden der Formel (V) – oben – in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid, Chloroform oder Tetrahydrofuran, bei Temperaturen zwischen −20 °C und +50 °C, vorzugsweise bei 0 °C bis 30 °C. Die Aufarbeitung kann wie oben für die Verbindungen der Formel (II) angegeben durchgeführt werden.

(b) Durch Umsetzung von Sulfonylguanidin-Derivaten der Formel (II) – oben – mit Hydroxylamin-Derivaten der Formel (VII) – oben – oder mit deren Hydrochloriden, gegebenenfalls in Gegenwart von Säureakzeptoren, wie z.B. Triethylamin, und gegebenenfalls in Gegenwart von Verdünnungsmitteln, wie z.B. Ethanol, bei Temperaturen zwischen 0 °C und 150 °C, vorzugsweise zwischen 10 °C und 100 °C. Die Verbindungen der Formel (XIII) fallen hierbei im allgemeinen kristallin an.

(c) Durch Umsetzung von Sulfonylisothioharnstoff-Derivaten der Formel (XV)

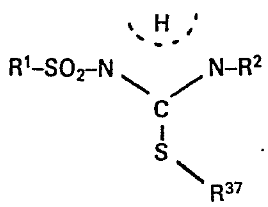

(XV)

in welcher

$R^1$ und $R^2$ die oben angegebenen Bedeutungen haben und

$R^{37}$ für $C_1$–$C_4$-Alkyl oder Benzyl steht, mit Hydroxylamin-Derivaten der Formel (VII) – oben – oder mit deren Hydrochloriden, gegebenenfalls in Gegenwart von Säureakzeptoren, wie z.B. Triethylamin, und gegebenenfalls in Gegenwart von Verdünnungsmitteln, wie z.B. Dioxan, bei Temperaturen zwischen 0 °C und 150 °C, vorzugsweise zwischen 20 °C und 120 °C. Die Verbindungen der Formel (XIII) fallen dabei im allgemeinen kristallin an.

(d) für den Fall, dass $R^1$ für den Rest 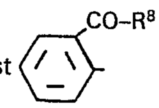

steht, worin

$R^8$ für $C_1$–$C_6$-Alkoxy, $C_3$–$C_6$-Cycloalkoxy, $C_3$–$C_6$-Alkenyloxy, $C_1$–$C_4$-Alkylthio, $C_1$–$C_4$-Alkylamino, $C_1$–$C_4$-Alkoxyamino, $C_1$–$C_4$-Alkoxy-$C_1$–$C_4$-alkylamino oder Di-($C_1$–$C_4$-alkyl)-amino steht, durch Umsetzung von Benzolactamsultamen der Formel (XVI)

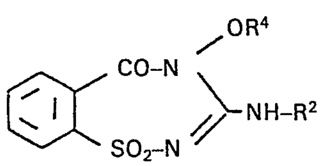

(XVI)

in welcher

$R^2$ und $R^4$ die oben angegebenen Bedeutungen haben, mit Verbindungen der Formel (XVII)

$M^1$–$R^8$ (XVII)

in welcher

$R^8$ die oben angegebene Bedeutung hat und

$M^1$ für Wasserstoff, Natrium oder Kalium steht, gegebenenfalls in Gegenwart von Verdünnungsmitteln, wie z.B. Methanol oder Ethanol, bei Temperaturen zwischen 0 °C und 150 °C, vorzugsweise zwischen 20 °C und 100 °C. Die Verbindungen der Formel (XIII) fallen dabei im allgemeinen kristallin an.

(e) Für den Fall, dass $R^1$ für den Rest

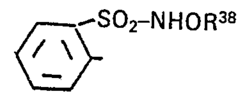

steht, worin

$R^{38}$ für $C_1$–$C_4$-Alkyl steht, durch Umsetzung von Benzodisultamen der Formel (XVIII)

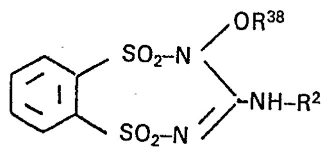

(XVIII)

in welcher

$R^2$ und $R^{38}$ die oben angegebenen Bedeutungen haben, mit Verbindungen der Formel (VII),

$H_2N$–$OR^4$ (VII)

in welcher

$R^4$ die oben angegebene Bedeutung hat, bzw. deren Hydrochloriden, gegebenenfalls in Gegenwart von Verdünnungsmitteln, wie z.B. Methanol oder Ethanol, bei Temperaturen zwischen 0 °C und 150 °C, vorzugsweise zwischen 20 °C und 100 °C. Die Verbindungen der Formel (XIII) fallen dabei im allgemeinen kristallin an.

Die oben als Ausgangsstoffe angegebenen Sulfonylisothioharnstoff-Derivate der Formel (XV) sind bekannt und/oder können nach an sich bekannten Verfahren hergestellt werden (vergl. EP-A 5 986).

Die weiterhin als Ausgangsstoffe angegebenen Benzolactame der Formel (XVI) sind bisher nicht aus der Literatur bekannt. Verschiedene Benzolactamsultame sind Gegenstand von nicht zum vorveröffentlichten Stand der Technik gehörenden Patentanmeldungen der Anmelderin (vergl. DE-OS 3 431 915, 3 431 920 und 3 431 927). Man erhält die Benzolactamsultame der Formel (XVI) durch Umsetzung von Guanidin-Derivaten der Formel (IV) – oben – mit 2-Chlorsulfonylbenzoylchlorid in Gegenwart von Säureakzeptoren, wie z.B. Pyridin, und gegebenenfalls in Gegenwart von Verdünnungsmitteln, wie z.B. Methylenchlorid, bei Temperaturen zwischen −30 °C und +50 °C, vorzugsweise zwischen −10 °C und +30 °C. Die Aufarbeitung kann wie oben für die Verbindungen der Formel (II) angegeben durchgeführt werden.

2-Chlorsulfonyl-benzoylchlorid (vergl. DE-OS 2 036 171) und die Verbindungen der Formel (XVII) sind bekannt.

Die oben weiter als Ausgangsstoffe angegebenen Benzodisultame der Formel (XVIII) sind bisher nicht aus der Literatur bekannt. Verschiedene Benzodisultame sind Gegenstand von nicht zum vorveröffentlichten Stand der Technik gehörenden Patentanmeldungen der Anmelderin (vergl. DE-OS 3 431 918, 3 431 922 und 3 431 929). Man erhält die Benzodisultame der Formel (XVIII) durch Umsetzung von Guanidin-Derivaten der Formel (IV) mit Benzol-1,2-disulfonsäure-dichlorid der Formel (XIX)

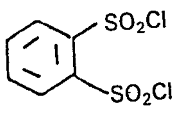

(XIX)

in Gegenwart von Säureakzeptoren, wie z.B. Pyridin, und gegebenenfalls in Gegenwart von Verdünnungsmitteln, wie z.B. Methylenchlorid, bei Temperaturen zwischen −80 °C und +100 °C, vorzugsweise zwischen −40 °C und +50 °C. Die Aufarbeitung kann wie oben für die Verbindungen der Formel (II) angegeben, durchgeführt werden.

Benzol-1,2-disulfonsäure-dichlorid der Formel (XIX) ist bekannt (vergl. J. Org. Chem. 31 (1966), S. 3289 ff).

Die beim erfindungsgemässen Herstellungsverfahren weiter als Ausgangsstoffe zu verwendenden Verbindungen sind durch die Formel (III) allgemein definiert. In dieser Formel haben vorzugsweise

X und $R^3$ die gleichen Bedeutungen, wie sie im Rahmen der Definition von X und $R^3$ in Formel (I) vorzugsweise genannt sind und

$M^1$ steht für Wasserstoff, Natrium oder Kalium.

Als Beispiele für die Ausgangsstoffe der Formel (III) seien genannt:

Phenol, 2-, 3- und 4-Fluor-phenol, 2-, 3- und 4-Chlorphenol, 2-, 3- und 4-Brom-phenol, 4-Jod-phenol, 2,3-Dichlor-phenol, 2,4-Dichlorphenol, 2,5-Dichlor-phenol, 4-Cyano-phenol, 2-, 3- und 4-Nitro-phenol, 4-Chlor-3-nitro-phenol, 3-Chlor-4-nitro-phenol, Brenzkatechin (1,2-Dihydroxy-benzol), Resorcin (1,3-Dihydroxy-benzol), Hydrochinon (1,4-Dihydroxybenzol), 2-, 3- und 4-Hydroxy-benzoesäure, 2-, 3- und 4-Methyl-phenol, 3,4-Dimethyl-phenol, 3,5-Dimethyl-phenol, 4-Isopropyl-phenol, 4-tert.-Butyl-phenol, 4-Chlor-3,5-dimethyl-phenol, 4-Hydroxy-benzylalkohol, 3-Methyl-4-nitro-phenol, 4-Chlor-3-methyl-phenol, 3- und 4-Trifluormethyl-phenol, 4-Hydroxy-phenylessigsäure-methylester, 4-(1-Methyl- 1-phenylethyl)-phenol, 4-Cyclohexyl-phenol, 3- und 4-Hydroxy-benzoesäure-methylester und -ethylester, 2-, 3- und 4-Methoxy-phenol, 4-Trifluormethoxy-phenol, 4-Methylthio-phenol, 3-Methyl-4-methylthio-phenol, 4-Trifluormethylthiophenol, 3-Dimethylamino-phenol, 3-Methyl-4-dimethylamino-phenol, 4-Methyl- 3-dimethylamino-phenol, 4-Acetylamino-phenol, 3-Hydroxy- und 4-Hydroxy-benzaldehyd, 4-Hydroxyacetophenon, 4-Hydroxy-benzophenon, 4-Hydroxy-biphenyl, 4,4′-Bis-hydroxy-biphenyl, 1-Naphthol, 2-Naphthol, 3-Hydroxy-biphenyl, 4-Hydroxyazobenzol, 4-Hydroxy-diphenylamin, 3-Hydroxy-diphenylamin, 3-Phenoxy-phenol, 4-Phenoxy-phenol, 4-(2,4-Dichlor-phenoxy)-phenol, 4-(4-Trifluormethyl-phenoxy)-phenol, 4-(3,5-Dichlor-2-pyridoxy)-phenol, 4-(3-Chlor-5-trifluormethyl-2-pyridoxy)-phenol, 3-Hydroxy-pyridin, 3-Hydroxy-6-methyl-pyridin, 8-Hydroxy-chinolin, Thiophenol, 4-Chlor-thiophenol, 4-Methyl-thiophenol, 2-Amino-thiophenol, 3-Amino-thiophenol, 4-Amino-thiophenol, 2-Methoxy-thiophenol, 3-Methoxy-thiophenol, 4-Methoxy-thiophenol, 2-Mercapto-benzoesäure und 4-Hydroxy-thiophenol, sowie die Natrium- und Kalium-Salze dieser Verbindungen.

Die Ausgangsstoffe der Formel (III) sind bekannte, weitgehend handelsübliche Produkte.

Das erfindungsgemässe Herstellungsverfahren wird vorzugsweise unter Verwendung von Verdünnungsmitteln durchgeführt. Als solche kommen praktisch alle inerten organischen Solventien und gegebenenfalls in Mischung damit auch Wasser in Betracht. Besonders geeignet sind Alkohole, wie z.B. Methanol, Ethanol, n- und i-Propanol, n-, i-, sec.- und tert.-Butanol, Ketone, wie z.B. Aceton, Methylethylketon und Methylisobutylketon, ferner Acetonitril, Dimethylformamid und Dimethylsulfoxid.

Das erfindungsgemässe Verfahren wird gegebenenfalls unter Verwendung von Basen durchgeführt. Als solche kommen praktisch alle üblicherweise verwendeten Säurebindemittel in Betracht. Hierzu gehören insbesondere Metall-hydroxide, -carbonate, -hydride und -alkoholate, wie z.B. Natrium-, Kalium-, und Calcium-hydroxid, Kaliumcarbonat, Natrium- und Calcium-hydrid, Natrium-methylat, -ethylat und -propylat, Kalium-methylat, -ethylat und tert.-Butylat sowie Aluminiumisopropylat.

Die Reaktionstemperaturen können beim erfindungsgemässen Verfahren innerhalb eines grösseren Bereichs variiert werden. Im allgemeinen arbeitet man bei Temperaturen zwischen 0 °C und 150 °C, vorzugsweise zwischen 10 °C und 100 °C. Das erfindungsgemässe Verfahren wird im allgemeinen bei Normaldruck durchgeführt.

Zur Durchführung des erfindungsgemässen Verfahrens setzt man je Mol Sulfonylguanidin-Derivat der Formel (II) im allgemeinen zwischen 1 Mol und 3 Mol, vorzugsweise zwischen 1,1 und 2,0 Mol Hydroxy- bzw. Mercapto-verbindung oder Salze hiervon der Formel (III) ein. Die Reaktionskomponenten werden gewöhnlich bei Raumtemperatur oder unter Aussenkühlung zusammengegeben und das Reaktionsgemisch wird, gegebenenfalls bei erhöhter Temperatur, bis zum Reaktionsende gerührt.

Die Aufarbeitung und Isolierung der neuen Verbindungen erfolgt nach üblichen Methoden:

Soweit die Produkte der Formel (I) als Metallsalze anfallen, kristallisieren sie im allgemeinen

aus der Reaktionslösung und können direkt durch Absaugen isoliert werden; andernfalls können sie nach Einengen durch Digerieren mit geeigneten organischen Lösungsmitteln, wie z.B. Ethanol oder Aceton, zur Kristallisation gebracht werden. Aus den salzartigen Produkten der Formel (I) können die entsprechenden salzfreien Verbindungen im allgemeinen durch Dispergieren in Wasser und Ansäuern, z.B. mit Essigsäure, erhalten werden. Alternativ können die salzfreien Produkte der Formel (I) nach Einengen der Reaktionsmischung, gegebenenfalls Ansäuern des Rückstandes, z.B. mit Salzsäure, Schütteln mit Wasser und einem damit praktisch nicht mischbaren organischen Lösungsmittel, wie z.B. Methylenchlorid oder Chloroform, Abtrennen der organischen Phase, Trocknen, Filtrieren und Einengen erhalten werden und mit geeigneten organischen Lösungsmitteln, wie z.B. Toluol oder Ethanol, zur Kristallisation gebracht werden.

Die erfindungsgemässen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dikotyle Kulturen der Gattungen:* Gossypium, Glycine, Beta, Daucus, Phaseolus, Pisum, Solanun, Linum, Ipomoea, Vicia, Nicotiana, Lycopersicon, Arachis, Brassica, Lactuca, Cucumis, Cucurbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

*Monikotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemässen Wirkstoffe können zur selektiven Bekämpfung mono- und dikotyler Unkräuter in mono- und dikotylen Kulturen wie z.B. Soja, Baumwolle, Reis, Getreide und Mais eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweisshydrolysate;

als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch als Mischungen mit bekannten Herbiziden zur Unkrautbekämpfung Verwendung finden, wobei Fertigformulierung oder Tankmischung möglich ist.

Die erfindungsgemässen Wirkstoffe können alleine und in Kombination mit anderen Wirkstoffen ausgebracht werden. Als Mischpartner kommen in Frage:

Harnstoffe (z.B. Methabenzthiazuron, Isoproturon, Chlortoluron und Fluometuron), Triazine (z.B. Atrazin), Triazinone (z.B. Metribuzin, Metamitron und 4-Amino-6-tert.-butyl-3-ethylthio-1,2,4-triazin-5(4H)-on), Triazindione (z.B. Ametridione), Chloracetamide (z.B. Alachlor und Metolachlor), Cyclohexandione (z.B. Sethoxydim), Heteroaryloxy- oder Aryloxyphenoxypropionsäuren (z.B. (2R)-2-[4-(3,5-Dichlor-pyridyl-2-oxy)-phenoxy]- propionsäure-2-(benzyloxy)-ethylester und (2R)-2-[4-(3,5-Dichlorpyridyl-2-oxy)-phenoxy]- propionsäure-(tri-methylsilyl)-methylester, Halogenalkancarbonsäuren (z.B. 2,4-D, 2,4-DP, MCPA und MCPP), Diphenylether (z.B. Fomesafen und Aclonifen), ferner Bentazon und Pyridazine (z.B. Pyridate).

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem grösseren Bereich schwanken. Sie hängt im wesentlichen von der Art des gewünschten Effekts ab. Im allgemeinen liegen die Aufwandmengen zwischen 0,001 und 15 kg Wirkstoff pro Hektar Bodenfläche, vorzugsweise zwischen 0,01 und 10 kg pro ha.

Die erfindungsgemässen Wirkstoffe (I) zeigen auch eine fungizide Wirksamkeit, z.B. eine Wirkung gegen Pyricularia oryzae am Reis und gegen Schorfpilze, wie z.B. Apfelschorf.

Die Herstellung und die Verwendung der erfindungsgemässen Wirkstoffe geht aus den nachfolgenden Beispielen hervor.

Herstellungsbeispiele

Beispiel 1:

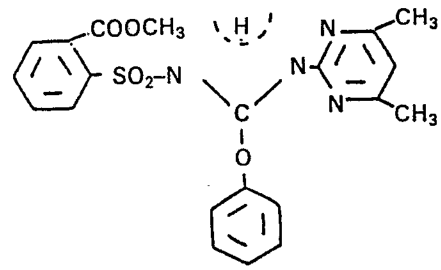

Eine Mischung aus 5,9 g (0,01 Mol) N'-(4,6-Dimethylpyrimidin-2-yl)- N''-methoxy-N'', N'''-bis- (2-methoxycarbonylbenzolsulfonyl)- guanidin, 2,4 g (0,02 Mol) Natriumphenolat, 30 ml Ethanol und 5 ml Wasser wird 10 Stunden bei 50 °C gerührt. Anschliessend wird im Wasserstrahlvakuum zur Trockne eingeengt, der Rückstand mit Ethanol digeriert und abgesaugt. Das kristalline Produkt wird aus wässriger Essigsäure umkristallisiert.

Man erhält 1,5 g (34% der Theorie) N'-(4,6-Dimethylpyrimidin-2-yl)- N''-(2-methoxycarbonyl-benzolsulfonyl)-O-phenylisoharnstoff vom Schmelzpunkt 120 °C.

Beispiel 2:

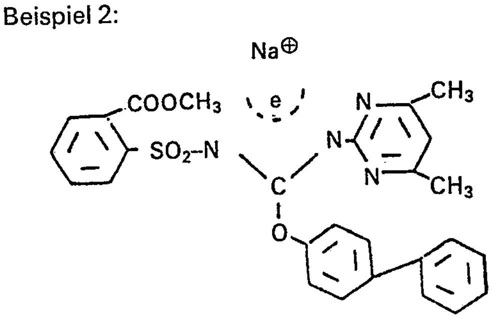

Eine Mischung aus 3,5 g (0,02 Mol) 4-Hydroxy-biphenyl, 1,1 g (0,02 Mol) Natrium-methylat und 30 ml Ethanol wird 2 Stunden bei 20 °C bis 30 °C gerührt. Dann werden 5,9 g (0,01 Mol) N'- (4,6-Dimethyl-pyrimidin-2-yl)- N''-methoxy-N''N'''-bis-(2-methoxycarbonyl-benzolsulfonyl)-guanidin dazugegeben und das Reaktionsgemisch wird 2 Tage bei 60 °C gerührt. Nach Erkalten wird abgesaugt.

Man erhält 4,8 g (89% der Theorie) N'-(4,6-Dimethylpyrimidin-2-yl)-N''-(2-methoxycarbonyl-benzolsulfonyl)-O-(biphenyl-4-yl)-isoharnstoff-Natriumsalz vom Schmelzpunkt 235 °C (Zersetzung).

Beispiel 3:

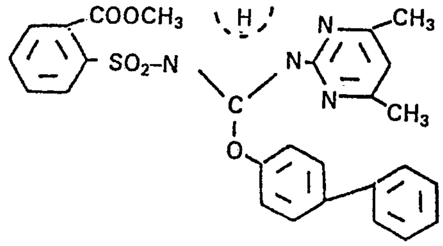

Zu einer Mischung aus 2,0 g (0,0037 Mol) N'-(4,6-Dimethyl-pyrimidin-2-yl)-N"-(2-methoxycarbonyl-benzolsulfonyl) -O-(biphenyl- 4-yl)-isoharnstoff-Natriumsalz (vergl. Beispiel 2) und 10 ml Wasser wird unter Rühren Essigsäure tropfenweise gegeben, bis ein pH-Wert von ca. 4,5 erreicht wird. Nach etwa 20-stündigem Rühren wird abgesaugt.

Man erhält 1,8 g (94% der Theorie) N'-(4,6-Dimethyl-pyrimidin- 2-yl)- N"-(2-methoxycarbonyl-benzolsulfonyl) -O-(biphenyl- 4-yl)-isoharnstoff vom Schmelzpunkt 71 °C.

Beispiel 4:

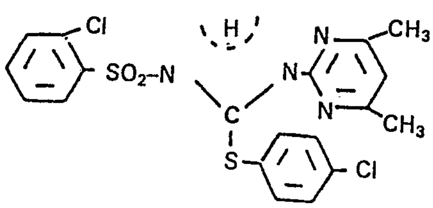

Eine Lösung von 1,5 g (0,027 Mol) Natriummethylat in 5 ml Methanol wird unter Rühren zu einer Mischung aus 13,8 g (0,025 Mol) N'-(4,6-Dimethyl-pyrimidin-2-yl)- N"-methoxy-N",N'"-bis- (2-chlor-benzolsulfonyl)- guanidin, 3,7 g (0,025 Mol) 4-Chlorthiophenol und 50 ml Ethanol gegeben und das Reaktionsgemisch wird 2 Stunden unter Rückfluss zum Sieden erhitzt. Nach Abkühlen wird mit Wasser und Methylenchlorid versetzt, im Scheidetrichter geschüttelt, die organische Phase abgetrennt, getrocknet, filtriert und zur Trockne eingeengt. Der Rückstand wird mit Diethylether zur Kristallisation gebracht.

Man erhält 3,0 g (26% der Theorie) N'-(4,6-Dimethyl-pyrimidin-2-yl)-N"-(2-chlor-benzolsulfonyl)-S-(4-chlor-phenyl)-isothioharnstoff vom Schmelzpunkt 140 °C.

Analog Beispiel 1 bis 4 können die in der nachfolgenden Tabelle 1 aufgeführten Verbindungen der Formel (Ic) hergestellt werden:

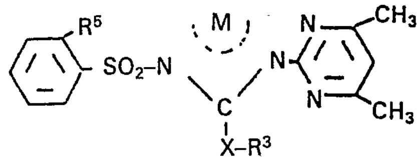

(Ic)

Tabelle 1

| Beisp. Nr. | M | X | $R^5$ | $R^3$ | Schmelz. punkt (° C) |
|---|---|---|---|---|---|
| 5 | H | O | Cl | 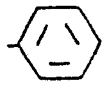 | 115 |
| 6 | Na | O | $COOCH_3$ | 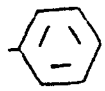 | 190 |
| 7 | 1/2 Ca | O | $COOCH_3$ | 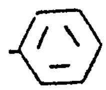 | 167 |
| 8 | K | O | $COOCH_3$ | 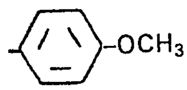 | 147 |
| 9 | Na | O | $COOCH_3$ | 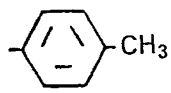 | 151 |
| 10 | Na | O | $COOCH_3$ | 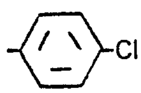 | 181 |
| 11 | Na | O | $COOCH_3$ | 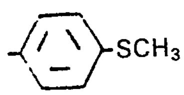 | 146 |
| 12 | Na | O | $COOCH_3$ | 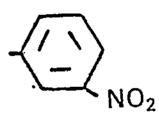 | 155 |
| 13 | Na | O | $COOCH_3$ | 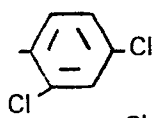 | 90 |
| 14 | Na | O | $COOCH_3$ | 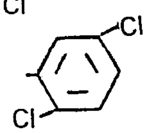 | 57 |
| 15 | H | O | $COOCH_3$ | 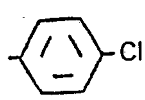 | 64 |
| 16 | Na | O | $COOCH_3$ | 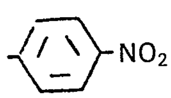 | 55 |
| 17 | H | O | $COOCH_3$ | 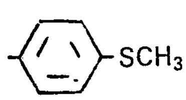 | 42 |
| 18 | H | O | $COOCH_3$ | 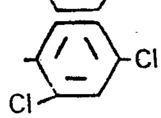 | (öl) |

Tabelle 1 Fortsetzung

| Beisp. Nr. | M | X | $R^5$ | $R^3$ | Schmelz. punkt (° C) |
|---|---|---|---|---|---|
| 19 | H | O | $COOCH_3$ | 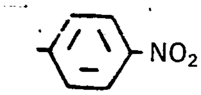 | (öl) |
| 20 | H | O | $COOCH_3$ | 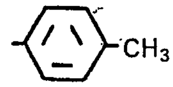 | 95 |
| 21 | H | O | Cl | 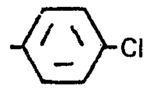 | 55 |
| 22 | Na | O | Cl | 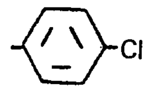 | 174 |
| 23 | Na | O | Cl | 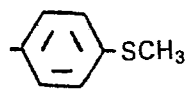 | 135 |
| 24 | Na | O | Cl | 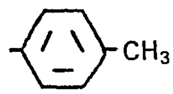 | 150 |
| 25 | Na | O | $COOCH_3$ | 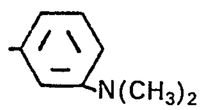 | 175 |
| 26 | H | O | $COOCH_3$ | 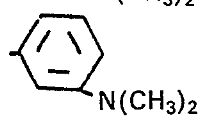 | 35 |
| 27 | Na | O | Cl | 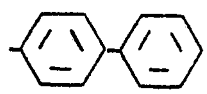 | 187 |
| 28 | H | O | Cl | 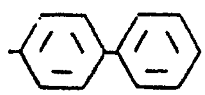 | 143 |
| 29 | Na | O | $COOCH_3$ | 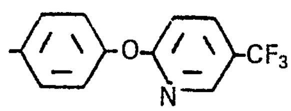 | 174 |
| 30 | Na | O | $COOCH_3$ | 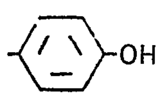 | 188 |
| 31 | Na | O | Cl | 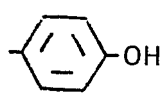 | 165 |
| 32 | Na | O | Cl | 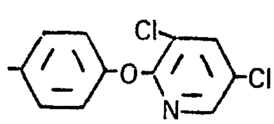 | 175 |

Tabelle 1 Fortsetzung

| Beisp. Nr. | M | X | $R^5$ | $R^3$ | Schmelz. punkt (° C) |
|---|---|---|---|---|---|
| 33 | Na | O | Cl | 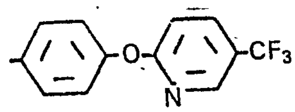 | 165 |
| 34 | Na | O | Br | 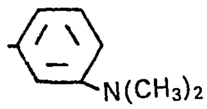 | 165 |
| 35 | Na | O | Br | 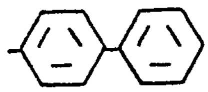 | 170 |
| 36 | Na | O | $COOCH_3$ | 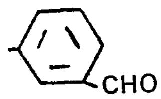 | 162 |
| 37 | Na | O | $COOCH_3$ | 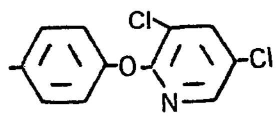 | 172 |
| 38 | H | S | Cl | 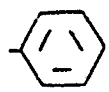 | 125 |
| 39 | H | S | Cl | 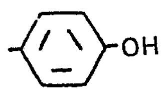 | 196 |
| 40 | H | S | Cl | 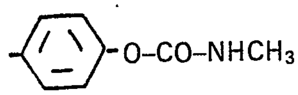 | 160 |
| 41 | H | S | Cl | 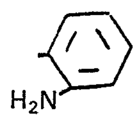 | 231 |
| 42 | H | S | Cl | 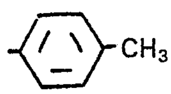 | 163 |
| 43 | H | S | $COOCH_3$ | 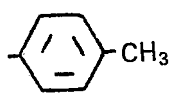 | (öl) |
| 44 | H | S | $COOCH_3$ | 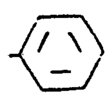 | 138 |
| 45 | H | S | $COOCH_3$ | 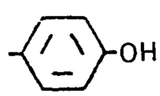 | 160 |
| 46 | H | O | $OCF_3$ | 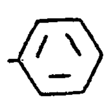 | 101 |

Tabelle 1 Fortsetzung

| Beisp. Nr. | M | X | $R^5$ | $R^3$ | Schmelzpunkt (°C) |
|---|---|---|---|---|---|
| 47 | H | S | $OCF_3$ | 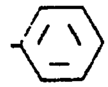 | 133 |
| 48 | Na | O | $COOCH_3$ | 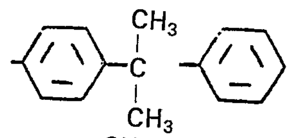 | 182 |
| 49 | Na | O | $COOCH_3$ | 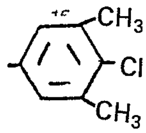 | 171 |
| 50 | Na | O | $COOCH_3$ | 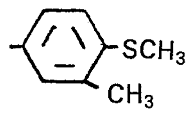 | 173 |
| 51 | Na | O | $COOCH_3$ | 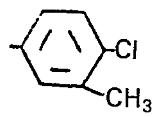 | 183 |
| 52 | Na | O | $COOCH_3$ | 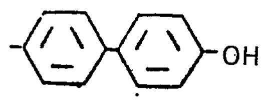 | 175 |
| 53 | Na | O | $COOCH_3$ | 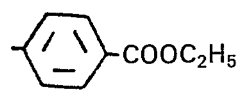 | 138 |
| 54 | Na | O | $COOCH_3$ | 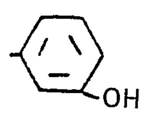 | 168 |
| 55 | Na | O | $COOCH_3$ | 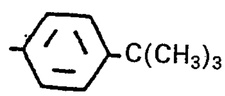 | 175 |
| 56 | Na | O | $COOCH_3$ | 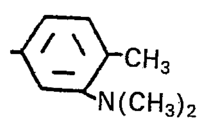 | 106 |
| 57 | Na | O | $COOCH_3$ | 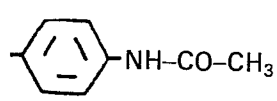 | 118 |
| 58 | Na | O | $COOCH_3$ | 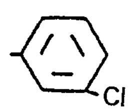 | 125 |
| 59 | Na | O | $COOCH_3$ | 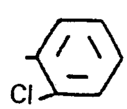 | 169 |
| 60 | Na | O | $COOCH_3$ | 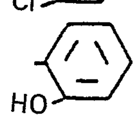 | 74 |

Tabelle 1 Fortsetzung

| Beisp. Nr. | M | X | $R^5$ | $R^3$ | Schmelz-punkt (°C) |
|---|---|---|---|---|---|
| 61 | H | O | $COOCH_3$ | 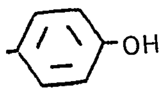 | 135 |
| 62 | H | O | $COOCH_3$ | 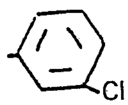 | 122 |
| 63 | H | O | $COOCH_3$ | 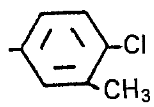 | 172 |
| 64 | H | O | $COOCH_3$ | 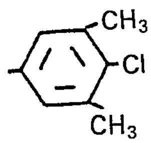 | 116 |

Beispiel 65:

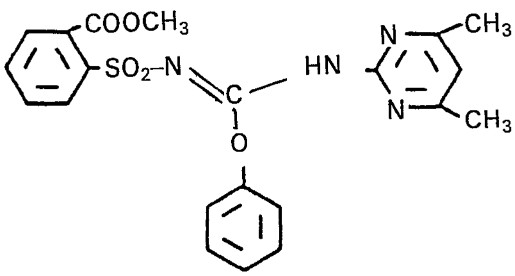

× $H_2SO_4$

Fp: 71 °C – 73 °C

Beispiel 66:

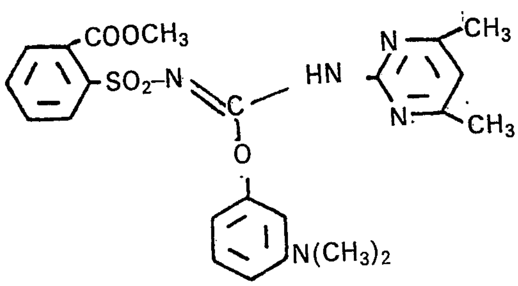

× 2 $CH_3SO_2H$

Fp: 112 °C

Beispiel 67:

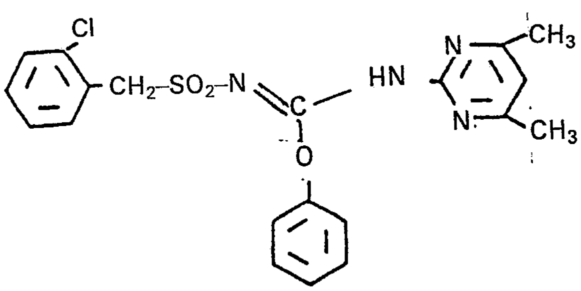

FP: 153 °C

Beispiel 45a

Mono-Natriumsalz der Verbindung von Beispiel 45 (amorph).

Analog Beispiel 1 bis 4 können weiterhin die in der nachfolgenden Tabelle 2 aufgeführten Verbindungen der Formel (Id) hergestellt werden:

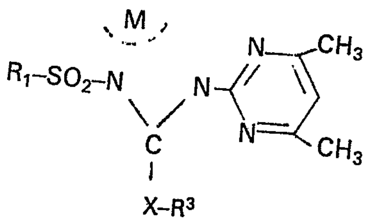

(Id)

Tabelle 2

| Beisp. Nr. | M | X | $R^1$ | $R^3$ | Schmelzpunkt (° C) |
|---|---|---|---|---|---|
| 68 | H | O | 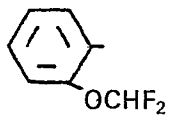 | 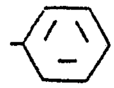 | 113 |
| 69 | H | S | 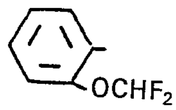 | 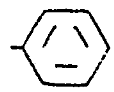 | 107 |
| 70 | H | O | 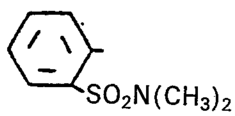 | 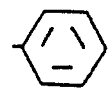 | 168 |
| 71 | H | S | 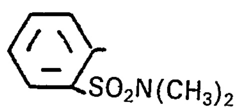 | 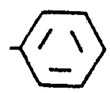 | |
| 72 | H | O | 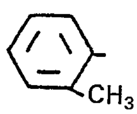 | 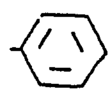 | |
| 73 | H | S | 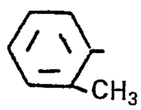 | 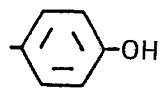 | |
| 74 | H | O | 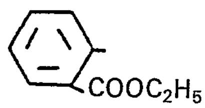 | 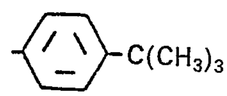 | |
| 75 | H | S | 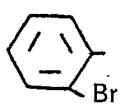 | 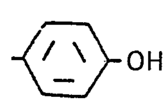 | |
| 76 | H | O | 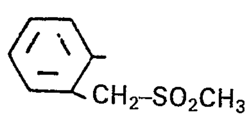 | 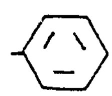 | |
| 77 | H | S | 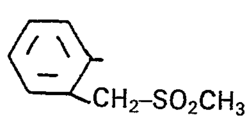 | 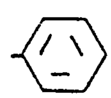 | |
| 78 | H | O | 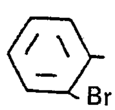 | 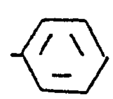 | 131 |

Tabelle 2 Fortsetzung

| Beisp. Nr. | M | X | $R^1$ | $R^3$ | Schmelzpunkt (°C) |
|---|---|---|---|---|---|
| 79 | H | S | 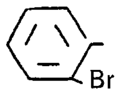 | 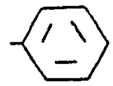 | |
| 80 | H | O | 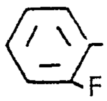 | 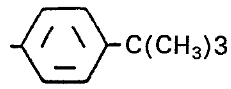 | 154 |
| 81 | H | S | 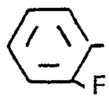 | 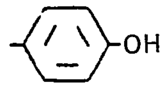 | |
| 82 | H | O | 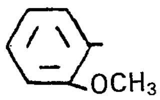 | 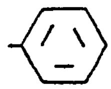 | |
| 83 | H | S | 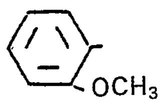 | 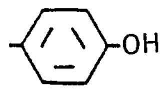 | |
| 84 | H | O | 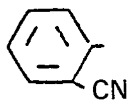 | 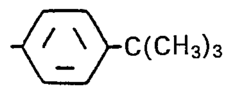 | |
| 85 | H | S | 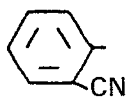 | 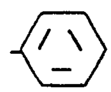 | |
| 86 | H | O | 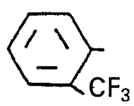 | 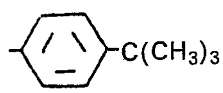 | 158 |
| 87 | H | S | 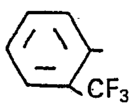 | 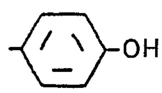 | |
| 88 | H | O | 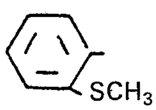 | 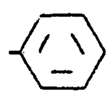 | |
| 89 | H | S | 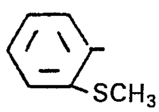 | 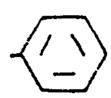 | |
| 90 | H | O | 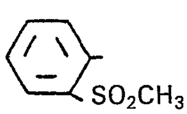 | 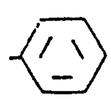 | |
| 91 | H | S | 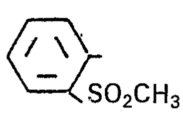 | 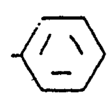 | |
| 92 | H | O | 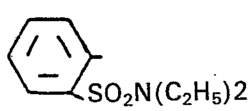 | 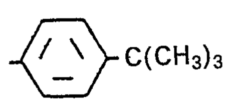 | 147 |

Tabelle 2 Fortsetzung

| Beisp. Nr. | M | X | $R^1$ | $R^3$ | Schmelz-punkt (°C) |
|---|---|---|---|---|---|
| 93 | H | S | 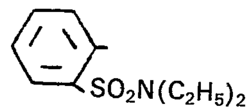 | 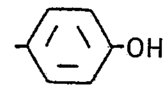 | 211 |
| 94 | H | O | 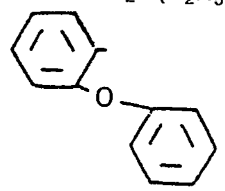 | 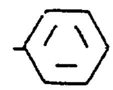 | |
| 95 | H | S | 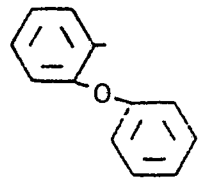 | 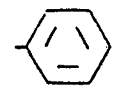 | |
| 96 | H | O | 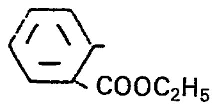 | 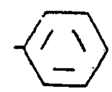 | 104 |
| 97 | H | S | 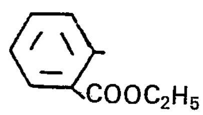 | 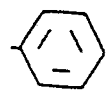 | |
| 98 | H | O | 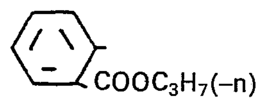 | 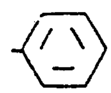 | 106 |
| 99 | H | S | 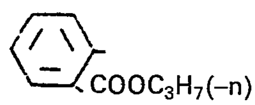 | 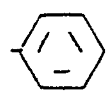 | |
| 100 | H | O | 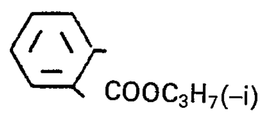 | 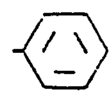 | |
| 101 | H | S | 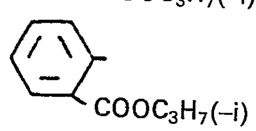 | 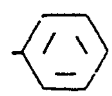 | |
| 102 | H | O | 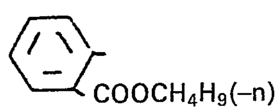 | 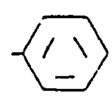 | 97 |
| 103 | H | S | 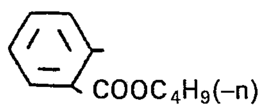 | 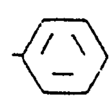 | |
| 104 | H | O | 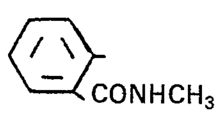 | 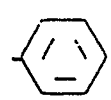 | |
| 105 | H | S | 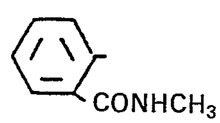 | 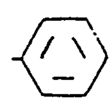 | |

Tabelle 2 Fortsetzung

| Beisp. Nr. | M | X | $R^1$ | $R^3$ | Schmelzpunkt (°C) |
|---|---|---|---|---|---|
| 106 | H | O | 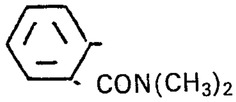 | 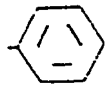 | |
| 107 | H | S | 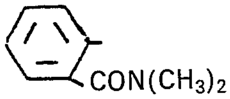 | 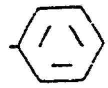 | |
| 108 | H | O | 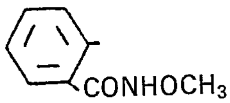 | 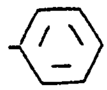 | |
| 109 | H | S | 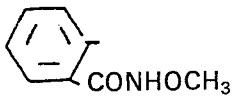 | 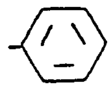 | |
| 110 | H | O | 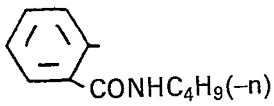 | 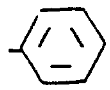 | |
| 111 | H | S | 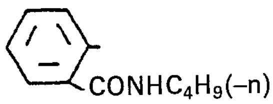 | 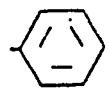 | |
| 112 | H | O | 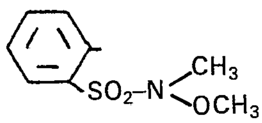 | 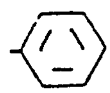 | |
| 113 | H | S | 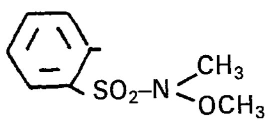 | 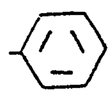 | |
| 114 | H | O | 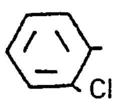 | 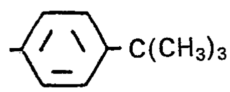 | |
| 115 | H | S | 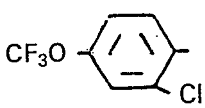 | 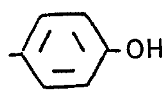 | |
| 116 | H | O | 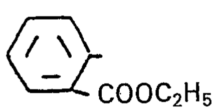 | 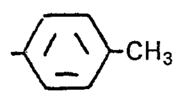 | |
| 117 | H | S | 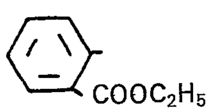 | 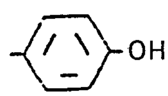 | |
| 118 | H | O | 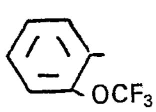 | 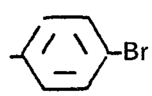 | |
| 119 | H | S | 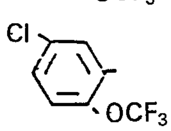 | 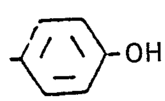 | |

Tabelle 2 Fortsetzung

| Beisp. Nr. | M | X | $R^1$ | $R^3$ | Schmelzpunkt (°C) |
|---|---|---|---|---|---|
| 120 | H | O | 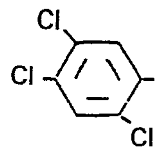 | 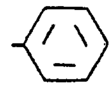 | |
| 121 | H | S | 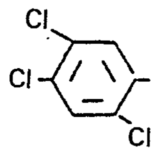 | 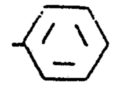 | |
| 122 | H | O | 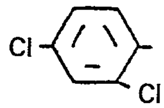 | 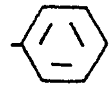 | |
| 123 | H | S | 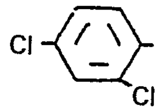 | 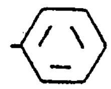 | |
| 124 | H | O | 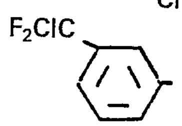 | 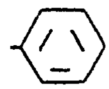 | |
| 125 | H | S | 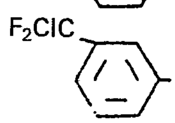 | 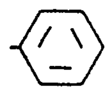 | |
| 126 | H | O | 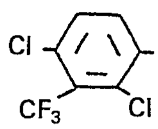 | 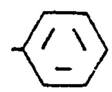 | |
| 127 | H | S | 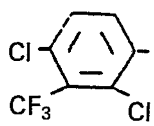 | 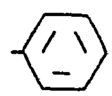 | |
| 128 | H | O | 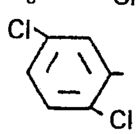 | 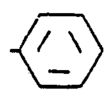 | |
| 129 | H | S | 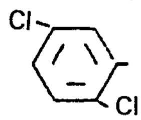 | 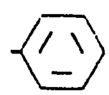 | |
| 130 | H | S | 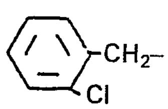 | 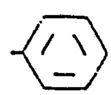 | |
| 131 | H | O | 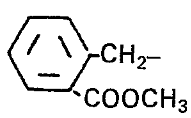 | 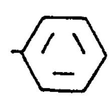 | |
| 132 | H | S | 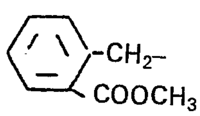 | 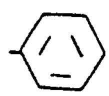 | |

Tabelle 2 Fortsetzung

| Beisp. Nr. | M | X | $R^1$ | $R^3$ | Schmelzpunkt (°C) |
|---|---|---|---|---|---|
| 133 | H | O | 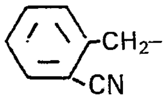 | 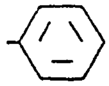 | |
| 134 | H | S | 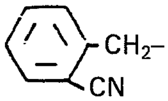 | 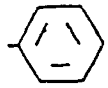 | |
| 135 | H | O | 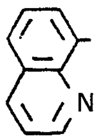 | 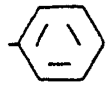 | |
| 136 | H | S | 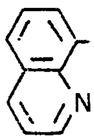 | 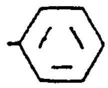 | |
| 137 | H | O | 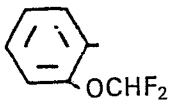 | 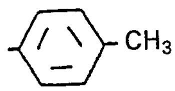 | 113 |
| 138 | H | S | 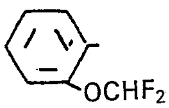 | 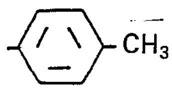 | |
| 139 | H | O | 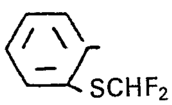 | 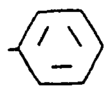 | |
| 140 | H | O | 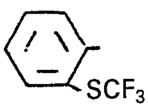 | 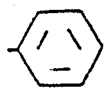 | |
| 141 | H | O | 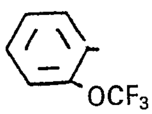 | 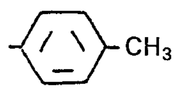 | 131 |
| 142 | H | S | 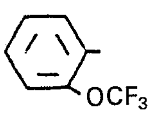 | 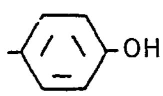 | 169 |
| 143 | H | S | 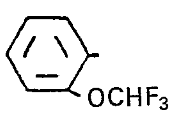 | 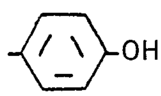 | |
| 144 | H | O | 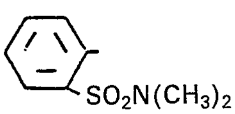 | 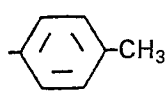 | 173 |
| 145 | H | O | 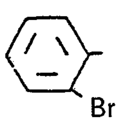 | 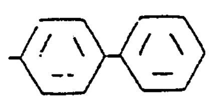 | 128 |

Tabelle 2 Fortsetzung

| Beisp. Nr. | M | X | $R^1$ | $R^3$ | Schmelzpunkt (°C) |
|---|---|---|---|---|---|
| 146 | H | O | 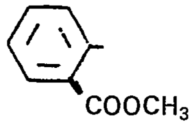 | 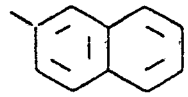 | 119 |
| 147 | H | O | 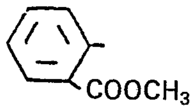 | 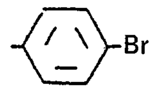 | 132 |
| 148 | H | O | 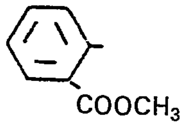 | 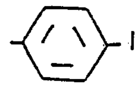 | 129 |
| 149 | H | S | 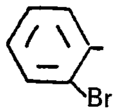 | 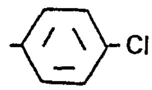 | 157 |
| 150 | H | O | 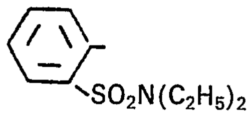 | 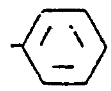 | 152 |
| 151 | H | O | 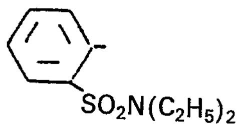 | 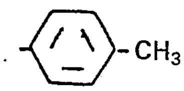 | 142 |
| 152 | H | O | 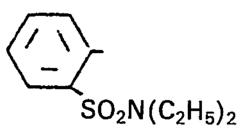 | 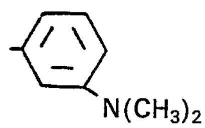 | 188 |
| 153 | H | O | 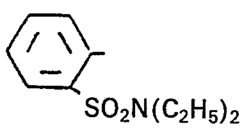 | 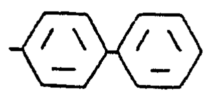 | 124 |
| 154 | H | O | 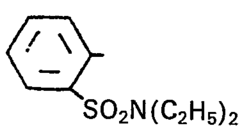 | 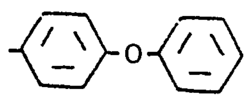 | 140 |
| 155 | H | O | 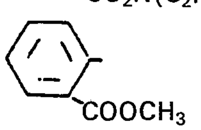 | 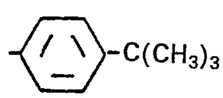 | 170 |
| 156 | H | O | 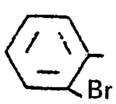 | 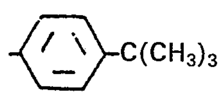 | 161 |
| 157 | H | O | 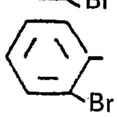 | 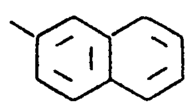 | |
| 158 | H | O | 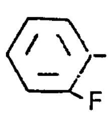 | 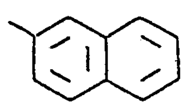 | 67 |
| 159 | H | S | 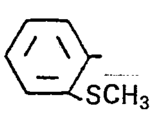 | 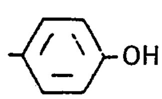 | |

Tabelle 2 Fortsetzung

| Beisp. Nr. | M | X | $R^1$ | $R^3$ | Schmelz-punkt (°C) |
|---|---|---|---|---|---|
| 160 | H | S | 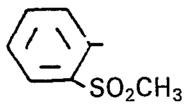 | 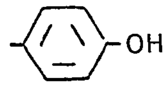 | |
| 161 | H | O | 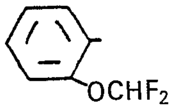 | 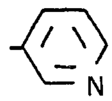 | 48 |
| 162 | H | S | 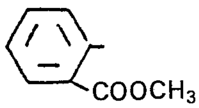 | 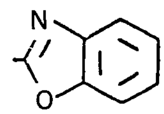 | 156 |
| 163 | Na | O | 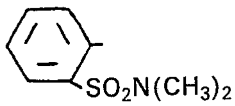 | 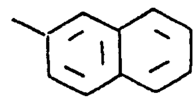 | 217 |
| 164 | H | O | 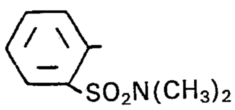 | 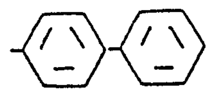 | 172 |
| 165 | H | O | 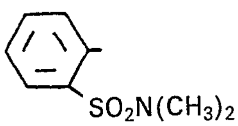 | 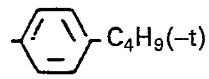 | 154 |
| 166 | H | O | 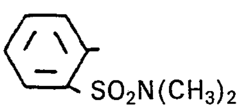 | 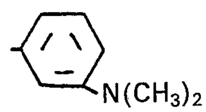 | 183 |

Analog Beispiel 1 bis 4 können weiterhin die in der nachfolgenden Tabelle 3 aufgeführten Verbindungen der Formel (Ie) hergestellt werden:

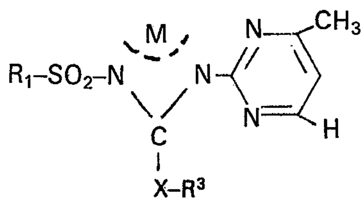

(Ie)

Tabelle 3

| Beisp. Nr. | M | X | $R^1$ | $R^3$ | Schmelz-punkt (°C) |
|---|---|---|---|---|---|
| (Ie– 1) | H | O | 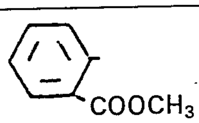 | 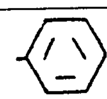 | 93–94 |
| (Ie– 2) | H | O | 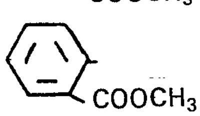 | 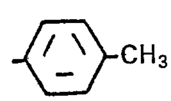 | (öl) |

Tabelle 3 Fortsetzung

| Beisp. Nr. | M | X | $R^1$ | $R^3$ | Schmelz-punkt (°C) |
|---|---|---|---|---|---|
| (Ie– 3) | H | O | 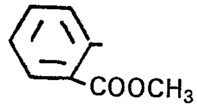 | 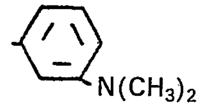 | |
| (Ie– 4) | H | O | 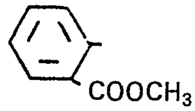 | 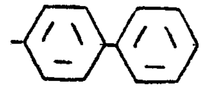 | 208 |
| (Ie– 5) | H | S | 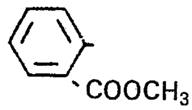 | 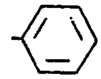 | 73 |
| (Ie– 6) | H | S | 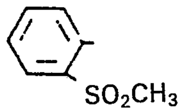 | 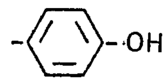 | |
| (Ie– 7) | H | O | 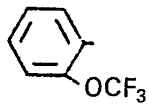 | 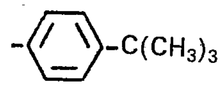 | |
| (Ie– 8) | H | S | 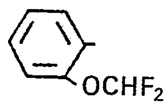 | 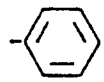 | |
| (Ie– 9) | K | O | 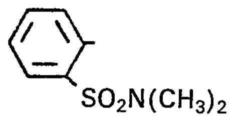 | 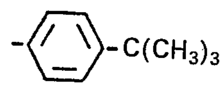 | |
| (Ie–10) | H | S | 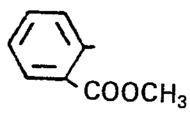 | 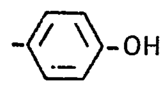 | 134 |
| (Ie–11) | H | O | 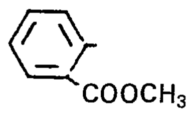 | 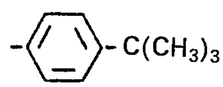 | 93 |
| (Ie–)12 | H | S | 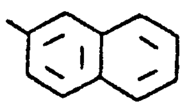 | 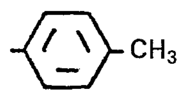 | |
| (Ie–13) | H | O | 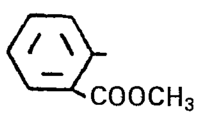 | 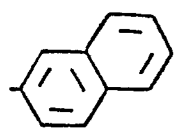 | 196 |
| (Ie–14) | H | O | 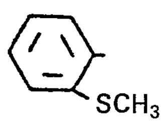 | 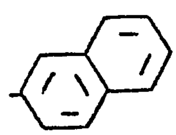 | |
| (Ie–15) | H | O | 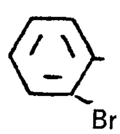 | 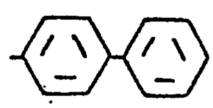 | |

Tabelle 3 Fortsetzung

| Beisp. Nr. | M | X | $R^1$ | $R^3$ | Schmelzpunkt (°C) |
|---|---|---|---|---|---|
| (Ie–16) | H | O | 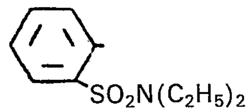 | 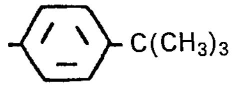 | |
| (Ie–17) | H | S | 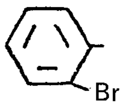 | 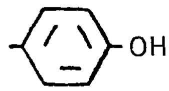 | |
| (Ie–18) | H | S | 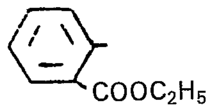 | 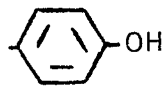 | |
| (Ie–19) | H | S | 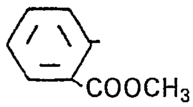 | 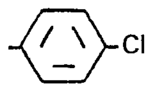 | 112 |
| (Ie–20) | H | S | 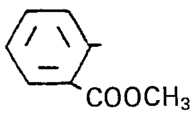 | 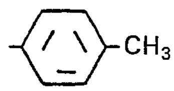 | 126 |
| (Ie–21) | H | S | 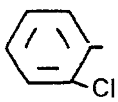 | 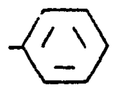 | |
| (Ie–22) | H | O | 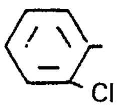 | 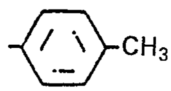 | |
| (Ie–23) | H | S | 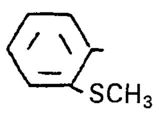 | 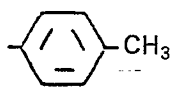 | |
| (Ie–24) | H | O | 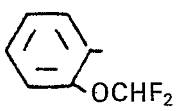 | 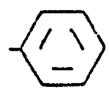 | |
| (Ie–25) | H | O | 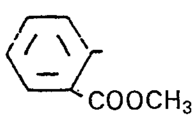 | 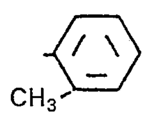 | 118 |
| (Ie–26) | H | O | 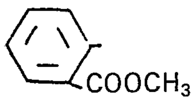 | 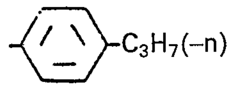 | 80 |

Analog Beispiel 1 bis 4 können weiterhin die in der nachfolgenden Tabelle 4 aufgeführten Verbindungen der Formel (If) hergestellt werden:

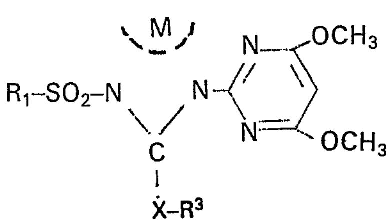

(If)

Tabelle 4

| Beisp. Nr. | M | X | $R^1$ | $R^3$ | Schmelz-punkt (°C) |
|---|---|---|---|---|---|
| (If–)1 | H | O | 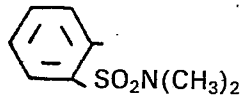 | 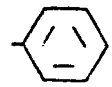 | 196 |
| (If–2) | H | S | 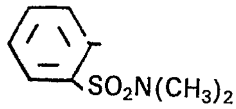 | 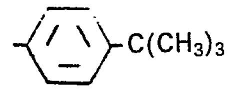 | 184 |
| (If–3) | H | O | 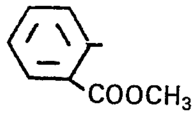 | 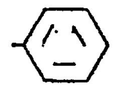 | 133 |
| (If–4) | H | S | 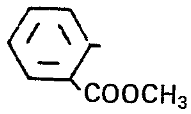 | 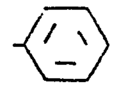 | 210 |
| (If–5) | H | O | 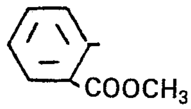 | 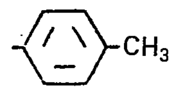 | |
| (If–6) | H | S | 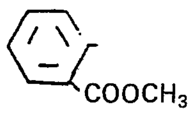 | 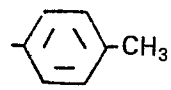 | |
| (If–7) | H | O | 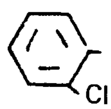 | 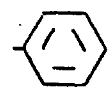 | 125 |
| (If–8) | H | S | 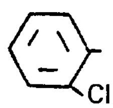 | 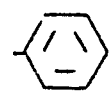 | 153 |
| (If–9) | H | O | 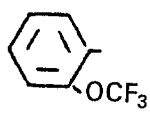 | 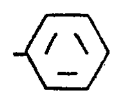 | 112 |
| (If–10) | H | O | 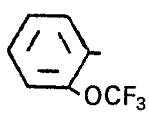 | 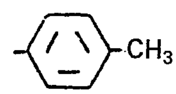 | 105 |
| (If–11) | H | O | 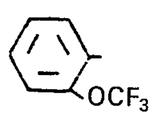 | 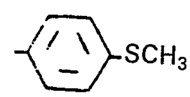 | 109 |
| (If–12) | H | S | 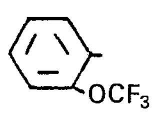 | 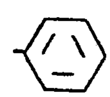 | 147 |
| (If–13) | H | O | 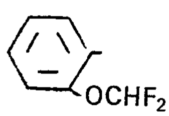 | 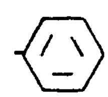 | 142 |
| (If–14) | H | S | 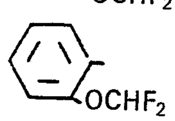 | 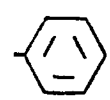 | 168 |

Tabelle 4 Fortsetzung

| Beisp. Nr. | M | X | R¹ | R³ | Schmelz-punkt (°C) |
|---|---|---|---|---|---|
| (If–15) | H | O | 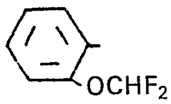 | 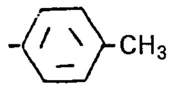 | 129 |
| (If–16) | H | S | 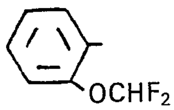 | 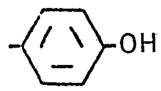 | |
| (If–17) | H | O | 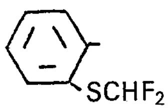 | 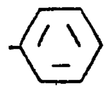 | |
| (If–18) | H | S | 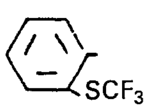 | 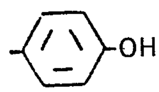 | |
| (If–)19 | H | S | 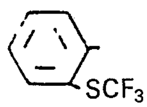 | 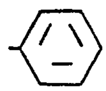 | |
| (If–20) | H | S | 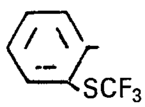 | 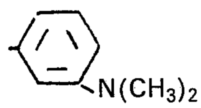 | |
| (If–21) | H | S | 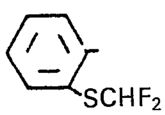 | 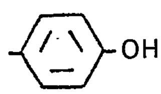 | |
| (If–22) | H | O | 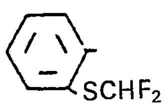 | 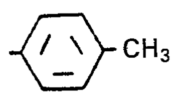 | |
| (If–23) | H | O | 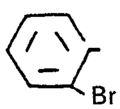 | 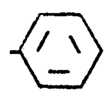 | |
| (If–24) | H | O | 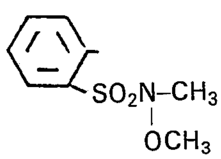 | 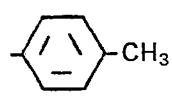 | |
| (If–25) | H | O | 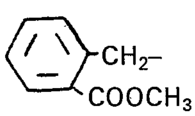 | 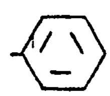 | |
| (If–26) | H | S | 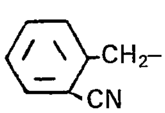 | 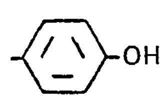 | |
| (If–27) | H | O | 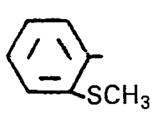 | 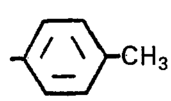 | |

Tabelle 4 Fortsetzung

| Beisp. Nr. | M | X | R¹ | R³ | Schmelz-punkt (°C) |
|---|---|---|---|---|---|
| (If–28) | H | O | 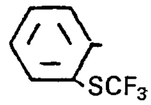 | 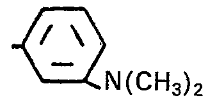 | |
| (If–29) | K | O | 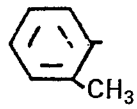 | 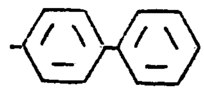 | |
| (If–30) | H | O | 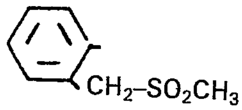 | 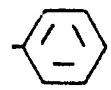 | |
| (If–31) | H | S | 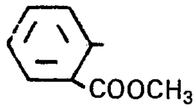 | 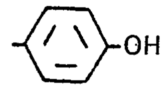 | |
| (If–32) | H | O | 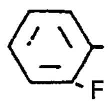 | 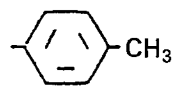 | |
| (If–33) | H | S | 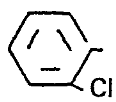 | 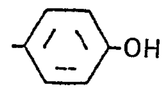 | |
| (If–34) | H | O | 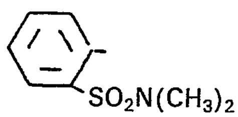 | 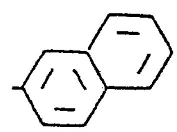 | 214 |
| (If–35) | H | O | 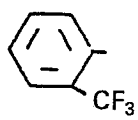 | 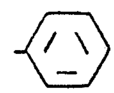 | |

Beispiel (If–34a)

Mono-Natriumsalz der Verbindung von Beispiel (If–34) vom Schmelzpunkt 214 °C.

Analog Beispiel 1 bis 4 können weiterhin die in der nachfolgenden Tabelle 5 aufgeführten Verbindungen der Formel (Ig) hergestellt werden:

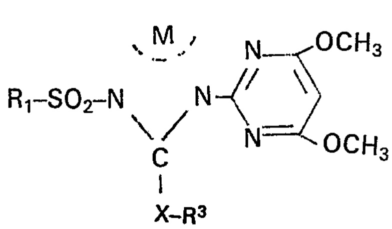

(Ig)

Tabelle 5

| Beisp. Nr. | M | X | R¹ | R³ | Schmelz-punkt (°C) |
|---|---|---|---|---|---|
| (Ig–)1 | H | O | 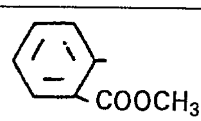 | 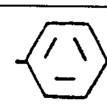 | |

Tabelle 5 Fortsetzung

| Beisp. Nr. | M | X | $R^1$ | $R^3$ | Schmelz-punkt (°C) |
|---|---|---|---|---|---|
| (Ig–2) | H | O | 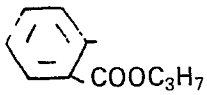 | 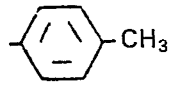 | |
| (Ig–3) | H | S | 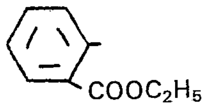 | 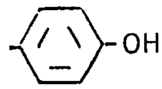 | |
| (Ig–4) | H | O | 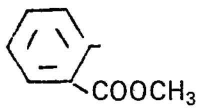 | 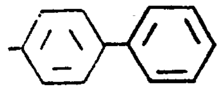 | |
| (Ig–5) | H | O | 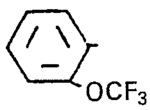 | 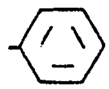 | |
| (Ig–6) | H | O | 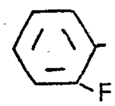 | 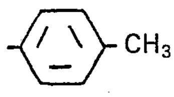 | |
| (Ig–7) | H | O | 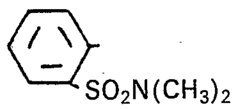 | 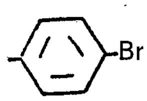 | |
| (Ig–8) | H | O | 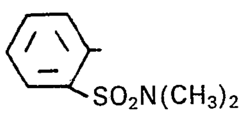 | 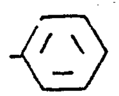 | |
| (Ig–9) | H | S | 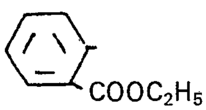 | 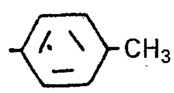 | |
| (Ig–10) | H | O | 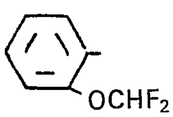 | 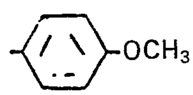 | |
| (Ig–11) | H | S | 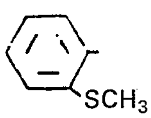 | 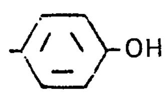 | |
| (Ig–12) | H | O | 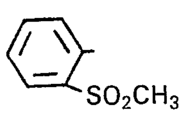 | 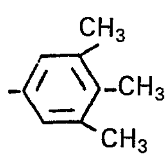 | |
| (Ig–13) | H | O | 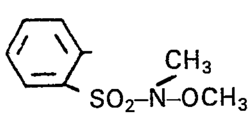 | 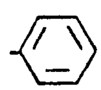 | |
| (Ig–14) | H | S | 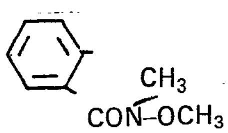 | 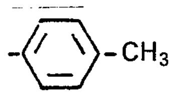 | |

Tabelle 5 Fortsetzung

| Beisp. Nr. | M | X | $R^1$ | $R^3$ | Schmelz-punkt (°C) |
|---|---|---|---|---|---|
| (Ig–15) | H | O | 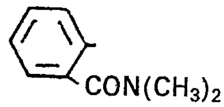 | 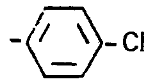 | |
| (Ig–16) | H | O | 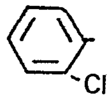 | 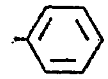 | |
| (Ig–17) | H | S | 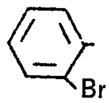 | 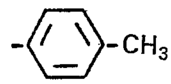 | |
| (Ig–18) | H | O | 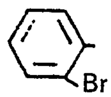 | 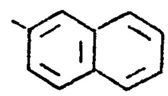 | 160 |
| (Ig–)19 | H | O | 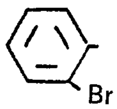 | 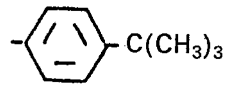 | 163 |
| (Ig–20) | H | O | 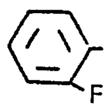 | 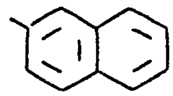 | 185 |
| (Ig–21) | H | O | 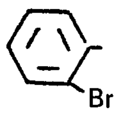 | 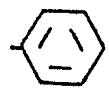 | 108 |
| (Ig–22) | H | O | 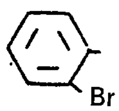 | 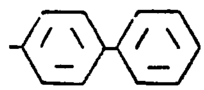 | amorph |
| (Ig–23) | H | O | 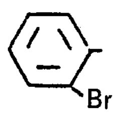 | 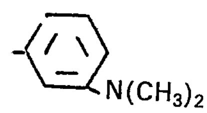 | 162 |
| (Ig–24) | H | O | 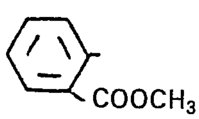 | 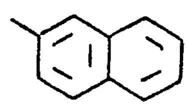 | 147 |

Analog Beispiel 1 bis 4 können weiterhin die in der nachfolgenden Tabelle 6 aufgeführten Verbindungen der Formel (Ih) hergestellt werden:

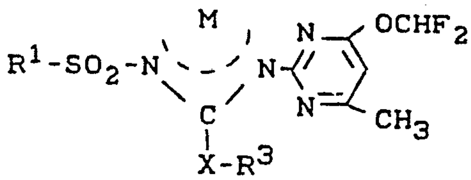

(Ih)

Tabelle 6

| Beisp. Nr. | M | X | $R^1$ | $R^3$ | Schmelz-punkt (°C) |
|---|---|---|---|---|---|
| (Ih–)1 | H | O | 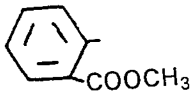 | 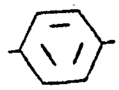 | |
| (Ih–2) | H | O | 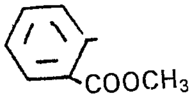 | 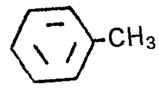 | |
| (Ih–3) | H | S | 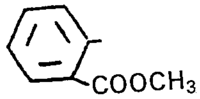 | 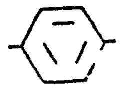 | |
| (Ih–4) | H | S | 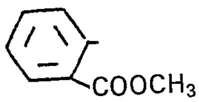 | 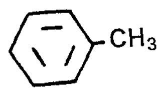 | |
| (Ih–5) | H | S | 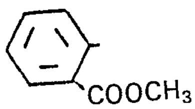 | 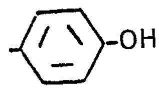 | |
| (Ih–6) | H | O | 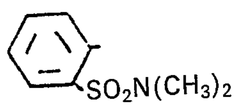 | 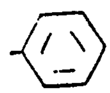 | |
| (Ih–7) | H | O | 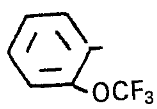 | 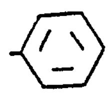 | |
| (Ih–8) | H | O | 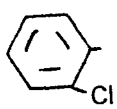 | 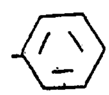 | |
| (Ih–9) | H | O | 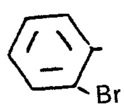 | 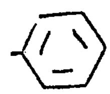 | |
| (Ih–10) | H | O | 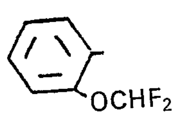 | 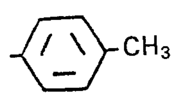 | |

Analog Beispiel 1 bis 4 können weiterhin die in der nachfolgenden Tabelle 7 aufgeführten Verbindungen der Formel (Ii) hergestellt werden:

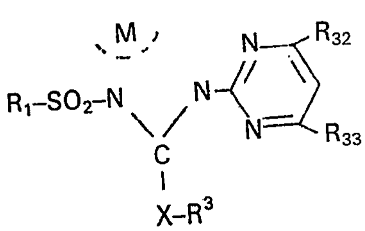

(Ii)

Tabelle 7

| Beisp. Nr. | M | X | R¹ | R³ | $R^{32}$ | $R^{33}$ | Schmelzpunkt (°C) |
|---|---|---|---|---|---|---|---|
| (Ii–)1 | H | S | 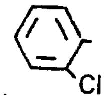 | 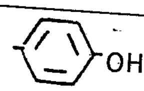 | $CH_3$ | $OCH_3$ | 190 |
| (Ii–2) | H | S | 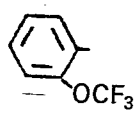 | 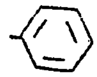 | $CH_3$ | $OCH_3$ | |
| (Ii–3) | H | S | 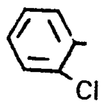 | 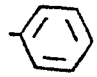 | $CH_3$ | $OCH_3$ | |
| (Ii–4) | H | S | 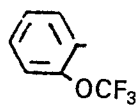 | 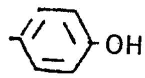 | $CH_3$ | $OCH_3$ | |
| (Ii–)5 | H | S | 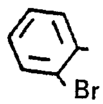 | 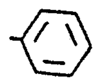 | $CH_3$ | $OCH_3$ | |
| (Ii–6) | H | S | 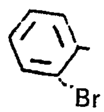 | 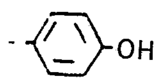 | $CH_3$ | $OCH_3$ | |
| (Ii–7) | H | O | 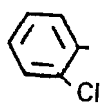 | 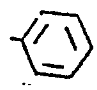 | $CH_3$ | $CH_3$ | |
| (Ii–8) | H | S | 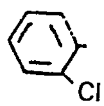 | 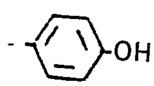 | $CH_3$ | $CH_3$ | |
| (Ii–9) | H | S | 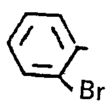 | 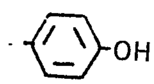 | $CH_3$ | $CH_3$ | |
| (Ii–10) | H | S | 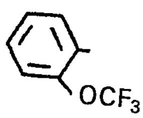 | 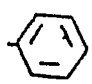 | $CH_3$ | $CH_3$ | |
| (Ii–11) | H | S | 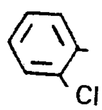 | 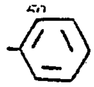 | $C_2H_5$ | $OCH_3$ | |
| (Ii–12) | H | S | 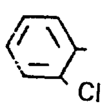 | 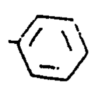 | $OCH_3$ | $OCH_3$ | |
| (Ii–13) | H | S | 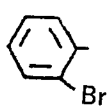 | 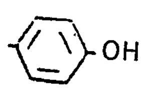 | $OCH_3$ | $OCH_3$ | |
| (Ii–14) | H | S | 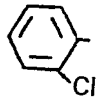 | 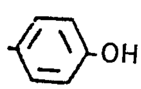 | $CH_3$ | $N(CH_3)_2$ | |

Tabelle 7 Fortsetzung

| Beisp. Nr. | M | X | $R^1$ | $R^3$ | $R^{32}$ | $R^{33}$ | Schmelzpunkt (°C) |
|---|---|---|---|---|---|---|---|
| (Ii–15) | H | S | 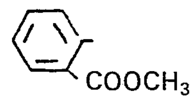 | 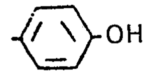 | $OCH_3$ | $N(CH_3)_2$ | |
| (Ii–16) | H | S | 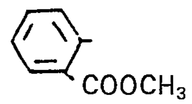 | 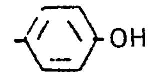 | $SCH_3$ | $NHC_2H_5$ | |

Herstellung von Ausgangsstoffen der Formel (II)

Beispiel (II-1)

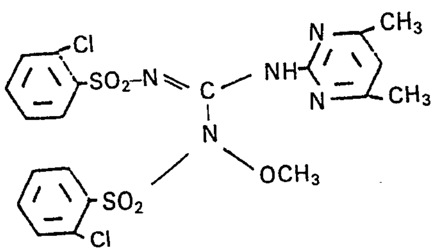

Eine Mischung aus 29,4 g (0,15 Mol) N'-(4,6-Dimethylpyrimidin- 2-yl)-N''-methoxy-guanidin, 63,3 g (0,3 Mol) 2-Chlor-benzolsulfonsäurechlorid und 150 ml Pyridin wird 2 Tage bei 20 °C gerührt. Nach weitgehendem Abdestillieren des Pyridins im Wasserstrahlvakuum wird der Rückstand mit 200 ml Wasser versetzt und mit 200 ml Methylenchlorid extrahiert. Die organische Phase wird abgetrennt, getrocknet und eingeengt. Der Rückstand wird durch Digerieren mit Ethanol zur Kristallisation gebracht.

Man erhält 41,2 g (51% der Theorie) N'-(4,6-Dimethylpyrimidin-2-yl) -N''-methoxy-N'', N'''-bis- (2-chlor-benzolsulfonyl)- guanidin vom Schmelzpunkt 164 °C bis 166 °C.

Beispiel (II-2)

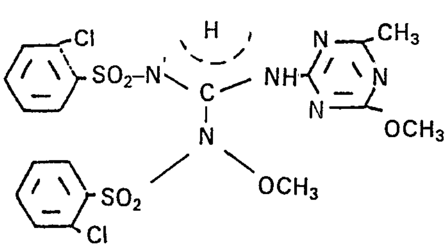

54 g (0,256 Mol) 2-Chlor-benzolsulfonsäurechlorid werden unter Rühren zu einer auf −20 °C gekühlten Mischung aus 97 g (0,25 Mol) N'-(4-Methoxy- 6-methyl-s-triazin- 2-yl)-N''-methoxy-N'''-(2-chlor-benzolsulfonyl)-guanidin und 300 ml Pyridin tropfenweise gegeben. Das Reaktionsgemisch wird 15 Stunden bei 20 °C gerührt und eingeengt. Der Rückstand wird in 300 ml Methylenchlorid aufgenommen, diese Lösung zweimal mit je 150 ml verdünnter Salzsäure gewaschen, filtriert und eingeengt. Das beim Verreiben des Rückstandes mit Ethanol kristallin anfallende Produkt wird durch Absaugen isoliert.

Man erhält 80 g (70% der Theorie) N'-(4-Methoxy-6-methyl-s-triazin-2-yl)-N''-methoxy-N'', N'''-bis- (2-chlor-benzolsulfonyl)- guanidin vom Schmelzpunkt 164 °C.

Beispiel (II-3)

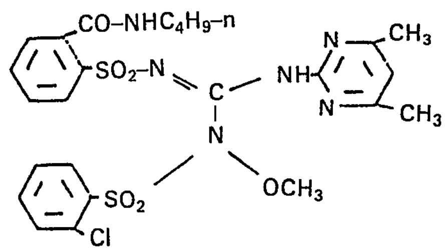

2,5 g (0,012 Mol) 2-Chlor-benzolsulfonsäurechlorid werden zu einer Mischung aus 5,0 g (0,011 Mol) N'-(4,6-Dimethyl-pyrimidin-2-yl)-N''-methoxy-N'''-(2-butylaminocarbonyl-benzolsulfonyl)-guanidin, 2,0 g (0,016 Mol) 4-Dimethylamino-pyridin und 30 ml Acetonitril gegeben. Das Reaktionsgemisch wird 3 Stunden unter Rückfluss zum Sieden erhitzt und anschliessend eingeengt. Der Rückstand wird in 100 ml Methylenchlorid aufgenommen. Diese Lösung wird mit 100 ml 5%iger Salzsäure und mit 100 ml Wasser gewaschen, getrocknet, filtriert und eingeengt.

Man erhält 1,8 g (30% der Theorie) N'-(4,6-Dimethyl-pyrimidin-2-yl)-N''-methoxy-N''-(2-chlorbenzolsulfonyl)-N'''-(2-butylaminocarbonyl-benzolsulfonyl)-guanidin als kristallinen Rückstand vom Schmelzpunkt 157 °C.

Analog können die in den nachstehenden Tabellen 8 und 9 aufgeführten Verbindungen der Formel (II) hergestellt werden:

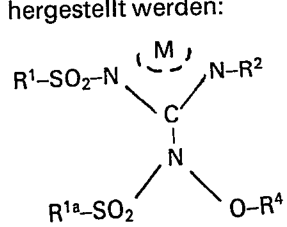

(II)

Tabelle 8

| Beisp. Nr. | $R^1=R^{1a}$ | $R^2$ | $R^4$ | M | Schmelz-punkt (°C) |
|---|---|---|---|---|---|
| (II–4) | 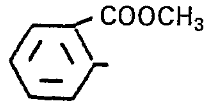 | 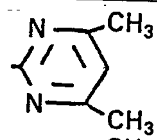 | $CH_3$ | H | 165 |
| (II–5) | 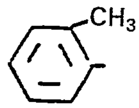 | 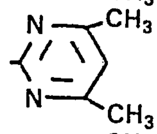 | $CH_3$ | H | 129 |
| (II–6) | 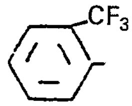 | 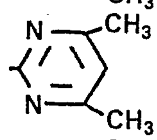 | $CH_3$ | H | 121 |
| (II–7) | 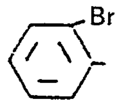 | 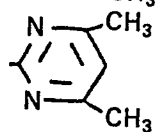 | $CH_3$ | H | 147 |
| (II–8) | 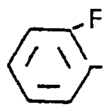 | 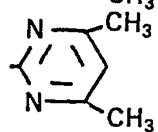 | $CH_3$ | H | 166 |
| (II–9) | 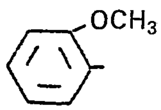 | 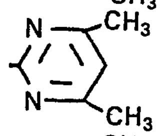 | $CH_3$ | H | 196 |
| (II–10) | 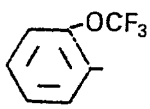 | 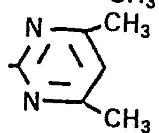 | $CH_3$ | H | 158 |
| (II–11) | 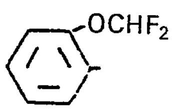 | 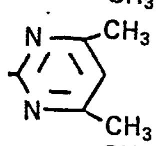 | $CH_3$ | H | 163 |
| (II–12) | 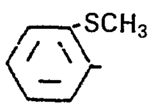 | 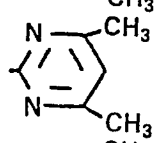 | $CH_3$ | H | 158 |
| (II–13) | 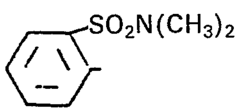 | 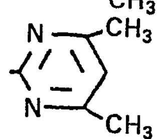 | $CH_3$ | H | 138 |
| (II–14) | 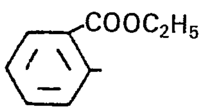 | 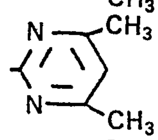 | $CH_3$ | H | 106 |
| (II–15) | 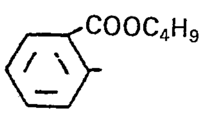 | 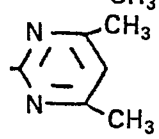 | $CH_3$ | H | 104 |
| (II–16) | 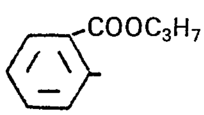 | 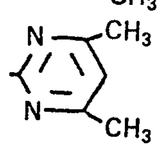 | $CH_3$ | H | 134 |

Tabelle 8 Fortsetzung

| Beisp. Nr. | $R^1 = R^{1a}$ | $R^2$ | $R^4$ | M | Schmelzpunkt (°C) |
|---|---|---|---|---|---|
| (II–17) | 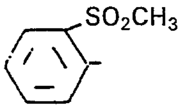 | 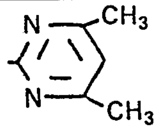 | $CH_3$ | H | 155 |
| (II–18) | 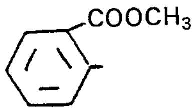 | 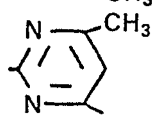 | $CH_3$ | H | 154 |
| (II–19) | 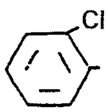 | 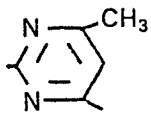 | $CH_3$ | H | 140–141 |
| (II–20) | 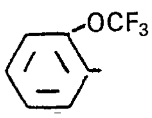 | 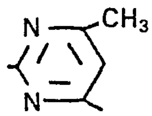 | $CH_3$ | H | 170 |
| (II–21) | 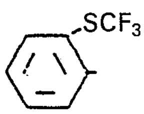 | 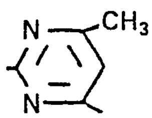 | $CH_3$ | H | 118 |
| (II–22) | 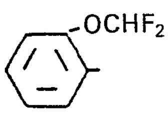 | 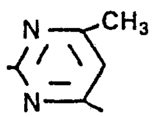 | $CH_3$ | H | 166–167 |
| (II–23) | 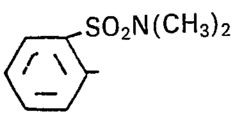 | 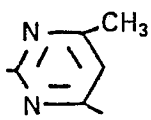 | $CH_3$ | H | |
| (II–24) | 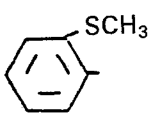 | 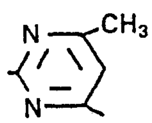 | $CH_3$ | H | 158–159 |
| (II–25) | 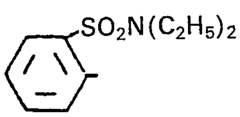 | 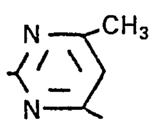 | $CH_3$ | H | 137–138 |
| (II–26) | 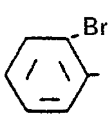 | 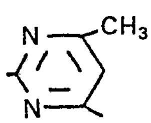 | $CH_3$ | H | 162–163 |
| (II–27) | 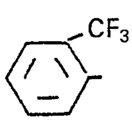 | 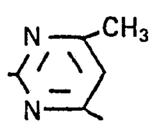 | $CH_3$ | H | |
| (II–28) | 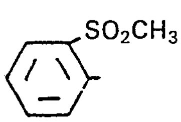 | 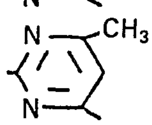 | $CH_3$ | H | |
| (II–29) | 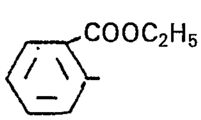 | 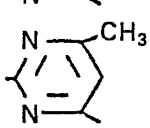 | $CH_3$ | H | |
| (II–30) | 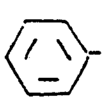 | 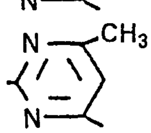 | $CH_3$ | H | 160 |

Tabelle 8 Fortsetzung

| Beisp. Nr. | $R^1=R^{1a}$ | $R^2$ | $R^4$ | M | Schmelz-punkt (°C) |
|---|---|---|---|---|---|
| (II–31) | 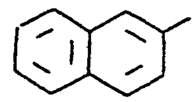 | 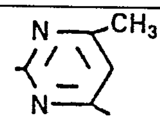 | $CH_3$ | H | 107 |
| (II–32) | 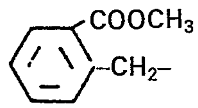 | 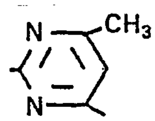 | $CH_3$ | H | |
| (II–33) | 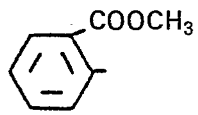 | 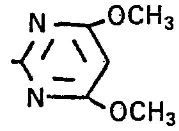 | $CH_3$ | H | 149 |
| (II–34) | 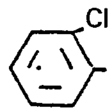 | 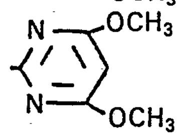 | $CH_3$ | H | 164–165 |
| (II–35) | 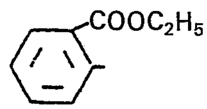 | 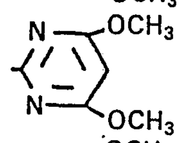 | $CH_3$ | H | |
| (II–36) | 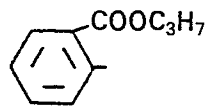 | 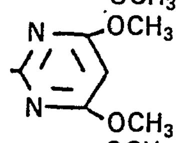 | $CH_3$ | H | |
| (II–37) | 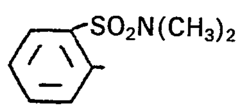 | 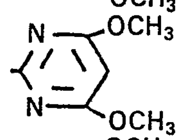 | $CH_3$ | H | 177 |
| (II–38) | 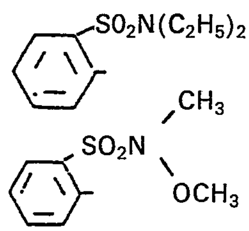 | 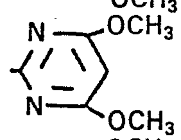 | $CH_3$ | H | |
| (II–39) | | 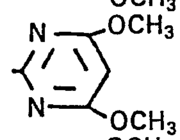 | $CH_3$ | H | |
| (II–40) | 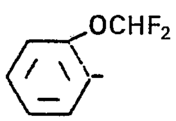 | 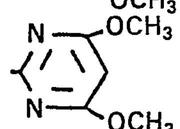 | $CH_3$ | H | 136 |
| (II–41) | 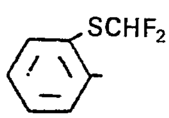 | 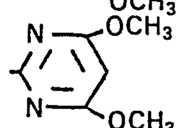 | $CH_3$ | H | |
| (II–42) | 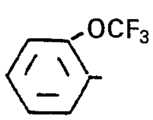 | 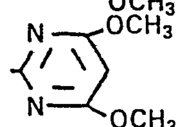 | $CH_3$ | H | 134 |
| (II–43) | 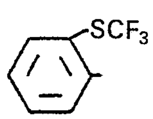 | 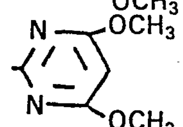 | $CH_3$ | H | |
| (II–44) | 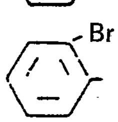 | 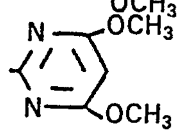 | $CH_3$ | H | |

Tabelle 8 Fortsetzung

| Beisp. Nr. | $R^1=R^{1a}$ | $R^2$ | $R^4$ | M | Schmelzpunkt (°C) |
|---|---|---|---|---|---|
| (II–45) | 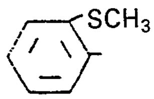 | 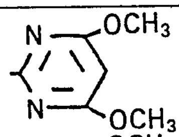 | $CH_3$ | H | |
| (II–46) | 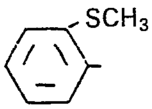 | 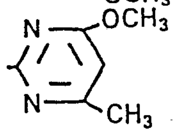 | $CH_3$ | H | 178 (Zers.) |
| (II–47) | 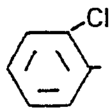 | 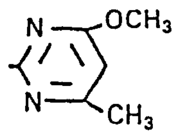 | $CH_3$ | H | 172 |
| (II–48) | 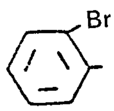 | 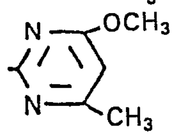 | $CH_3$ | H | 174 |
| (II–49) | 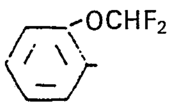 | 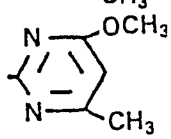 | $CH_3$ | H | |
| (II–50) | 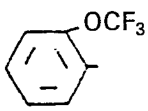 | 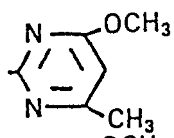 | $CH_3$ | H | 194 |
| (II–51) | 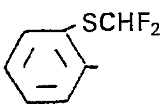 | 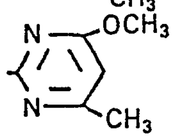 | $CH_3$ | H | |
| (II–52) | 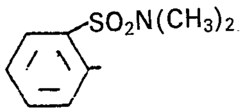 | 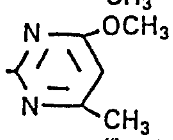 | $CH_3$ | H | 141 |
| (II–53) | 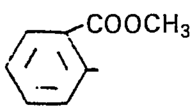 | 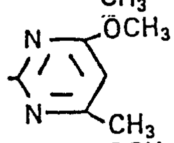 | $CH_3$ | H | 152 |
| (II–54) | 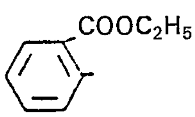 | 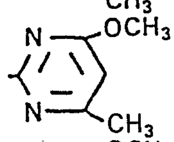 | $CH_3$ | H | |
| (II–55) | 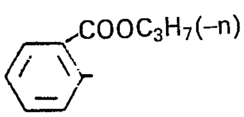 | 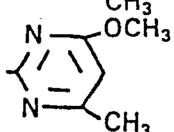 | $CH_3$ | H | |
| (II–56) | 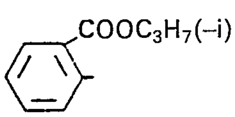 | 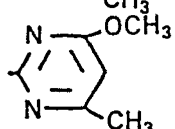 | $CH_3$ | H | $n_D^{20}$ =1.5391 |
| (II–57) | 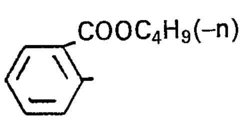 | 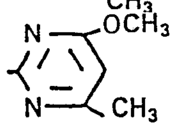 | $CH_3$ | H | |

Tabelle 8 Fortsetzung

| Beisp. Nr. | $R^1 = R^{1a}$ | $R^2$ | $R^4$ | M | Schmelzpunkt (°C) |
|---|---|---|---|---|---|
| (II–58) | 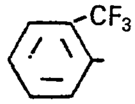 | 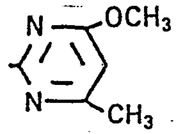 | $CH_3$ | H | 165 |
| (II–59) | 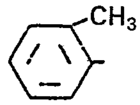 | 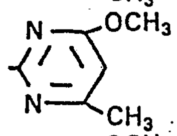 | $CH_3$ | H | |
| (II–60) | 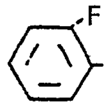 | 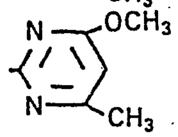 | $CH_3$ | H | 174 |
| (II–61) | 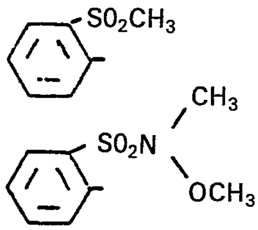 | 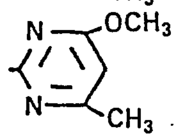 | $CH_3$ | H | |
| (II–62) | | 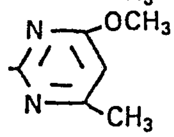 | $CH_3$ | H | |
| (II–63) | 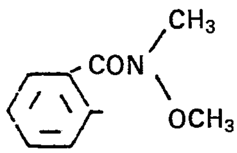 | 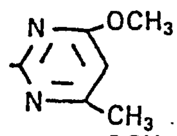 | $CH_3$ | H | |
| (II–64) | 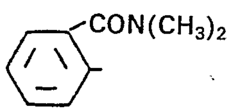 | 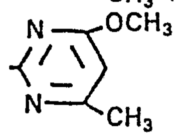 | $CH_3$ | H | |
| (II–65) | 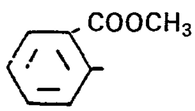 | 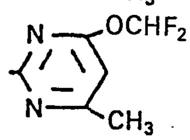 | $CH_3$ | H | |
| (II–66) | 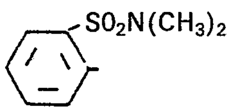 | 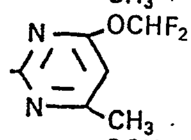 | $CH_3$ | H | |
| (II–67) | 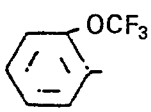 | 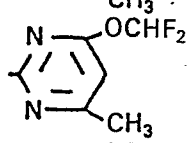 | $CH_3$ | H | |
| (II–68) | 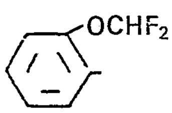 | 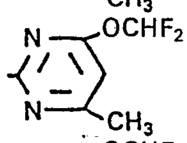 | $CH_3$ | H | |
| (II–69) | 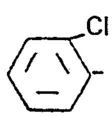 | 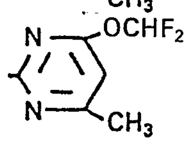 | $CH_3$ | H | |
| (II–70) | 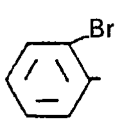 | 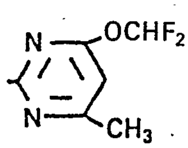 | $CH_3$ | H | |

Tabelle 8 Fortsetzung

| Beisp. Nr. | $R^1=R^{1a}$ | $R^2$ | $R^4$ | M | Schmelzpunkt (°C) |
|---|---|---|---|---|---|
| (II–71) | 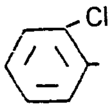 | 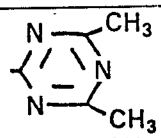 | $CH_3$ | H | |
| (II–72) | 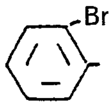 | 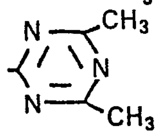 | $CH_3$ | H | |
| (II–73) | 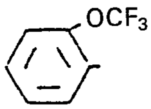 | 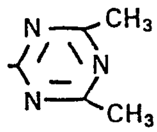 | $CH_3$ | H | |
| (II–74) | 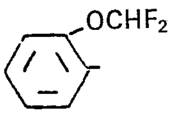 | 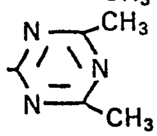 | $CH_3$ | H | |
| (II–75) | 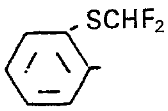 | 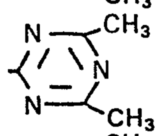 | $CH_3$ | H | |
| (II–76) | 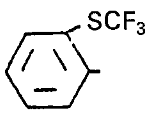 | 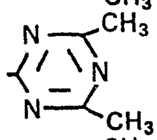 | $CH_3$ | H | |
| (II–77) | 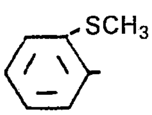 | 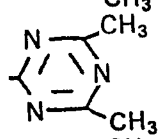 | $CH_3$ | H | |
| (II–78) | 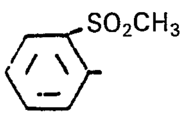 | 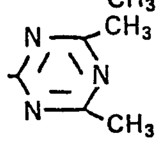 | $CH_3$ | H | |
| (II–79) | 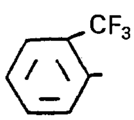 | 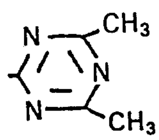 | $CH_3$ | H | |
| (II–80) | 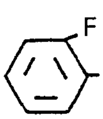 | 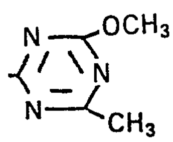 | $CH_3$ | H | |
| (II–81) | 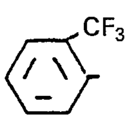 | 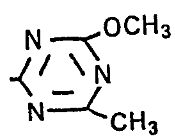 | $CH_3$ | H | |
| (II–82) | 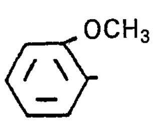 | 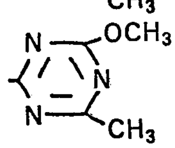 | $CH_3$ | H | |
| (II–83) | 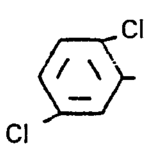 | 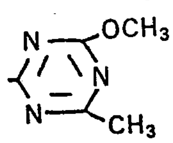 | $CH_3$ | H | |

Tabelle 8 Fortsetzung

| Beisp. Nr. | $R^1=R^{1a}$ | $R^2$ | $R^4$ | M | Schmelz-punkt (°C) |
|---|---|---|---|---|---|
| (II-84) | 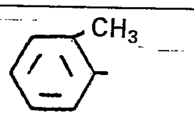 | 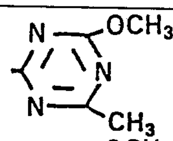 | $CH_3$ | H | |
| (II-85) | 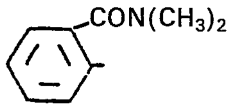 | 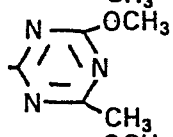 | $CH_3$ | H | |
| (II-86) | 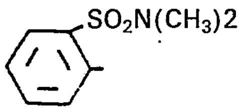 | 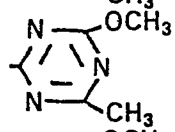 | $CH_3$ | H | |
| (II-87) | 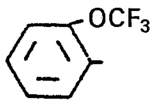 | 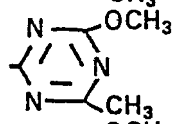 | $CH_3$ | H | |
| (II-88) | 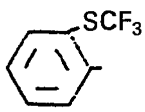 | 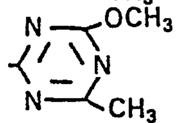 | $CH_3$ | H | |
| (II-89) | 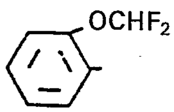 | 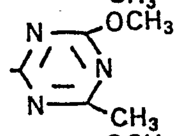 | $CH_3$ | H | |
| (II-90) | 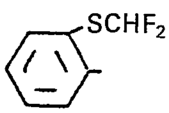 | 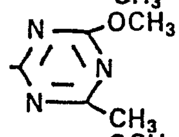 | $CH_3$ | H | |
| (II-91) | 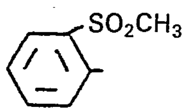 | 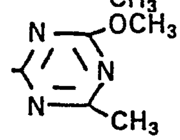 | $CH_3$ | H | |
| (II-92) | 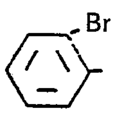 | 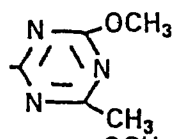 | $CH_3$ | H | |
| (II-93) | 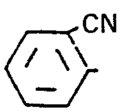 | 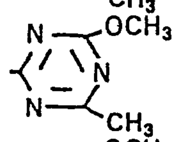 | $CH_3$ | H | |
| (II-94) | 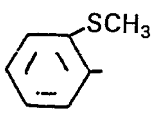 | 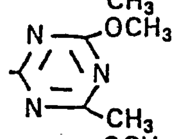 | $CH_3$ | H | |
| (II-95) | 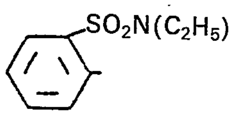 | 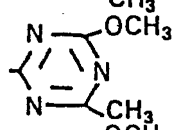 | $CH_3$ | H | |
| (II-96) | 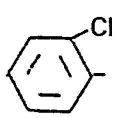 | 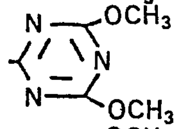 | $CH_3$ | H | |
| (II-97) | 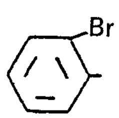 | 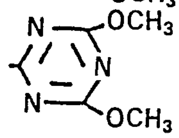 | $CH_3$ | H | |

Tabelle 8 Fortsetzung

| Beisp. Nr. | $R^1=R^{1a}$ | $R^2$ | $R^4$ | M | Schmelz-punkt (°C) |
|---|---|---|---|---|---|
| (II–98) | 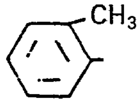 | 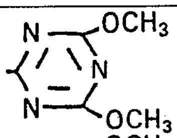 | $CH_3$ | H | |
| (II–99) | 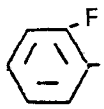 | 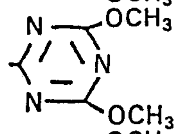 | $CH_3$ | H | |
| (II–100) | 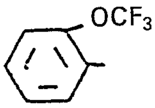 | 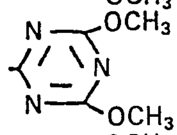 | $CH_3$ | H | |
| (II–101) | 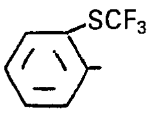 | 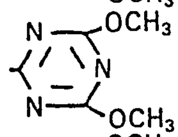 | $CH_3$ | H | |
| (II–102) | 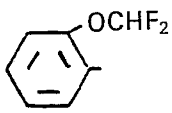 | 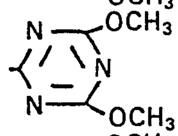 | $CH_3$ | H | |
| (II–103) | 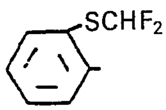 | 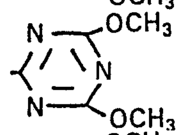 | $CH_3$ | H | |
| (II–104) | 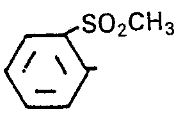 | 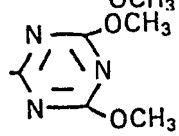 | $CH_3$ | H | |
| (II–105) | 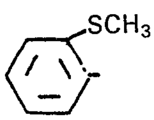 | 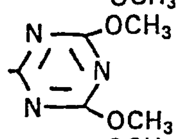 | $CH_3$ | H | |
| (II–106) | 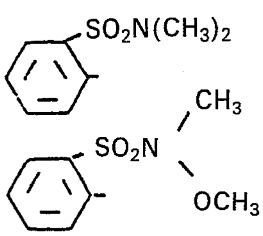 | 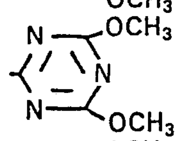 | $CH_3$ | H | |
| (II–107) | | 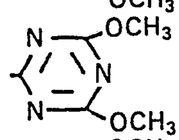 | $CH_3$ | H | |
| (II–108) | 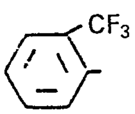 | 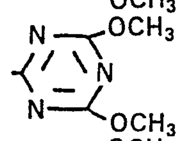 | $CH_3$ | H | |
| (II–109) | 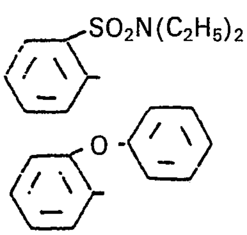 | 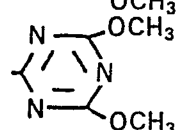 | $CH_3$ | H | |
| (II–110) | | 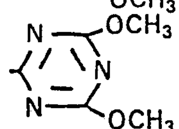 | $CH_3$ | H | |
| (II–111) | 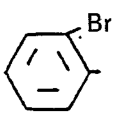 | 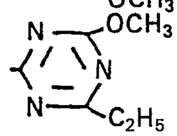 | $CH_3$ | H | |

Tabelle 8

| Beisp. Nr. | $R^1=R^{1a}$ | $R^2$ | $R^4$ | M | Schmelz-punkt (°C) |
|---|---|---|---|---|---|
| (II–112) | 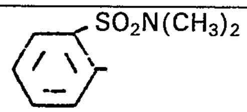 | 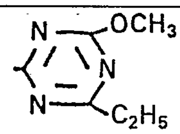 | $CH_3$ | H | |
| (II–113) | 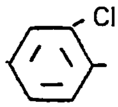 | 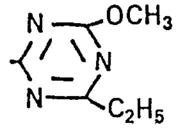 | $CH_3$ | H | amorph |
| (II–114) | 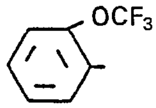 | 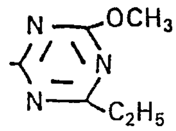 | $CH_3$ | H | |
| (II–115) | 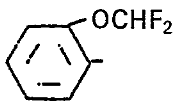 | 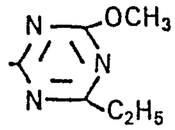 | $CH_3$ | H | |
| (II–116) | 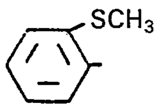 | 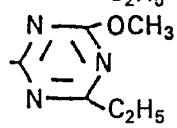 | $CH_3$ | H | |
| (II–117) | 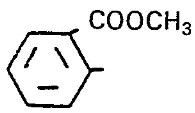 | 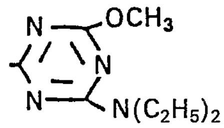 | $CH_3$ | H | amorph |
| (II–118) | 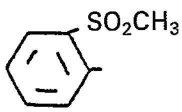 | 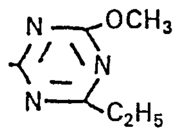 | $CH_3$ | H | |
| (II–119) | 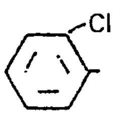 | 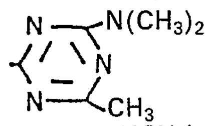 | $CH_3$ | H | 203 (Zers.) |
| (II–120) | 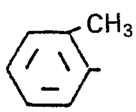 | 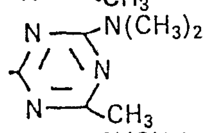 | $CH_3$ | H | |
| (II–121) | 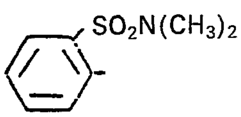 | 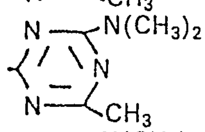 | $CH_3$ | H | |
| (II–122) | 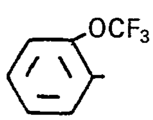 | 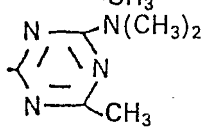 | $CH_3$ | H | |
| (II–123) | 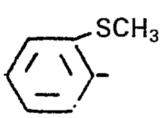 | 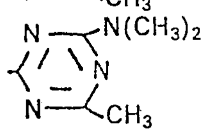 | $CH_3$ | H | |
| (II–124) | 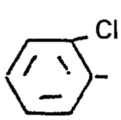 | 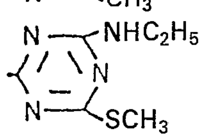 | $CH_3$ | H | amorph |
| (II–125) | 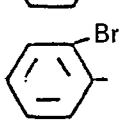 | 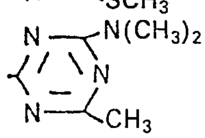 | $CH_3$ | H | |

Tabelle 8 Fortsetzung

| Beisp. Nr. | $R^1=R^{1a}$ | $R^2$ | $R^4$ | M | Schmelz-punkt (°C) |
|---|---|---|---|---|---|
| (II–126) | 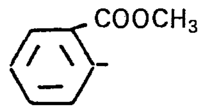 | 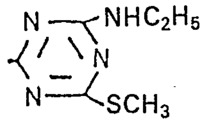 | $CH_3$ | H | amorph |
| (II–127) | 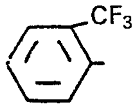 | 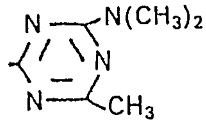 | $CH_3$ | H | |
| (II–128) | 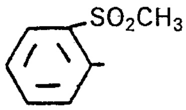 | 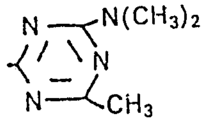 | $CH_3$ | H | |
| (II–129) | 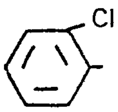 | 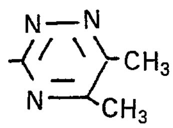 | $CH_3$ | H | 179 |
| (II–130) | 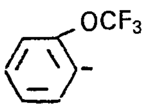 | 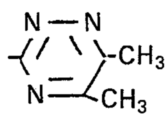 | $CH_3$ | H | |
| (II–131) | 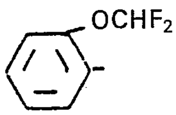 | 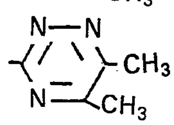 | $CH_3$ | H | |
| (II–132) | 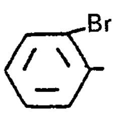 | 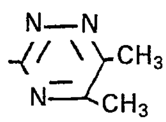 | $CH_3$ | H | |
| (II–133) | 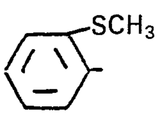 | 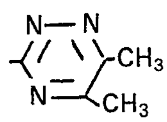 | $CH_3$ | H | |
| (II–134) | 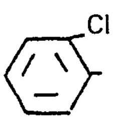 | 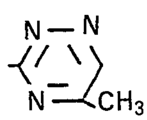 | $CH_3$ | H | |
| (II–135) | 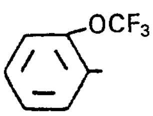 | 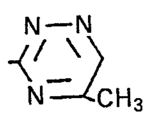 | $CH_3$ | H | |
| (II–136) | 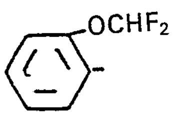 | 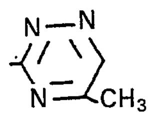 | $CH_3$ | H | |
| (II–137) | 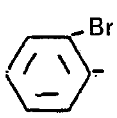 | 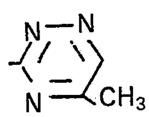 | $CH_3$ | H | |
| (II–138) | 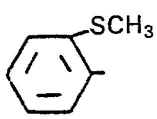 | 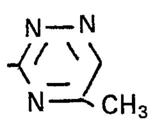 | $CH_3$ | H | |
| (II–139) | 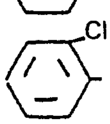 | 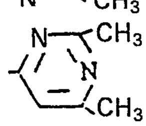 | $CH_3$ | H | |

Tabelle 8 Fortsetzung

| Beisp. Nr. | $R^1 = R^{1a}$ | $R^2$ | $R^4$ | M | Schmelz-punkt (°C) |
|---|---|---|---|---|---|
| (II–140) | 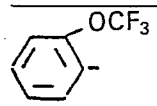 | 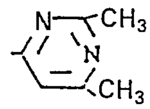 | $CH_3$ | H | |
| (II–141) | 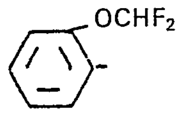 | 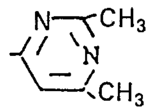 | $CH_3$ | H | |
| (II–142) | 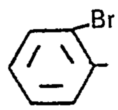 | 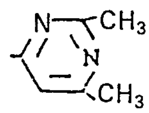 | $CH_3$ | H | |
| (II–143) | 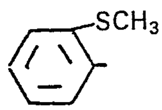 | 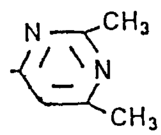 | $CH_3$ | H | |
| (II–144) | 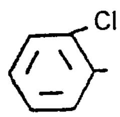 | 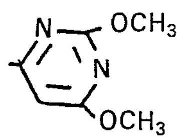 | $CH_3$ | H | |
| (II–145) | 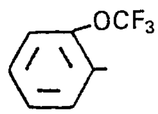 | 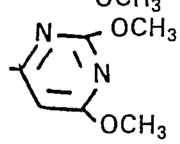 | $CH_3$ | H | |
| (II–146) | 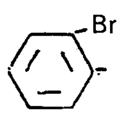 | 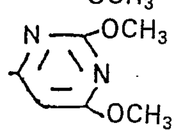 | $CH_3$ | H | |
| (II–147) | 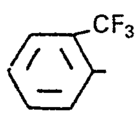 | 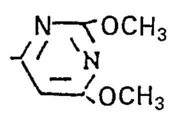 | $CH_3$ | H | |
| (II–148) | 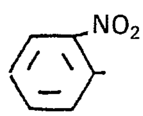 | 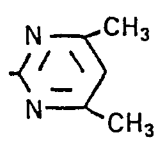 | $CH_3$ | H | 65 |
| (II–149) | 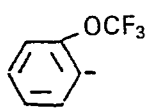 | 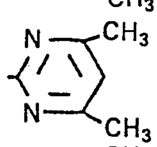 | $C_2H_5$ | H | 163 |
| (II–150) | 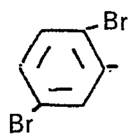 | 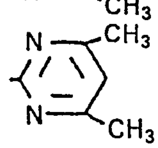 | $CH_3$ | H | 115 |
| (II–151) | 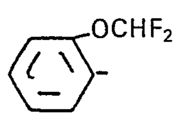 | 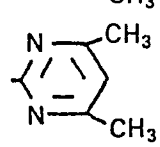 | $C_2H_5$ | H | 159–160 |
| (II–152) | 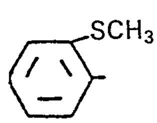 | 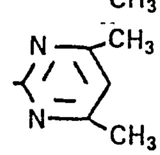 | $CH_3$ | H | 182–183 |

Tabelle 8 Fortsetzung

| Beisp. Nr. | $R^1 = R^{1a}$ | $R^2$ | $R^4$ | M | Schmelz-punkt (°C) |
|---|---|---|---|---|---|
| (II-153) | 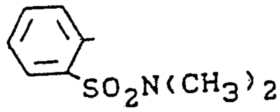 | 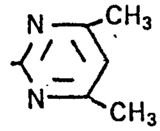 | $C_4H_9$(-s) | H | 149 |
| (II-154) | 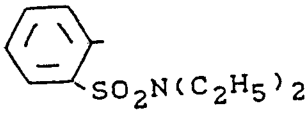 | 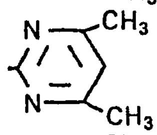 | $CH_3$ | H | 141 |
| (II-155) | 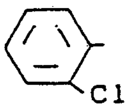 | 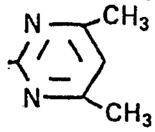 | $C_3H_7$(-n) | H | 160 |
| (II-156) | 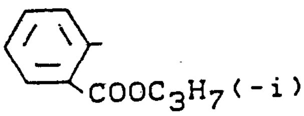 | 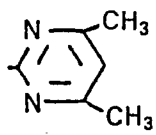 | $CH_3$ | H | 65 |
| (II-157) | 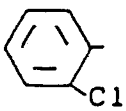 | 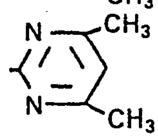 | $C_3H_7$(-i) | H | 185 |
| (II-158) | 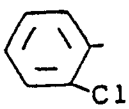 | 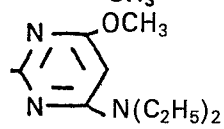 | $CH_3$ | H | 133 |
| (II-159) | 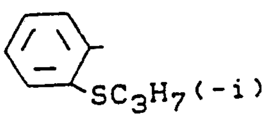 | 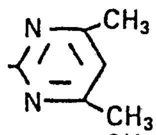 | $CH_3$ | H | amorph |
| (II-160) | 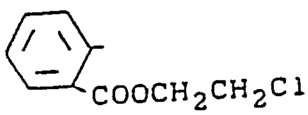 | 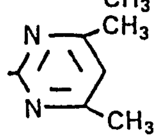 | $CH_3$ | H | 136 |
| (II-161) | 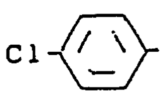 | 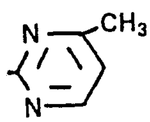 | $CH_3$ | H | 148 |
| (II-162) | 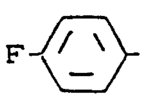 | 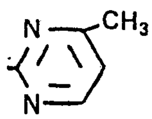 | $CH_3$ | H | 135 |
| (II-163) | 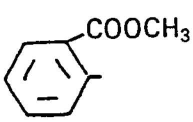 | 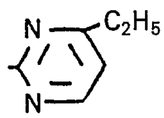 | $CH_3$ | H | 141 |
| (II-164) | 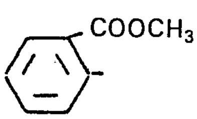 | 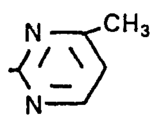 | $C_2H_5$ | H | 135 |
| (II-165) | 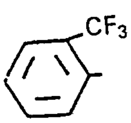 | 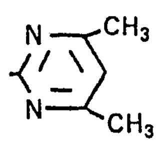 | $C_2H_5$ | H | 148 |

Tabelle 8 Fortsetzung

| Beisp. Nr. | $R^1=R^{1a}$ | $R^2$ | $R^4$ | M | Schmelzpunkt (°C) |
|---|---|---|---|---|---|
| (II–166) | 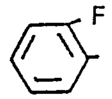 | 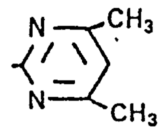 | $C_2H_5$ | H | 140 |
| (II–167) | 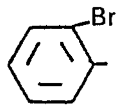 | 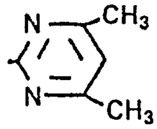 | $C_2H_5$ | H | 166 |
| (II–168) | 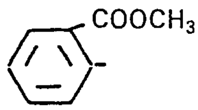 | 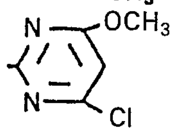 | $CH_3$ | H | 159 |
| (II–169) | 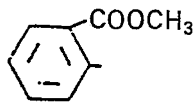 | 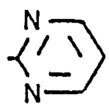 | $CH_3$ | H | 144–147 |
| (II–170) | 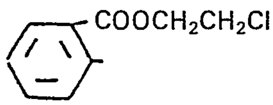 | 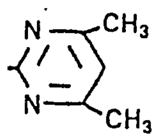 | $C_2H_5$ | H | 142 |
| (II–171) | 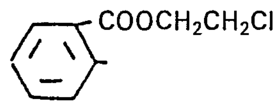 | 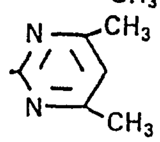 | $C_3H_7$(-n) | H | 104–106 |

Tabelle 9

| Beisp. Nr. | $R^1$ | $R^{1a}$ | $R^2$ | $R^4$ | M | Schmelz-punkt (°C) |
|---|---|---|---|---|---|---|
| (II–172) | 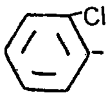 | 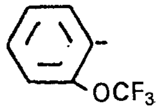 | 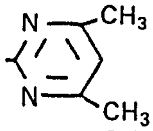 | $CH_3$ | H | 160 |
| (II–173) | 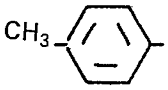 | 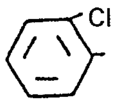 | 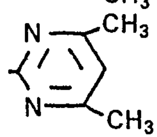 | $CH_3$ | H | 85 |
| (II–174) | 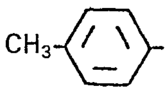 | 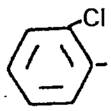 | 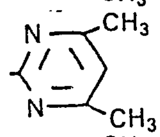 | $CH_2$- 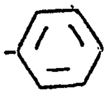 | H | 168 |
| (II–175) | 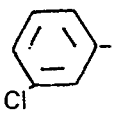 | 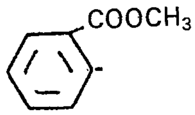 | 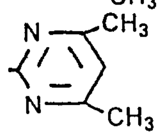 | $CH_3$ | H | 158 |
| (II–176) | 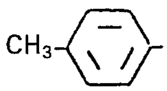 | 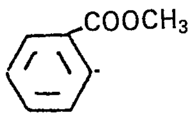 | 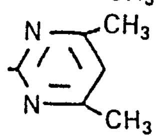 | $CH_3$ | H | 150 |
| (II–177) | 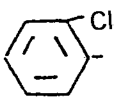 | 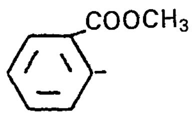 | 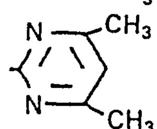 | $CH_3$ | H | 149 |
| (II–178) | 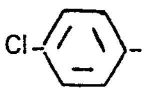 | 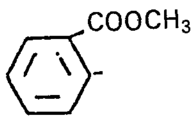 | 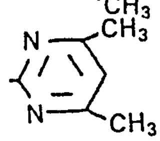 | $CH_3$ | H | 145 |
| (II–179) | 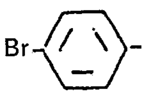 | 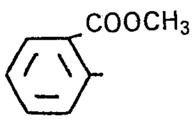 | 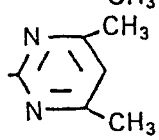 | $CH_3$ | H | 153 |
| (II–180) | 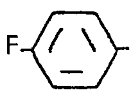 | 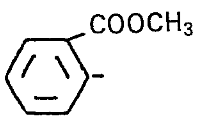 | 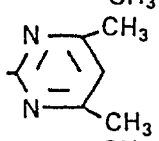 | $CH_3$ | H | 155 |
| (II–181) | 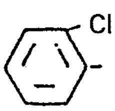 | 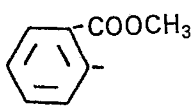 | 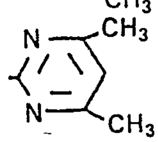 | $CH_3$ | H | 160 |
| (II–182) | 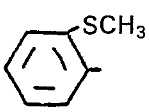 | 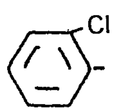 | 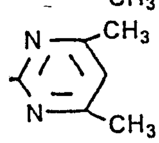 | $-CH_2$- 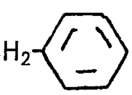 | H | 156 |
| (II–183) | 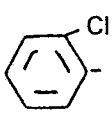 | 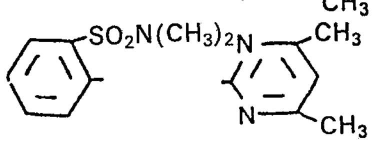 | | $CH_2CH(CH_3)_2$ | H | 156 |
| (II–184) | 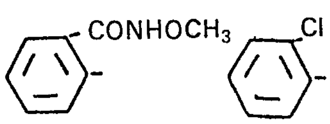 | | 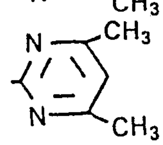 | $CH_3$ | H | amorph |

Tabelle 9 Fortsetzung

| Beisp. Nr. | $R^1$ | $R^{1a}$ | $R^2$ | $R^4$ | M | Schmelz-punkt (°C) |
|---|---|---|---|---|---|---|
| (II–185) | 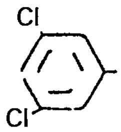 | 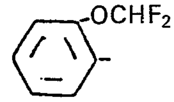 | 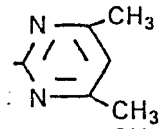 | $CH_3$ | H | 171 |
| (II–186) | 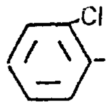 | 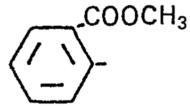 | 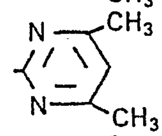 | $CH_2CH(CH_3)_2$ | H | 156 |
| (II–187) | 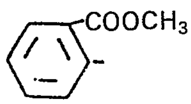 | 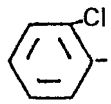 | 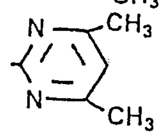 | $CH_2CH(CH_3)_2$ | H | 119 |
| (II–188) | 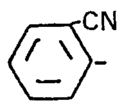 | 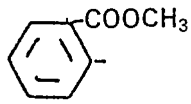 | 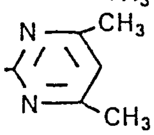 | $CH_3$ | H | 170 |
| (II–189) | 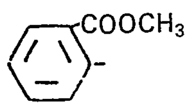 | 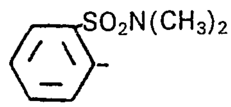 | 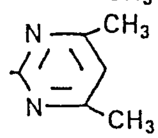 | $CH_2CH(CH_3)_2$ | H | 157 |
| (II–190) | 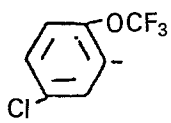 | 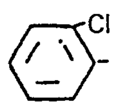 | 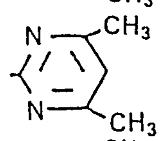 | $CH_2$-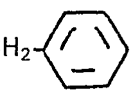 | H | amorph |
| (II–191) | 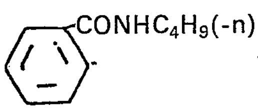 | 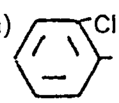 | 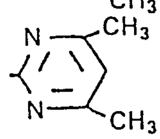 | $CH_3$ | H | amorph |
| (II–192) | 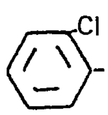 | 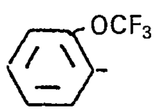 | 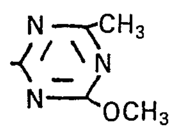 | $CH_3$ | H | 172 |
| (II–193) | 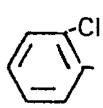 | 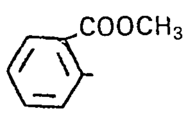 | 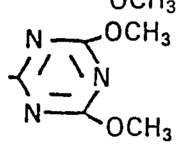 | $CH_3$ | H | 105 |
| (II–194) | 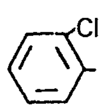 | 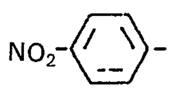 | 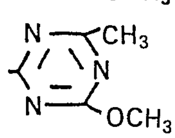 | $CH_3$ | H | 207 |
| (II–195) | 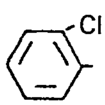 | 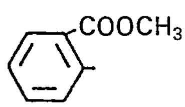 | 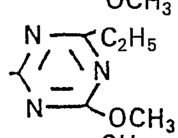 | $CH_3$ | H | amorph |
| (II–196) | 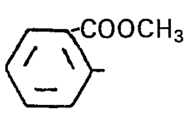 | 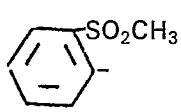 | 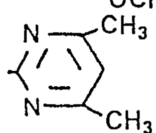 | $CH_3$ | H | 173 |
| (II–197) | 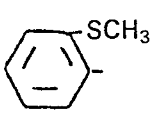 | 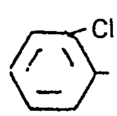 | 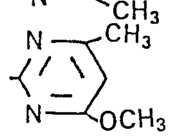 | $CH_3$ | H | amorph |

Tabelle 9 Fortsetzung

| Beisp. Nr. | $R^1$ | $R^{1a}$ | $R^2$ | $R^4$ | M | Schmelzpunkt (°C) |
|---|---|---|---|---|---|---|
| (II–198) | 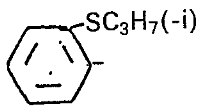 | 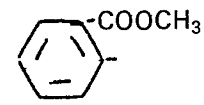 | 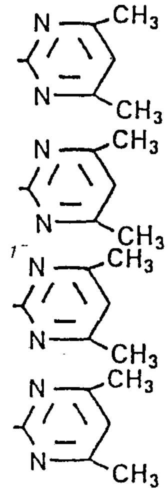 | $CH_3$ | H | 145 |
| (II–199) | 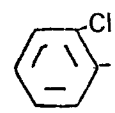 | 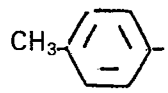 | | $-C_4H_9(-n)$ | H | amorph |
| (II–200) | 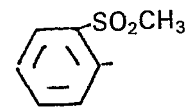 | 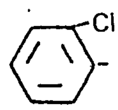 | | 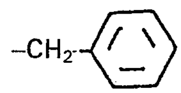 | H | 174 |
| (II–201) | 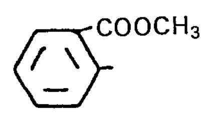 | 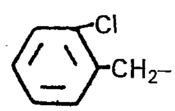 | | $-CH_3$ | H | 77–79 |

Herstellung von Ausgangsstoffen der Formel (IV)

Beispiel (IV-1)

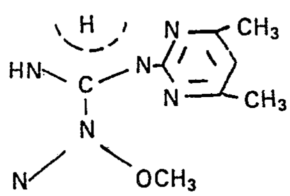

Eine Mischung aus 109 g (0,67 Mol) O-Methylhydroxylamin-Hydrochlorid, 99 g (0,67 Mol) 2-Cyanamino-4,6-dimethylpyrimidin und 600 ml Ethanol wird 7 Stunden unter Rückfluss zum Sieden erhitzt. Anschliessend wird der Alkohol im Wasserstrahlvakuum abdestilliert, der Rückstand in heissem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55% der Theorie) N'-(4,6-Dimethylpyrimidin-2-yl)- N''-methoxy-guanidin vom Schmelzpunkt 134 °C bis 136 °C.

Analog können die in der nachstehenden Tabelle 10 aufgeführten Verbindungen der Formel (IV) hergestellt werden:

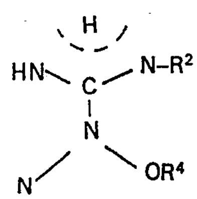

(IV)

Tabelle 10

| Beisp. Nr. | $R^4$ | $R^2$ | Schmelzpunkt (°C) |
|---|---|---|---|
| (IV–2) | $-CH_2CH(CH_3)_2$ | 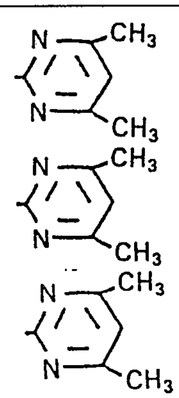 | 78 |
| (IV–3) | $-CH(CH_3)_2$ | | 84 |
| (IV–4) | $-CH(CH_3)-CH_2CH_3$ | | 52 |

Tabelle 10 Fortsetzung

| Beisp. Nr. | $R^4$ | $R^2$ | Schmelzpunkt (°C) |
|---|---|---|---|
| (IV–5) | $-C_4H_9(-n)$ | 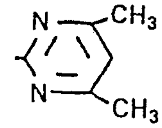 | öl |
| (IV–6) | $-C_3H_7(-n)$ | 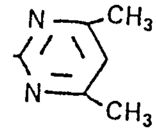 | öl |
| (IV–7) | $-CH_2-$ 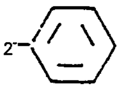 | 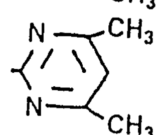 | 102 |
| (IV–8) | $-C_2H_5$ | 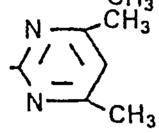 | 88 |
| (IV–9) | $-CH_3$ | 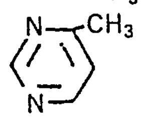 | 152 |
| (IV–10) | $-CH_3$ | 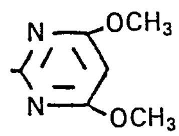 | 122 |
| (IV–11) | $-CH_3$ | 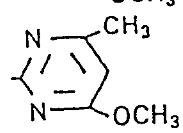 | 126 |
| (IV–12) | $-CH_3$ | 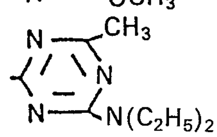 | 112 |
| (IV–13) | $-CH_3$ | 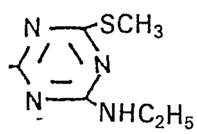 | 117 |
| (IV–14) | $-CH_2-CH(CH_3)_2$ | 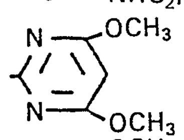 | 76 |
| (IV–15) | $-CH-CH_2CH_3$<br>$CH_3$ | 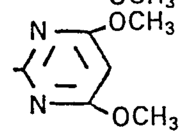 | 68 |
| (IV–16) | $-C_2H_5$ | 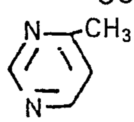 | 95 |
| (IV–17) | $-CH_3$ | 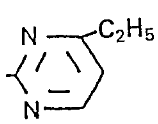 | 98 |

Tabelle 10 Fortsetzung

| Beisp. Nr. | $R^4$ | $R^2$ | Schmelzpunkt (°C) |
|---|---|---|---|
| (IV–18) | $-CH_3$ | 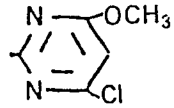 | 112 |
| (IV–19) | $-CH_3$ | 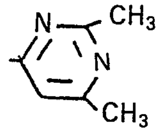 | 143 |
| (IV–20) | $-CH_3$ | 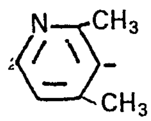 | 110 |
| (IV–21) | $-CH_2-$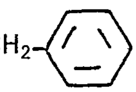 | 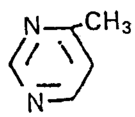 | 150 |
| (IV–22) | $-C_4H_9$(-n) | 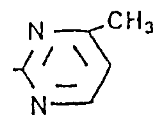 | |
| (IV–23) | $-C_4H_9$(-s) | 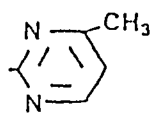 | |
| (IV–24) | $-CH_3$ | 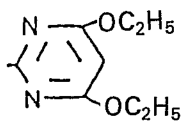 | |
| (IV–25) | $-CH_3$ | 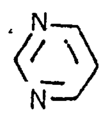 | 107–109 |
| (IV–26) | $-CH_2-$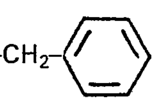 | 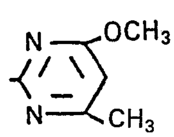 | $n_D^{20} = 1.5645$ |
| (IV–27) | $-CH_2-$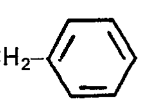 | 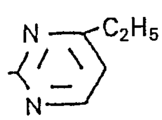 | 112 |
| (IV–28) | $-CH_2-$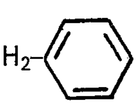 | 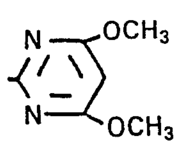 | 74 |

Tabelle 10 Fortsetzung

| Beisp. Nr. | $R^4$ | $R^2$ | Schmelz-punkt (°C) |
|---|---|---|---|
| (IV-29) | $-CH_2$ 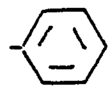 | 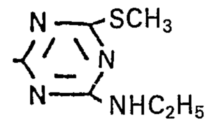 | 122 |
| (IV-30) | $-CH_2$ 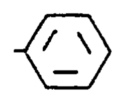 | 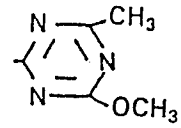 | 112 |
| (IV-31) | $-CH_3$ | 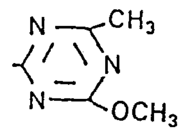 | 126 |
| (IV-32) | $-CH_2$ 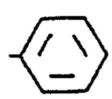 | 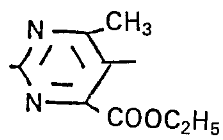 | 130 |

Herstellung der Ausgangsstoffe der Formel (XIII)

Beispiel (XIII-1)

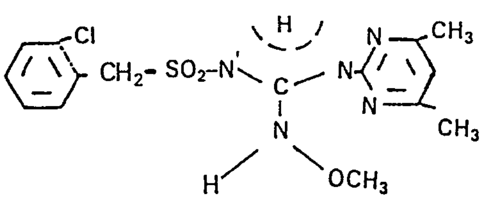

Eine Lösung von 16,8 g (0,15 Mol) Diazabicyclooctan in 50 ml Methylenchlorid wird zu einer auf 0 °C gekühlten Mischung aus 9,8 g (0,05 Mol) N'- (4,6-Dimethyl-pyrimidin- 2-yl)-N''-methoxy-guanidin, 28,2 g (0,12 Mol) 2-Chlorphenyl-methansulfonsäurechlorid und 50 ml Methylenchlorid unter Rühren tropfenweise gegeben. Das Reaktionsgemisch wird 2 Stunden bei 10 °C und weiter 15 Stunden bei 20 °C gerührt. Anschliessend werden 50 ml 2N Salzsäure dazu gegeben, nach Durchschütteln die organische Phase abgetrennt, mit Wasser gewaschen, getrocknet, filtriert und eingeengt. Der ölige Rückstand wird mit Isopropanol zur Kristallisation gebracht.

Man erhält 3,0 g (12% der Theorie) N'-(4,6-Dimethylpyrimidin-2-yl)-N''-methoxy-N'''-(2-chlorbenzylsulfonyl)-guanidin vom Schmelzpunkt 130 °C.

Beispiel (XIII-2)

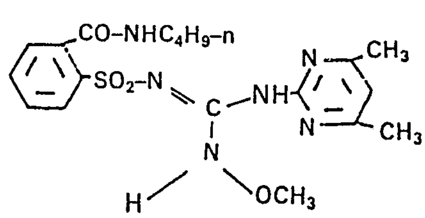

4,0 g (0,025 Mol) Butylamin werden bei 20 °C–30 °C zu einer Mischung aus 9,1 g (0,025 Mol) der Verbindung der nachstehenden Formel

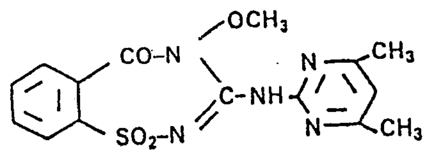

sowie 60 ml Diethylether und 5 ml Dioxan unter Rühren gegeben. Das Reaktionsgemisch wird 15 Stunden bei 20 °C gerührt und das kristallin angefallene Produkt durch Absaugen isoliert.

Man erhält 8,1 g (75% der Theorie) N'-(4,6-Dimethylpyrimidin-2-yl)-N''-methoxy-N'''-(2-butylaminocarbonyl-benzolsulfonyl)-guanidin vom Schmelzpunkt 169 °C.

Beispiel (XIII-3)

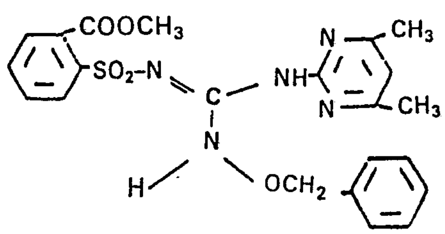

Eine Mischung aus 8,8 g (0,02 Mol) der Verbindung nachstehender Formel

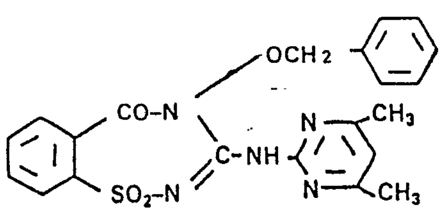

sowie 2,2 g (0,04 Mol) Natrium-methylat und 70 ml Methanol wird 8 Stunden unter Rückfluss zum Sieden erhitzt. Nach Abkühlen auf −10 °C wird abgesaugt, der Feststoff in 200 ml Wasser gelöst, die Lösung filtriert und das Filtrat mit konz. Salzsäure angesäuert. Das kristallin angefallene Produkt wird durch Absaugen isoliert.

Man erhält 3,2 g (34% der Theorie) N'-(4,6-Dimethylpyrimidin-2-yl)- N''-benzyloxy- N'''-(2-methoxycarbonylbenzolsulfonyl)-guanidin vom Schmelzpunkt 128 °C.

Beispiel (XIII-4)

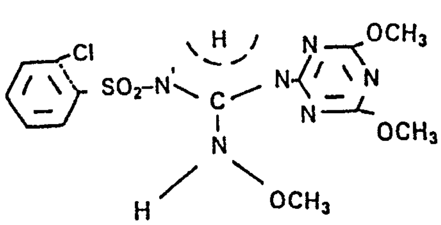

Eine Mischung aus 10 g (0,026 Mol) N'-(4,6-Dimethoxy-s-triazin- 2-yl) -N''-(2-chlor-benzolsulfonyl) -S-methyl-isothioharnstoff, 3,0 g (0,065 Mol) O-Methyl-hydroxylamin und 80 ml Dioxan wird 60 Stunden bei 20 °C gerührt. Dann wird eingeengt, der Rückstand mit Ethanol verrieben und das kristallin angefallene Produkt durch Absaugen isoliert.

Man erhält 5,8 g (57% der Theorie) N'-(4,6-Dimethoxy-s-triazin-2-yl)- N''-methoxy- N'''-(2-chlor-benzolsulfonyl)-guanidin vom Schmelzpunkt 150 °C.

Beispiel (XIII-5)

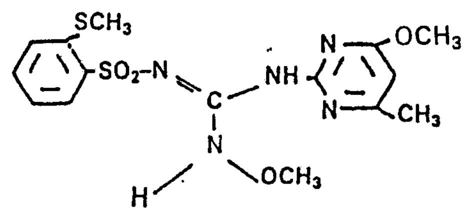

Eine Mischung aus 16,0 g (0,14 Mol) N'-(4-Methoxy-6-methyl-pyrimidin-2-yl) -N''-(2-methylthio-benzolsulfonyl) -S-methyl-isothioharnstoff, 4,0 g (0,085 Mol) O-Methylhydroxylamin und 80 ml Dioxan wird 15 Stunden bei 30 °C bis 33 °C gerührt. Anschliessend wird eingeengt, der Rückstand durch Verreiben mit Ethanol zur Kristallisation gebracht und das Produkt durch Absaugen isoliert.

Man erhält 8,4 g (53% der Theorie) N'-(4-Methoxy-6- methyl-pyrimidin- 2-yl)- N''-methoxy-N'''-(2-methylthio-benzolsulfonyl)-guanidin vom Schmelzpunkt 134 °C.

Analog Beispiel (XIII-1) bis Beispiel (XIII-6) können die in den nachstehenden Tabellen 11 und 12 aufgeführten Verbindungen der Formeln (XIIIa) bzw. (XIII) hergestellt werden:

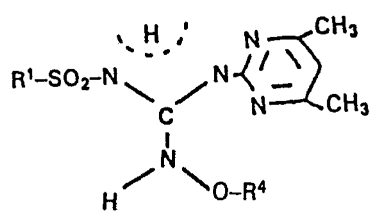

(XIIIa)

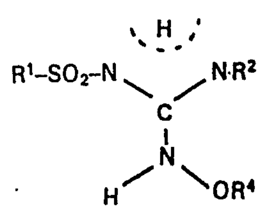

(XIII)

Tabelle 11 Verbindungen der Formel (XIIIa)

| Beisp. Nr. | $R^1$ | $R^4$ | Schmelz-punkt (°C) |
|---|---|---|---|
| (XIIIa–1) | 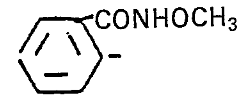 | $-CH_3$ | 151 |
| (XIIIa–2) | 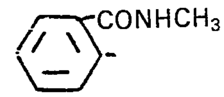 | $-CH_3$ | 177 |
| (XIIIa–3) | 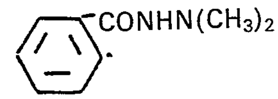 | $-CH_3$ | 178 |
| (XIIIa–4) | 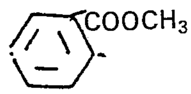 | $-CH_3$ | 132 |
| (XIIIa–5) | 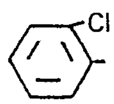 | $-CH_3$ | 142 |
| (XIIIa–6) | 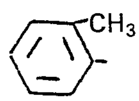 | $-CH_3$ | 114 |
| (XIIIa–7) | 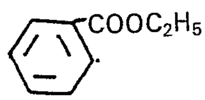 | $-CH_3$ | 125 |
| (XIIIa–8) | 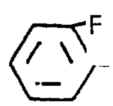 | $-CH_3$ | 160 |
| (XIIIa–9) | 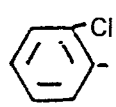 | $-CH_2-$ 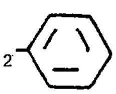 | 169 |
| (XIIIa–10) | 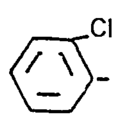 | $-C_4H_9(-n)$ | 116 |
| (XIIIa–11) | 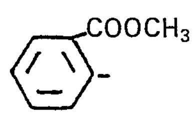 | $-C_4H_9(-n)$ | 95 |
| (XIIIa–12) | 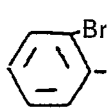 | $-CH_3$ | 166 |
| (XIIIa–13) | 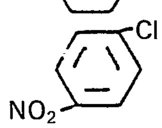 | $-CH_3$ | 160 |
| (XIIIa–14) | 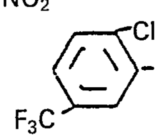 | $-CH_3$ | 134 |

Tabelle 11 Fortsetzung

| Beisp. Nr. | $R^1$ | $R^4$ | Schmelzpunkt (°C) |
|---|---|---|---|
| (XIIIa-15) | 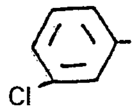 | $-CH_3$ | |
| (XIIIa-16) | 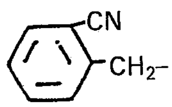 | $-CH_3$ | 166–168 |
| (XIIIa-17) | 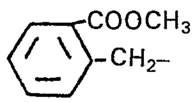 | $-CH_3$ | 117–125 |
| (XIIIa-18) | 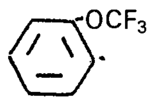 | $-CH_3$ | 128 |
| (XIIIa-19) | 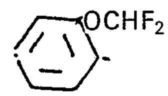 | $-CH_3$ | 138 |
| (XIIIa-20) | 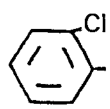 | $-CH_2-CH(CH_3)_2$ | 105 |
| (XIIIa-21) | 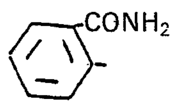 | $-CH_3$ | 222 |
| (XIIIa-22) | 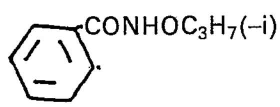 | $-CH_3$ | 178 |
| (XIIIa-23) | 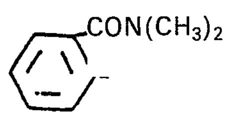 | $-CH_3$ | 153 |
| (XIIIa-24) | 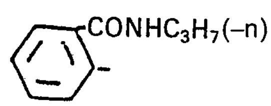 | $-CH_3$ | 138 |
| (XIIIa-25) | 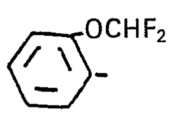 | 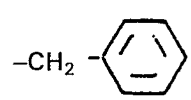 | 242 (Zers.) |
| (XIIIa-26) | 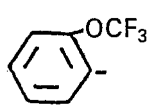 | $-C_2H_5$ | 115 |
| (XIIIa-27) | 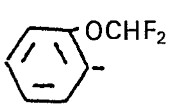 | $-C_2H_5$ | 136 |

Tabelle 11 Fortsetzung

| Beisp. Nr. | $R^1$ | $R^4$ | Schmelz-punkt (°C) |
|---|---|---|---|
| (XIIIa-28) | 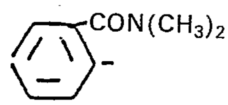 | $-C_2H_5$ | ~148 |
| (XIIIa-29) | 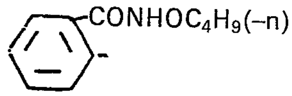 | 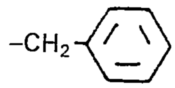 | öl |
| (XIIIa-30) | 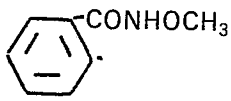 | $C_4H_9(-n)$ | öl |
| (XIIIa-31) | 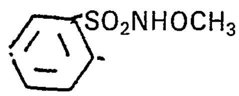 | 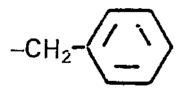 | 154 |
| (XIIIa-32) | 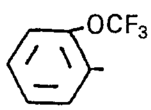 | 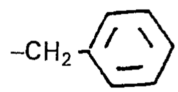 | 141 |
| (XIIIa-33) | 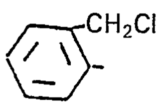 | $-CH_3$ | 101–103 |

Tabelle 12 Verbindungen der Formel (XIII)

| Beisp. Nr. | $R^1$ | $R^2$ | $R^4$ | Schmelz-punkt (°C) |
|---|---|---|---|---|
| (XIII-6) | 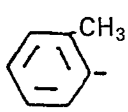 | 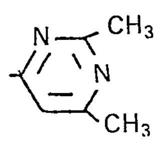 | $-CH_3$ | 148 |
| (XIII-7) | 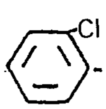 | 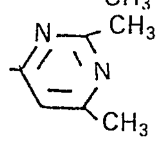 | $-CH_3$ | 135 |
| (XIII-8) | 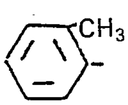 | 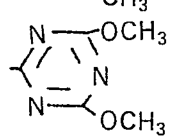 | $-CH_3$ | 105 |
| (XIII-9) | 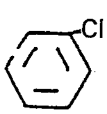 | 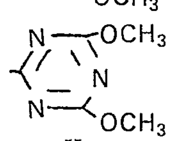 | $-CH_3$ | |
| (XIII-10) | 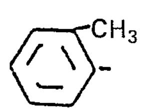 | 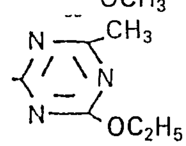 | $-CH_3$ | 144 |

Tabelle 12 Fortsetzung

| Beisp. Nr. | $R^1$ | $R^2$ | $R^4$ | Schmelzpunkt (°C) |
|---|---|---|---|---|
| (XIII-11) | 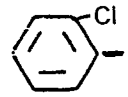 | 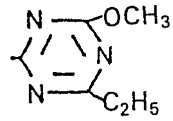 | $-CH_3$ | 115 |
| (XIII-12) | 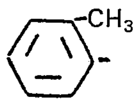 | 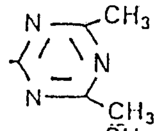 | $-CH_3$ | 132 |
| (XIII-13) | 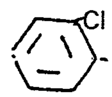 | 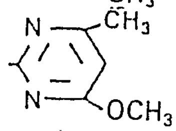 | $-CH_3$ | 138 |
| (XIII-14) | 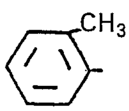 | 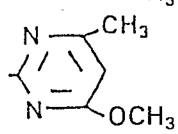 | $-CH_3$ | 108 |
| (XIII-15) | 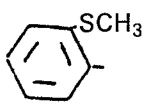 | 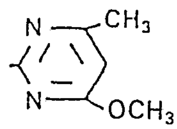 | $-CH_3$ | 134 |
| (XIII-16) | 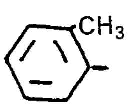 | 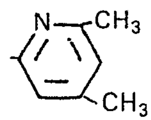 | $-CH_3$ | 91 |
| (XIII-17) | 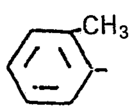 | 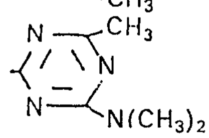 | $-CH_3$ | 105 |
| (XIII-18) | 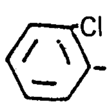 | 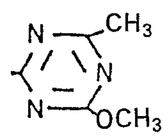 | $-CH_3$ | 122 |
| (XIII-19) | 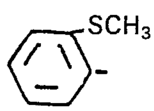 | 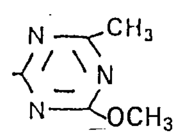 | $-CH_3$ | 132 |
| (XIII-20) | 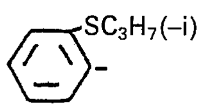 | 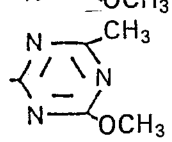 | $-CH_3$ | 164 |
| (XIII-21) | 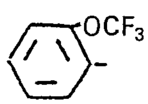 | 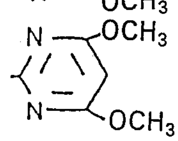 | $-CH_3$ | 137 |
| (XIII-22) | 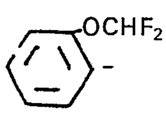 | 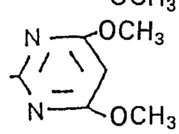 | $-CH_3$ | 135 |
| (XIII-23) | 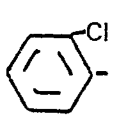 | 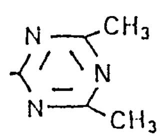 | $-CH_3$ | 122 |

Tabelle 12 Fortsetzung

| Beisp. Nr. | $R^1$ | $R^2$ | $R^4$ | Schmelzpunkt (°C) |
|---|---|---|---|---|
| (XIII–24) | 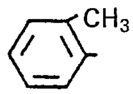 | 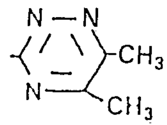 | $-CH_3$ | 119 |
| (XIII–25) | 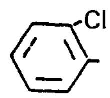 | 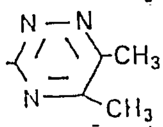 | $-CH_3$ | 165 |
| (XIII–26) | 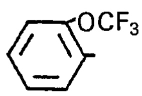 | 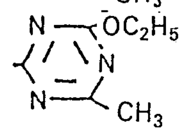 | 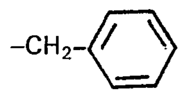 | 136 |

Herstellung von Ausgangsstoffen der Formel (XV)

Beispiel (XV-1)

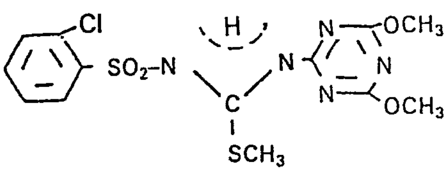

11 g (0,4 Mol) Natriumhydrid (80%ig) werden bei 20 °C portionsweise zu einer Suspension von 31,2 g (0,2 Mol) 2-Amino-4,6-dimethoxy-striazin in 200 ml Tetrahydrofuran gegeben. Nach zwölfstündigem Rühren werden 60 g (0,2 Mol) N-(2-Chlor-benzolsulfonyl)-S',S''-dimethyl-iminodithiokohlensäureester dazu gegeben, wobei die Reaktionstemperatur auf 60 °C ansteigt. Das Reaktionsgemisch wird 5 Stunden bei 20 °C gerührt, mit 800 ml Wasser verdünnt und filtriert. Nach Ansäuern mit konzentrierter Salzsäure kristallisiert das Produkt und wird durch Absaugen isoliert.

Man erhält 42 g (48% der Theorie) N'-(4,6-Dimethoxy-s-triazin-2-yl)-N''-(2-chlor-benzolsulfonyl)-S-methyl-isothioharnstoff vom Schmelzpunkt 176 °C.

Analog Beispiel (XV-1) können die in der nachstehenden Tabelle 13 aufgeführten Verbindungen der Formel (XV) hergestellt werden:

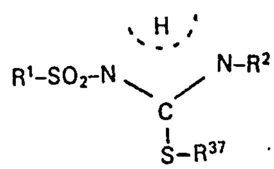

(XV)

Tabelle 13

| Beisp. Nr. | $R^1$ | $R^2$ | $R^{37}$ | Schmelzpunkt (°C) |
|---|---|---|---|---|
| (XV–2) | 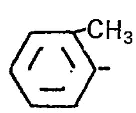 | 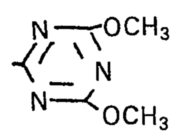 | $CH_3$ | 159 |
| (XV–3) | 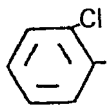 | 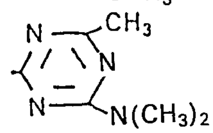 | $CH_3$ | 175 |
| (XV–4) | 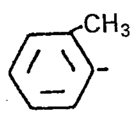 | 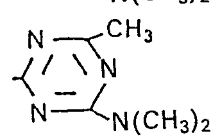 | $CH_3$ | 157 |

Tabelle 13 Fortsetzung

| Beisp. Nr. | $R^1$ | $R^2$ | $R^{37}$ | Schmelzpunkt (°C) |
|---|---|---|---|---|
| (XV–5) | 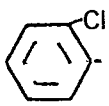 | 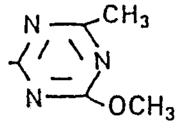 | $CH_3$ | 148 |
| (XV–6) | 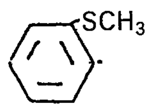 | 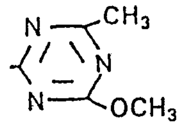 | $CH_3$ | 167 |
| (XV–7) | 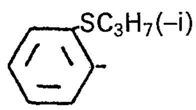 | 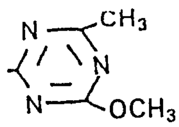 | $CH_3$ | 136 |
| (XV–8) | 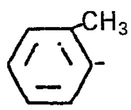 | 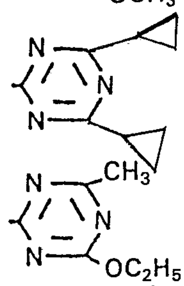 | $CH_3$ | 170 |
| (XV–9) | 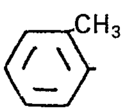 | | $CH_3$ | 104 |
| (XV–10) | 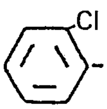 | 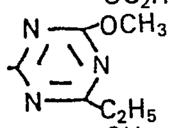 | $CH_3$ | 132 |
| (XV–11) | 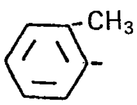 | 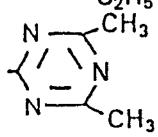 | $CH_3$ | 119 |
| (XV–12) | 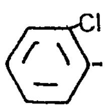 | 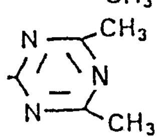 | $CH_3$ | 151 |
| (XV–13) | 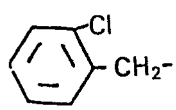 | 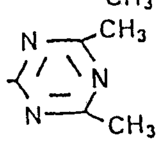 | $CH_3$ | 179 |
| (XV–14) | 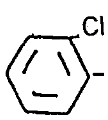 | 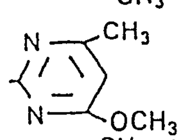 | $CH_3$ | 148 |
| (XV–15) | 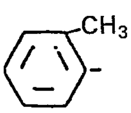 | 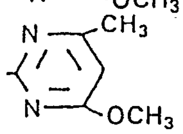 | $CH_3$ | 163 |
| (XV–16) | 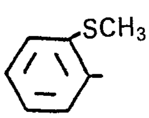 | 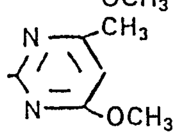 | $CH_3$ | 163 |
| (XV–17) | 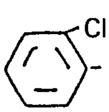 | 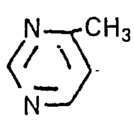 | $CH_3$ | 152–153 |

Tabelle 13 Fortsetzung

| Beisp. Nr. | $R^1$ | $R^2$ | $R^{37}$ | Schmelz-punkt (°C) |
|---|---|---|---|---|
| (XV–18) | 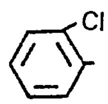 | 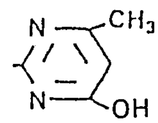 | $CH_3$ | 277 (Zers.) |
| (XV–19) | 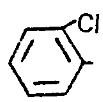 | 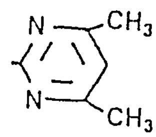 | $CH_3$ | 157 |
| (XV–20) | 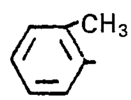 | 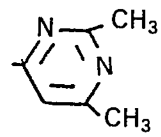 | $CH_3$ | 175 |
| (XV–21) | 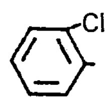 | 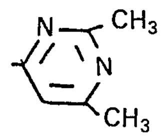 | $CH_3$ | 145 |
| (XV–22) | 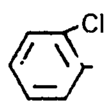 | 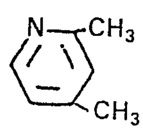 | $CH_3$ | 118 |
| (XV–23) | 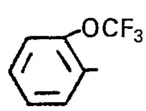 | 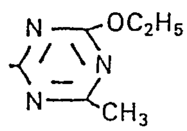 | $CH_3$ | 103 |
| (XV–24) | 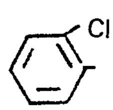 | 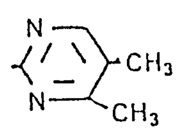 | $CH_3$ | 133 |

Herstellung von Verbindungen der Formel (XVI)

Beispiel (XVI-1)

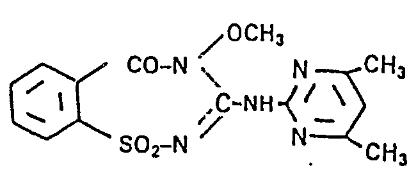

Eine Lösung von 60 g (0,25 Mol) 2-Chlorcarbonyl-benzolsulfonsäurechlorid in 100 ml Methylenchlorid wird zu einer auf −10 °C gekühlten Mischung aus 49 g (0,25 Mol) N'-(4,6-Dimethyl-pyrimidin-2-yl)-N"methoxy-guanidin, 50 g (0,63 Mol) Pyridin und 200 ml Methylenchlorid unter Rühren tropfenweise gegeben. Das Reaktionsgemisch wird 3 Stunden bei 20 °C gerührt, dann zweimal mit je 200 ml 5%iger Salzsäure und einmal mit 200 ml Wasser gewaschen, getrocknet, filtriert und eingeengt. Der Rückstand wird mit Methanol digeriert und das kristallin angefallene Produkt durch Absaugen isoliert.

Man erhält 65 g (72% der Theorie) der Verbindung der oben angegebenen Strukturformel vom Schmelzpunkt 185 °C.

Analog Beispiel (XVI-1) können die in der nachfolgenden Tabelle 14 aufgeführten Verbindungen der Formel (XVI) hergestellt werden:

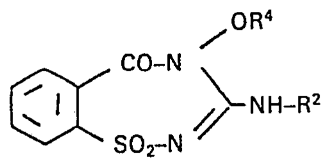

(XVI)

Tabelle 14

| Beisp. Nr. | $R^2$ | $R^4$ | Schmelzpunkt (°C) |
|---|---|---|---|
| (XVI-2) | 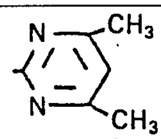 | 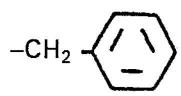 | 162 |
| (XVI-3) | 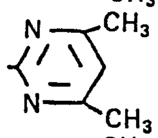 | $-C_4H_9(-n)$ | amorph |
| (XVI-4) | 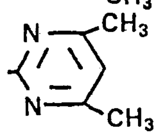 | $-C_3H_7(-n)$ | 71–78 |
| (XVI-5) | 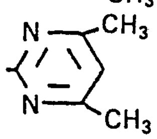 | $-C_2H_5$ | 181–183 |
| (XVI-6) | 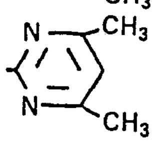 | $-C_3H_7(-i)$ | 155 |
| (XVI-7) | 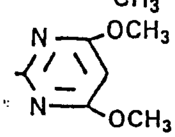 | $-CH_3$ | 180 |

Herstellung von Ausgangsstoffen der Formel (XVIII)

Beispiel (XVIII-1)

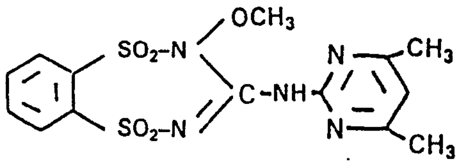

14 g (0,05 Mol) Benzol-1,2-disulfonsäuredichlorid werden portionsweise bei −20 °C zu einer Mischung aus 10 g (0,05 Mol) N'-(4,6-Dimethyl-pyrimidin- 2-yl)- N"-methoxyguanidin, 12 g (0,15 Mol) Pyridin und 100 ml Methylenchlorid gegeben. Man rührt 3 Stunden bei −20 °C und 15 Stunden bei +20 °C nach.

Dann wird das Reaktionsgemisch mit eisgekühlter verdünnter Salzsäure und Eiswasser gewaschen. Die Methylenchloridlösung wird getrocknet und eingeengt. Der Rückstand wird mit Ethanol verrieben. Der hierbei kristallin angefallene Rückstand wird durch Absaugen isoliert.

Man erhält 11,5 g (58% der Theorie) der Verbindung der oben angegebenen Strukturformel vom Schmelzpunkt 158 °C (Zers.).

Die Aufarbeitung des Reaktionsgemisches kann auch in der Weise erfolgen, dass man nach beendeter Reaktion vollständig eindampft, den Rückstand in Dioxan aufnimmt, filtriert, erneut eindampft und den Rückstand umkristallisiert.

Analog Beispiel (XVIII-1) können die in der nachfolgenden Tabelle 15 aufgeführten. Verbindungen der Formel (XVIII) hergestellt werden:

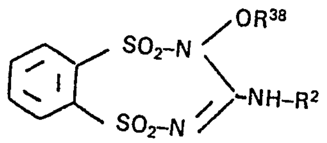

(XVIII)

Tabelle 15

| Beisp. Nr. | $R^{38}$ | $R^2$ | Schmelzpunkt (°C) |
|---|---|---|---|
| (XVIII-2) | $C_2H_5$ | 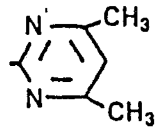 | 104 |
| (XVIII-3) | $-C_3H_7(-i)$ | 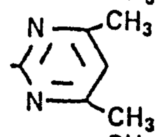 | amorph |
| (XVIII-4) | $-C_3H_7(-i)$ | 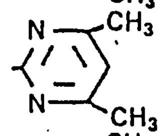 | 134 |
| (XVIII-5) | $-C_4H_9(-n)$ | 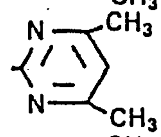 | 179 (Zers.) |
| (XVIII-6) | $-C_4H_9(-i)$ | 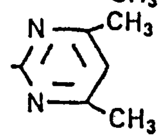 | |
| (XVIII-7) | $-C_4H_7(-s)$ | 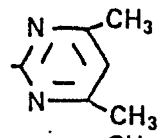 | |
| (XVIII-8) | $-CH_3$ | 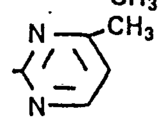 | |
| (XVIII-9) | $-CH_3$ | 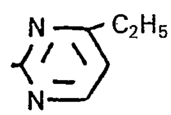 | |
| (XVIII-10) | $-CH_3$ | 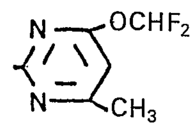 | |
| (XVIII-11) | $-CH_3$ | 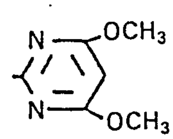 | |
| (XVIII-12) | $-C_4H_9(-s)$ | 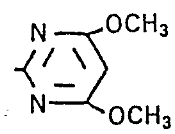 | |

Tabelle 15 Fortsetzung

| Beisp. Nr. | $R^{38}$ | $R^2$ | Schmelzpunkt (°C) |
|---|---|---|---|
| (XVIII-13) | $-C_4H_9(-i)$ | 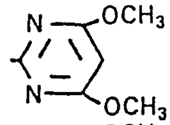 | |
| (XVIII-14) | $-CH_3$ | 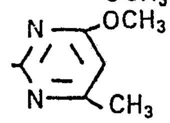 | 151 (Zers.) |
| (XVIII-15) | $-CH_3$ | 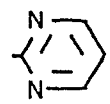 | 187 |

Herstellung von Ausgangsstoffen der Formel (V)

Beispiel (V-1)

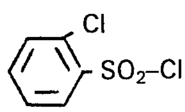

295 ml Phosphorylchlorid ('Phosphoroxychlorid') werden bei 20 °C bis 30 °C tropfenweise zu einer Mischung aus 172 g (0,8 Mol) 2-Chlor-benzolsulfonsäure-Natriumsalz, 300 ml Acetonitril und 300 ml Sulfolan gegeben. Das Reaktionsgemisch wird 4 Stunden bei 70 °C gerührt, anschliessend auf 5 °C abgekühlt und mit Eiswasser verdünnt. Nach Extrahieren mit Petrolether, Waschen der Extraktionslösung mit Wasser, Trocknen, Filtrieren und Einengen wird das im Rückstand verbliebene Produkt durch Vakuumdestillation gereinigt.

Man erhält 117 g (70% der Theorie) 2-Chlorbenzolsulfonsäurechlorid vom Siedepunkt 110 °C/1 mbar.

Beispiel (V-2)

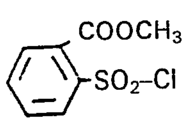

75 g (0,5 Mol) 2-Aminobenzoesäure-methylester werden in 176 ml konzentrierter Salzsäure und 100 ml Essigsäure gelöst. Hierzu wird bei 0 °C eine Lösung aus 34,4 g Natriumnitrit in 70 ml Wasser getropft. Nach 15 minütigem Nachrühren wird das Reaktionsgemisch langsam zu einer auf 0 °C gekühlten gesättigten Lösung von Schwefeldioxid in 450 ml Essigsäure gegeben. Nach Entfernung des Kühlbades wird bis zum Ende der Gasentwicklung gerührt, wobei 10 g Kupfer(II)-chlorid portionsweise eingetragen werden. Nach Verdünnen mit Eiswasser, Extrahieren mit Methylenchlorid, Waschen der Extraktionslösung mit Wasser, Trocknen, Filtrieren und Einengen wird das im Rückstand verbliebene Produkt durch Vakuumdestillation gereinigt.

Man erhält 45 g (38% der Theorie) 2-Methoxycarbonylbenzolsulfonsäurechlorid vom Siedepunkt 150 °C/1,33 mbar.

Analog können die in der nachstehenden Tabelle 16 aufgeführten Verbindungen der Formel (V) hergestellt werden:

$R^1-SO_2-Cl$ (V)

Tabelle 16

| Beisp. Nr. | $R^1$ | Siedepunkt/mbar |
|---|---|---|
| (V-3) | 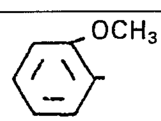 | (Öl, Zersetzung bei Destillation) |
| (V-4) | 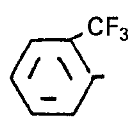 | (Öl) |

Tabelle 16 Fortsetzung

| Beisp. Nr. | $R^1$ | Siedepunkt/mbar |
|---|---|---|
| (V–5) | 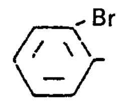 | 142 °C/4 |
| (V–6) | 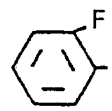 | 106 °C/4 |
| (V–7) | 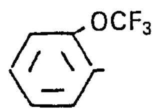 | (Schmelzpunkt: 32 °C) |
| (V–8) | 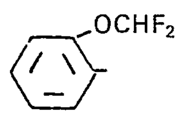 | (Öl, Zersetzung bei Destillation) |
| (V–9) | 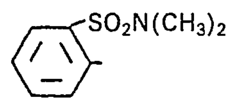 | (Schmelzpunkt: 103 °C) |
| (V–10) | 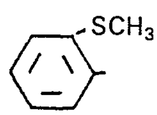 | (Öl, Zersetzung bei Destillation) |
| (V–11) | 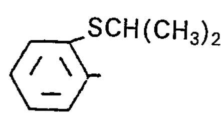 | 90 °C/1,33 |
| (V–12) | 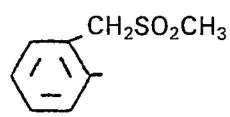 | (Schmelzpunkt: 120 °C) |
| (V–13) | 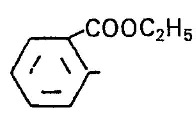 | 155 °C/5,32 mbar |
| (V–14) | 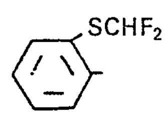 | |
| (V–15) | 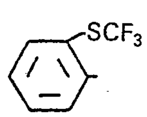 | (Schmelzpunkt: 41–43 °C) |
| (V–16) | 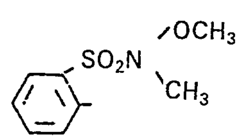 | (Schmelzpunkt: 99 °C) |
| (V–17) | 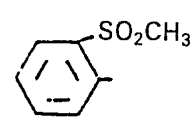 | (Schmelzpunkt: 128 °C) |

Tabelle 16 Fortsetzung

| Beisp. Nr. | $R^1$ | Siedepunkt/mbar |
|---|---|---|
| (V–18) | 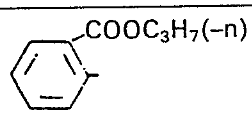 | |
| (V–19) | 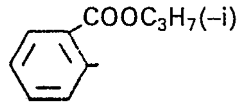 | |
| (V–20) | 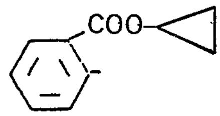 | |
| (V–21) | 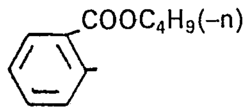 | |
| (V–22) | 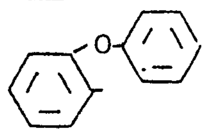 | |
| (V–23) | 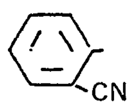 | |
| (V–24) | 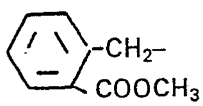 | (Schmelzpunkt: 84 °C) |
| (V–25) | 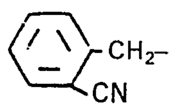 | |
| (V–26) | 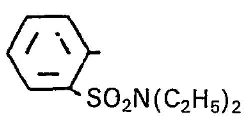 | (Schmelzpunkt: 73 °C) |
| (V–27) | 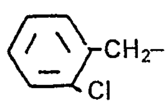 | (Schmelzpunkt: 84 °C) |

Analog können auch die Verbindungen der Formel (XIV) hergestellt werden.

Herstellung der Ausgangsstoffe der Formel (VI)

Beispiel (VI-1)

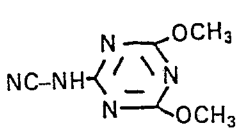

52,7 g (0,3 Mol) 2-Chlor-4,6-dimethoxy-s-triazin werden zu einer Lösung von 30 g (0,3 Mol) Cyanamid-Dinatriumsalz in 600 ml Aceton gegeben, und das Reaktionsgemisch wird 6 Stunden unter Rückfluss zum Sieden erhitzt. Nach Abdestillieren des Lösungsmittels wird der kristalline Rückstand in 250 ml Wasser gelöst und die Lösung mit konzentrierter Salzsäure angesäuert. Das kristallin angefallene Produkt wird durch Absaugen isoliert.

Man erhält 33 g (61% der Theorie) 2-Cyanamino-4,6-dimethoxy-s-triazin mit einem Schmelzpunkt über 300 °C.

Beispiel (VI-2)

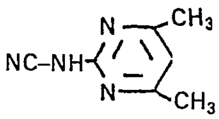

Ein Gemisch aus 42 g (0,5 Mol) Cyanoguanidin ('Dicyandiamid') und 50 g (0,5 Mol) 2,4-Pentandion ('Acetylaceton') wird 15 Stunden auf 120 °C erhitzt. Dann wird nach Abkühlen das Reaktionsgemisch mit 500 ml Wasser versetzt und die Lösung bei 0 °C bis 10 °C mit Salzsäure angesäuert. Das hierbei kristallin angefallene Produkt wird durch Absaugen isoliert.

Man erhält 51,8 g (70% der Theorie) 2-Cyanamino-4,6-dimethyl-pyrimidin vom Schmelzpunkt 205 °C.

Beispiel (VI-3)

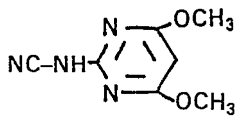

Eine auf 100 °C erhitzte Lösung von 24 g (0,427 Mol) Kaliumhydroxid in 100 ml Wasser wird bei 100 °C unter Rühren zu einer Mischung aus 9,2 g (0,043 Mol) 4,6-Dimethoxy-pyrimidin-2-yl-thioharnstoff in 70 ml Wasser gegeben. Man rührt 2 Minuten bei 100 °C nach und gibt dann eine auf 100 °C erwärmte Lösung von 16,2 g (0,05 Mol) Blei-II-acetat in 30 ml Wasser hinzu. Man erhitzt noch 5 Minuten unter Rückfluss, kühlt dann auf 0 °C bis 5 °C ab und versetzt die wässrige Lösung mit 30 ml Eisessig. Das hierbei kristallin ausfallende Produkt wird durch Absaugen isoliert.

Man erhält 6,3 g (81,5% der Theorie) 2-Cyanamino-4,6-dimethoxy-pyrimidin vom Schmelzpunkt 202 °C.

Analog können die in der nachstehenden Tabelle 17 aufgeführten Verbindungen der Formel (VI) hergestellt werden:

$$NC{-}NH{-}R^2 \qquad (VI)$$

Tabelle 17

| Beisp. Nr. | $R^2$ | Schmelzpunkt (°C) |
|---|---|---|
| (VI-4) | 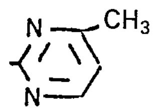 | 203 (Zers.) |
| (VI-5) | 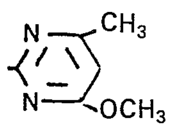 | 258 (Zers.) |
| (VI-6) | 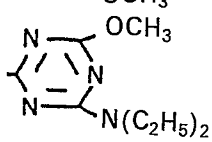 | 114 |
| (VI-7) | 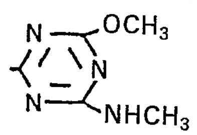 | 210 |
| (VI-8) | 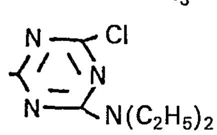 | 156 |
| (VI-9) | 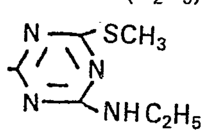 | |
| (VI-10) | 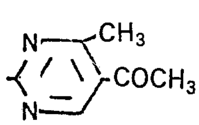 | 174 |

Tabelle 17 Fortsetzung

| Beisp. Nr. | $R^2$ | Schmelzpunkt (°C) |
|---|---|---|
| (VI–11) | 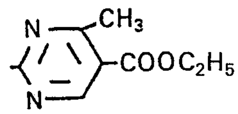 | 126 |
| (VI–12) | 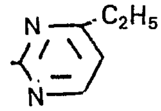 | 146 |
| (VI–13) | 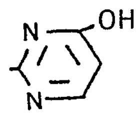 | > 300 |
| (VI–14) | 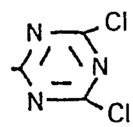 | > 250 |
| (VI–15) | 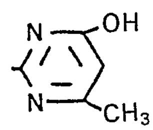 | > 270 |
| (VI–16) | 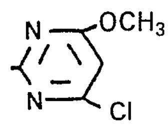 | 200 |
| (VI–17) | 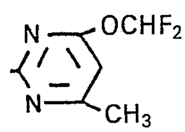 | 174 |
| (VI–18) | 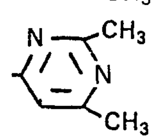 | 234 |
| (VI–19) | 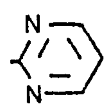 | 186 (Zers.) |
| (VI–20) | 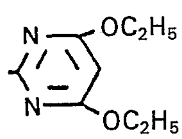 | 235–237 (Zers.) |

Herstellung der Ausgangsstoffe der Formel (XI)

Beispiel (XI-1)

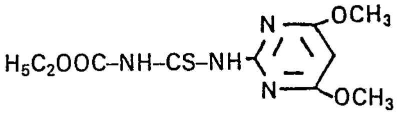

Eine Mischung aus 15,5 g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60 °C gerührt. Dann wird auf 10 °C abgekühlt, und das kristallin angefallene Produkt durch Absaugen isoliert.

Man erhält 22,5 g (79% der Theorie) 1-(Ethoxycarbonyl)-3- (4,6-dimethoxy-pyrimidin- 2-yl)-thioharnstoff vom Schmelzpunkt 194 °C (Zers.).

Analog Beispiel (XI-1) können die in der nachstehenden Tabelle 18 aufgeführten Verbindungen der Formel (XI) hergestellt werden:

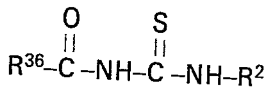

(XI)

Tabelle 18

| Beisp. Nr. | $R^{36}$ | $R^2$ | Schmelzpunkt (°C) |
|---|---|---|---|
| (XI–2) | 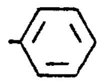 | 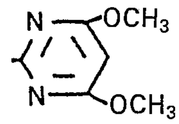 | 189 |
| (XI–3) | 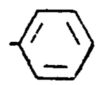 | 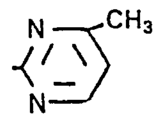 | 198–199 (Zers.) |
| (XI–4) | $-OC_2H_5$ | 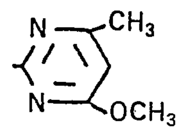 | 217 |
| (XI–5) | 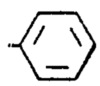 | 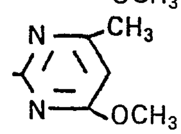 | 190 |
| (XI–6) | 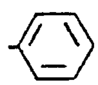 | 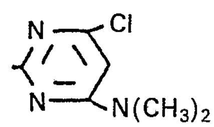 | 168 |
| (XI–7) | 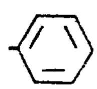 | 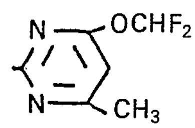 | 182 |
| (XI–8) | $-OC_2H_5$ | 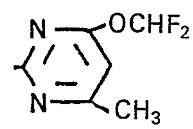 | 184–185 |
| (XI–9) | $-OC_2H_5$ | 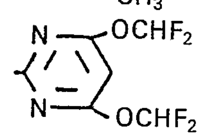 | 173 |
| (XI–10) | $-OC_2H_5$ | 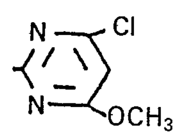 | 160–162 |
| (XI–11) | $-OC_2H_5$ | 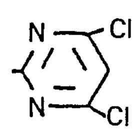 | 132–136 |
| (XI–12) | $-OC_2H_5$ | 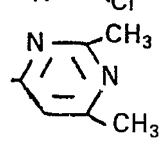 | 169 |
| (XI–13) | 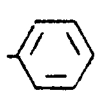 | 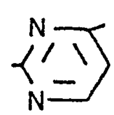 | 173 |

Tabelle 18 Fortsetzung

| Beisp. Nr. | $R^{36}$ | $R^2$ | Schmelzpunkt (°C) |
|---|---|---|---|
| (XI–14) | 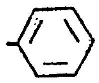 | 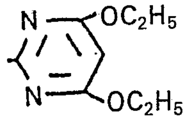 | 179 |
| (XI–15) | $-OC_2H_5$ | 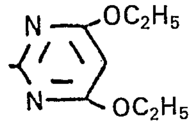 | 159 |
| (XI–16) | $-OC_2H_5$ | 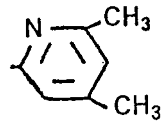 | 140 |
| (XI–17) | 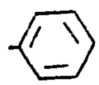 | 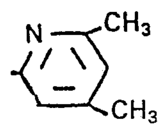 | 145 |

Herstellung der Ausgangsstoffe der Formel (XII)

Beispiel (XII-1)

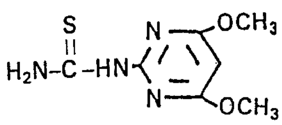

Eine Mischung aus 5,0 g (0,0175 Mol) 1-(Ethoxycarbonyl)-3- (4,6-dimethoxy-pyrimidin- 2-yl)-thioharnstoff, 4,0 g (0,1 Mol) Natriumhydroxyd und 100 ml Wasser wird 2 Tage bei 20 °C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94% der Theorie) (4,6-Dimethoxy-pyrimidin- 2-yl)- thioharnstoff vom Schmelzpunkt 245 °C bis 248 °C (Zers.).

Analog Beispiel (XII-1) können die in der nachstehenden Tabelle 19 aufgeführten Verbindungen der Formel (XII) hergestellt werden:

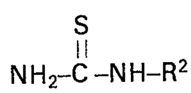 (XII)

Tabelle 19

| Beisp. Nr. | $R^2$ | Schmelzpunkt (°C) |
|---|---|---|
| (XII–2) | 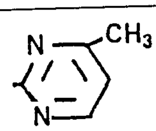 | 264–265 |
| (XII–3) | 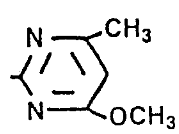 | 205–207 |
| (XII–4) | 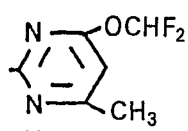 | 192–194 |
| (XII–5) | 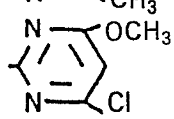 | 225–227 |

Tabelle 19 **Fortsetzung**

| Beisp. Nr. | $R^2$ | Schmelzpunkt (°C) |
|---|---|---|
| (XII-6) | 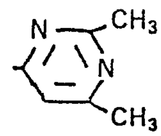 | 248 |
| (XII-7) | 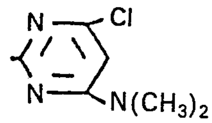 | |
| (XII-8) | 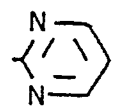 | 263 |
| (XII-9) | 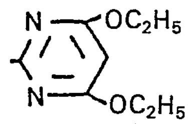 | 166 |
| (XII-10) | 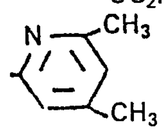 | 259–260 |

Beispiel A
Pre-emergence-Test/Gewächshaus

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Bei diesem Test zeigen z.B. die folgenden Verbindungen der Herstellungsbeispiele eine ausgezeichnete Wirksamkeit: (1) und (39)

Beispiel B
Post-emergence-Test/Gewächshaus

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Bei diesem Test zeigen z.B. die folgenden Verbindungen der Herstellungsbeispiele eine ausgezeichnete Wirksamkeit: (1) und (39).

## Patentansprüche

1. Sulfonyliso(thio)harnstoff-Derivate der allgemeinen Formel (I)

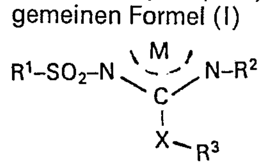 (I)

in welcher

$R^1$ für den Rest 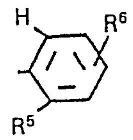 steht, worin

$R^5$ und $R^6$ gleich oder verschieden sind und für Wasserstoff, Halogen, Cyano, Nitro, $C_1$–$C_6$-Alkyl [welches gegebenenfalls durch Fluor, Chlor, Brom, Cyano, Carboxy, $C_1$–$C_4$-Alkoxycarbonyl, $C_1$–$C_4$-Alkylamino-carbonyl, Di-($C_1$–$C_4$-alkyl)-amino-carbonyl, Hydroxy, $C_1$–$C_4$-Alkoxy, Formyloxy, $C_1$–$C_4$-Alkyl-carbonyloxy, $C_1$–$C_4$-Alkoxy-carbonyloxy, $C_1$–$C_4$-Alkylamino-carbonyloxy, $C_1$–$C_4$-Alkylthio, $C_1$–$C_4$-Alkylsulfinyl, $C_1$–$C_4$-Alkylsulfonyl, Di-($C_1$–$C_4$-alkyl)-aminosulfonyl, $C_3$–$C_6$-Cycloalkyl oder Phenyl substituiert ist], für $C_2$–$C_6$-Alkenyl [welches gegebenenfalls durch Fluor, Chlor, Brom, Cyano, $C_1$–$C_4$-Alkoxycarbonyl, Carboxy oder Phenyl substituiert ist], für $C_2$–$C_6$-Alkinyl [welches gegebenenfalls durch Fluor, Chlor, Brom, Cyano, $C_1$–$C_4$-Alkoxy-carbonyl, Carboxy oder Phenyl substituiert ist], für $C_1$–$C_4$-Alkoxy [welches gegebenenfalls durch Fluor, Chlor, Brom, Cyano, Carboxy, $C_1$–$C_4$-Alkoxy-carbonyl, $C_1$–$C_4$-Alkoxy, $C_1$–$C_4$-Alkylthio, $C_1$–$C_4$-Alkylsulfinyl oder $C_1$–$C_4$-Alkylsulfonyl substituiert ist], für $C_1$–$C_4$-Alkylthio [welches gegebenenfalls durch Fluor, Chlor, Brom, Cyano, Carboxy, $C_1$–$C_4$-Alkoxycarbonyl, $C_1$–$C_4$-Alkylthio, $C_1$–$C_4$-Alkylsulfinyl oder $C_1$–$C_4$-Alkylsulfonyl substituiert ist], für $C_3$–$C_6$-Alkenyloxy [welches gegebenenfalls durch Fluor, Chlor, Brom, Cyano oder $C_1$–$C_4$-Alkoxy-carbonyl substituiert ist], für $C_2$–$C_6$-Alkenylthio [welches gegebenenfalls durch Fluor, Chlor, Brom, Cyano, Nitro, $C_1$–$C_3$-Alkylthio oder $C_1$–$C_4$-Alkoxycarbonyl substituiert ist], $C_3$–$C_6$-Alkinyloxy, $C_3$–$C_6$-Alkinylthio oder für den Rest $-S(O)_p-R^7$ stehen, wobei

p für die Zahlen 1 oder 2 steht und

$R^7$ für $C_1$–$C_4$-Alkyl [welches gegebenenfalls durch Fluor, Chlor, Brom, Cyano oder $C_1$–$C_4$-Alkoxy-carbonyl substituiert ist], $C_3$–$C_6$-Alkenyl, $C_3$–$C_6$-Alkinyl, $C_1$–$C_4$-Alkoxy, $C_1$–$C_4$-Alkoxyamino, $C_1$–$C_4$-Alkoxy- $C_1$–$C_4$-alkylamino, $C_1$–$C_4$-Alkylamino oder Di-($C_1$–$C_4$-alkyl)-amino steht,

$R^5$ und $R^6$ weiterhin für Phenoxy, für $C_1$–$C_4$-Alkylcarbonylamino, $C_1$–$C_4$-Alkoxy-carbonylamino, $C_1$–$C_4$-Alkylamino-carbonyl-amino, Di-($C_1$–$C_4$-alkyl)-amino-carbonylamino oder für den Rest $-CO-R^8$ stehen, wobei

$R^8$ für $C_1$–$C_6$-Alkyl, $C_1$–$C_6$-Alkoxy, $C_3$–$C_6$-Cycloalkoxy, $C_3$–$C_6$-Alkenyloxy, $C_1$–$C_4$-Alkylthio, $C_1$–$C_4$-Alkylamino, $C_1$–$C_4$-Alkoxyamino, $C_1$–$C_4$-Alkoxy-$C_1$–$C_4$-alkyl-amino oder Di-($C_1$–$C_4$-alkyl)-amino steht [welche gegebenenfalls durch Fluor und/oder Chlor substituiert sind],

$R^5$ und $R^6$ weiterhin für $C_1$–$C_4$-Alkylsulfonyloxy, Di-($C_1$–$C_4$-alkyl)-aminosulfonylamino oder für den Rest $-CH=N-R^9$ stehen, wobei

$R^9$ für gegebenenfalls durch Fluor, Chlor, Cyano, Carboxy, $C_1$–$C_4$-Alkoxy, Carbonyl, $C_1$–$C_4$-Alkylthio, $C_1$–$C_4$-Alkylsulfinyl oder $C_1$–$C_4$-Alkylsulfonyl substituiertes $C_1$–$C_6$-Alkyl, für gegebenenfalls durch Fluor oder Chlor substituiertes Benzyl, für gegebenenfalls durch Fluor oder Chlor substituiertes $C_3$–$C_6$-Alkenyl oder $C_3$–$C_6$-Alkinyl, für gegebenenfalls durch Fluor, Chlor, Brom, $C_1$–$C_4$-Alkyl, $C_1$–$C_4$-Alkoxy, Trifluormethyl, Trifluormethoxy oder Trifluormethylthio substituiertes Phenyl, für gegebenenfalls durch Fluor und/oder Chlor substituiertes $C_1$–$C_6$-Alkoxy, $C_3$–$C_6$-Alkenoxy, $C_3$–$C_6$-Alkinoxy oder Benzyloxy, für Amino, $C_1$–$C_4$-Alkylamino, Di-($C_1$–$C_4$-alkyl)amino, Phenylamino, $C_1$–$C_4$-Alkyl-carbonyl-amino, $C_1$–$C_4$-Alkoxy-carbonylamino, $C_1$–$C_4$-Alkyl-sulfonylamino oder für gegebenenfalls durch Fluor, Chlor, Brom oder Methyl substituiertes Phenylsulfonylamino steht, worin weiter

$R^1$ für den Rest 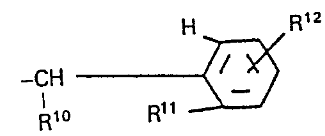 steht, worin

$R^{10}$ für Wasserstoff oder $C_1$–$C_4$-Alkyl steht,

$R^{11}$ und $R^{12}$ gleich oder verschieden sind und für Wasserstoff, Fluor, Chlor, Brom, Nitro, Cyano, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], Carboxy, $C_1$–$C_4$-Alkoxy-carbonyl, $C_1$–$C_4$-Alkylsulfonyl oder Di-($C_1$–$C_4$-alkyl)-aminosulfonyl stehen; worin weiter

$R^1$ für den Rest 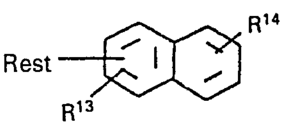 steht, worin

$R^{13}$ und $R^{14}$ gleich oder verschieden sind und für Wasserstoff, Fluor, Chlor, Brom, Nitro, Cyano, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist], stehen; worin weiter

$R^1$ für den Rest 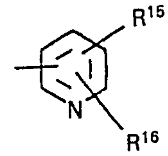 steht, worin

$R^{15}$ und $R^{16}$ gleich oder verschieden sind und für Wasserstoff, Fluor, Chlor, Brom, Nitro, Cyano, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], für $C_1$–$C_4$-Alkylthio, $C_1$–$C_4$-Alkylsulfinyl oder $C_1$–$C_4$-Alkylsulfonyl

[welche gegebenenfalls durch Fluor und/oder Chlor substituiert sind], sowie für Di-($C_1$–$C_4$-alkyl)-aminosulfonyl oder $C_1$–$C_4$-Alkoxy-carbonyl stehen; worin weiter

$R^1$ für den Rest 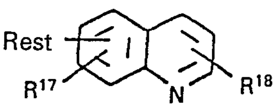 steht, worin

$R^{17}$ und $R^{18}$ gleich oder verschieden sind und für Wasserstoff, Fluor, Chlor, Brom, $C_1$–$C_4$-Alkyl [welches gegebenenfalls durch Fluor und/oder Brom substituiert ist], $C_1$–$C_4$-Alkoxy [welches gegebenenfalls durch Fluor und/oder Chlor substituiert ist], für $C_1$–$C_4$-Alkylthio, $C_1$–$C_4$-Alkylsulfinyl oder $C_1$–$C_4$-Alkylsulfonyl [welche gegebenenfalls durch Fluor und/oder Chlor substituiert sind], oder für Di-($C_1$–$C_4$-alkyl)-aminosulfonyl stehen; worin weiter

$R^1$ für den Rest 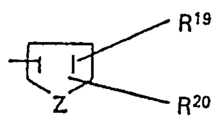 steht, worin

$R^{19}$ und $R^{20}$ gleich oder verschieden sind und für Wasserstoff, Fluor, Chlor, Brom, Cyano, Nitro, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylthio, $C_1$–$C_4$-Alkylsulfinyl oder $C_1$–$C_4$-Alkylsulfonyl [welches gegebenenfalls durch Fluor und/oder Chlor substituiert ist], Di-($C_1$–$C_4$-alkyl)-amino-sulfonyl oder $C_1$–$C_4$-Alkoxy-carbonyl stehen, und

Z für Sauerstoff, Schwefel oder die Gruppierung N–$Z^1$ steht, wobei

$Z^1$ für Wasserstoff, $C_1$–$C_4$-Alkyl [welches gegebenenfalls durch Fluor, Chlor, Brom oder Cyano substituiert ist], $C_3$–$C_6$-Cycloalkyl, Benzyl, Phenyl [welches gegebenenfalls durch Fluor, Chlor, Brom oder Nitro substituiert ist], $C_1$–$C_4$-Alkylcarbonyl, $C_1$–$C_4$-Alkoxy-carbonyl oder Di-($C_1$–$C_4$-alkyl)-aminocarbonyl steht; worin weiter

$R^1$ für den Rest 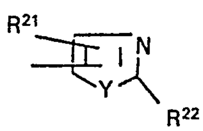 steht, worin

$R^{21}$ für Wasserstoff, $C_1$–$C_5$-Alkyl oder Halogen steht,

$R^{22}$ für Wasserstoff oder $C_1$–$C_5$-Alkyl steht und

Y für Schwefel oder die Gruppierung N–$R^{23}$ steht, wobei

$R^{23}$ für Wasserstoff oder $C_1$–$C_5$-Alkyl steht; worin weiter

$R^2$ für den Rest 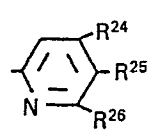 steht, worin

$R^{24}$ und $R^{26}$ gleich oder verschieden sind und für Wasserstoff, Fluor, Chlor, Brom, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist] stehen mit der Massgabe, dass wenigstens einer der Reste $R^{24}$ und $R^{26}$ von Wasserstoff verschieden ist, und

$R^{25}$ für Wasserstoff, Fluor, Chlor, Brom, Cyano oder $C_1$–$C_4$-Alkyl [welches gegebenenfalls durch Fluor und/oder Chlor substituiert ist] steht; worin weiter

$R^2$ für den Rest 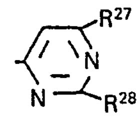 steht, worin

$R^{27}$ und $R^{28}$ gleich oder verschieden sind und für Wasserstoff, Fluor, Chlor, Brom, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], $C_1$–$C_4$-Alkylamino oder Di-($C_1$–$C_4$-alkyl)-amino stehen mit der Massgabe, dass wenigstens einer der Reste $R^{27}$ und $R^{28}$ von Wasserstoff verschieden ist;worin weiter

$R^2$ für den Rest 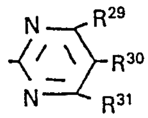 steht, worin

$R^{29}$ für Wasserstoff, Fluor, Chlor, Brom, Hydroxy, $C_1$–$C_4$-Alkyl [welches gegebenenfalls durch Fluor und/oder Chlor substituiert ist] oder $C_1$–$C_4$-Alkoxy [welches gegebenenfalls durch Fluor und/oder Chlor substituiert ist] steht,

$R^{30}$ für Wasserstoff, Fluor, Chlor, Brom, $C_1$–$C_4$-Alkyl [welches gegebenenfalls durch Fluor und/oder Chlor substituiert ist], Cyano, Formyl, $C_1$–$C_4$-Alkyl-carbonyl oder $C_1$–$C_4$-Alkoxycarbonyl steht und

$R^{31}$ für Wasserstoff, Fluor, Chlor, Brom, Hydroxy, $C_1$–$C_4$-Alkyl [welches gegebenenfalls durch Fluor und/oder Chlor substituiert ist], $C_1$–$C_4$-Alkoxy [welches gegebenenfalls durch Fluor und/oder Chlor substituiert ist], Amino, $C_1$–$C_4$-Alkyl-amino oder Di-($C_1$–$C_4$-alkyl)-amino steht, oder

$R^{30}$ und $R^{31}$ gemeinsam für $C_3$–$C_4$-Alkandiyl stehen; worin weiter

$R^2$ für den Rest 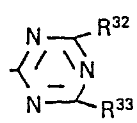 steht, worin

$R^{32}$ und $R^{33}$ gleich oder verschieden sind und für Fluor, Chlor, Brom, Hydroxy, $C_1$–$C_4$-Alkyl [welches gegebenenfalls durch Fluor und/oder Chlor substituiert ist], $C_3$–$C_5$-Cycloalkyl, $C_1$–$C_4$-Alkoxy [welches gegebenenfalls durch Fluor und/oder Chlor substituiert ist], $C_1$–$C_4$-Alkylthio oder für $C_1$–$C_4$-Alkylamino bzw. Di-($C_1$–$C_4$-alkyl)-amino stehen; worin weiter

$R^2$ für den Rest 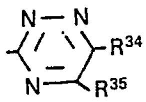 steht, worin

$R^{34}$ und $R^{35}$ gleich oder verschieden sind und für Wasserstoff, Methyl oder Methoxy stehen; worin weiter

$R^3$ für einen Phenylrest steht, welcher gegebenenfalls substituiert ist durch einen oder mehrere Reste aus der Reihe Halogen, Cyano, Nitro, Hydroxy, Carboxy, $C_1$–$C_6$-Alkyl [welches gegebenenfalls durch Fluor, Chlor, Brom, Nitro, Cyano, Hydroxy, Carboxy, $C_1$–$C_4$-Alkoxy-carbonyl, $C_1$–$C_4$-Alkoxy, $C_1$–$C_4$-Alkylthio oder Phenyl substituiert ist], $C_3$–$C_6$-Cycloalkyl, $C_1$–$C_4$-Alkoxy [welches gegebenenfalls durch Fluor, Chlor, Brom, Cyano, Carboxy, $C_1$–$C_4$-Alkoxy, $C_1$–$C_4$-Alkylthio oder $C_1$–$C_4$-Alkoxy-carbonyl substituiert ist], $C_1$–$C_4$-Alkylthio [welches gegebenenfalls durch Fluor, Chlor, Brom, Cyano, Carboxy, $C_1$–$C_4$-Alkoxy-carbonyl substituiert ist], Amino, $C_1$–$C_4$-Alkoxy-amino bzw. Di-($C_1$–$C_4$-alkyl)-amino [welche gegebenenfalls durch Fluor, Chlor, Brom, Cyano, Carboxy, $C_1$–$C_4$-Alkoxy oder $C_1$–$C_4$-Alkoxy-carbonyl substituiert sind], $C_1$–$C_4$-Alkyl-carbonyl-amino, $C_1$–$C_4$-Alkoxy-carbonylamino, (Di)-$C_1$–$C_4$-Alkylamino-carbonyl-amino, Formyl, $C_1$–$C_4$-Alkyl-carbonyl, Benzoyl, $C_1$–$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$–$C_4$-Alkyl-carbonyloxy, $C_1$–$C_4$-Alkoxy-carbonyloxy, $C_1$–$C_4$-Alkyl-amino-carbonyloxy und Di-($C_1$–$C_4$-alkyl)-amino-carbonyloxy, oder welcher gegebenenfalls durch einen Benzorest [welcher gegebenenfalls durch Fluor, Chlor, Brom, Cyano, Nitro, Methyl und/oder Trifluormethyl substituiert ist] anelliert ist; worin weiter

$R^3$ für einen fünf- oder sechsgliedrigen heteroaromatischen Ring steht, welcher 1 bis 3 Stickstoffatome und/oder ein Sauerstoff- oder Schwefelatom enthält und welcher gegebenenfalls benzanelliert ist und/oder durch Fluor, Chlor, Brom, Cyano, Nitro, $C_1$–$C_3$-Alkyl oder $C_1$–$C_3$-Alkoxy [wobei letztere gegebenenfalls durch Fluor und/oder Chlor substituiert sind] substituiert ist; worin weiter

X für Sauerstoff oder Schwefel steht und

M für Wasserstoff, ein Natrium-, Kalium-, Magnesium-, Calcium-, Aluminium-, Mangan-, Eisen-, Cobalt-, oder Nickel-Äquivalent steht, sowie Addukte von Verbindungen der Formel (I) mit Halogenwasserstoffsäuren, mit Schwefelsäure, mit gegebenenfalls durch Fluor und/oder Chlor substituierten Alkansulfonsäuren mit 1 bis 4 Kohlenstoffatomen oder mit Benzol- oder Naphthalinsulfonsäuren, welche gegebenenfalls durch Fluor, Chlor, Brom oder Methyl substituiert sind.

2. Sulfonyliso(thio)harnstoff-Derivate der allgemeinen Formel (I) gemäss Anspruch 1, dadurch gekennzeichnet, dass darin

(A) $R^1$ für den Rest 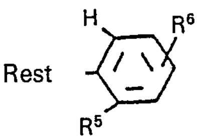 steht, worin

$R^5$ für Fluor, Chlor, Brom, Methyl, Trifluormethyl, Methoxy, Difluormethoxy, Trifluormethoxy, $C_1$–$C_3$-Alkylthio, Difluormethylthio, Trifluormethylthio, $C_1$–$C_3$-Alkylsulfinyl, $C_1$–$C_3$-Alkylsulfonyl, Dimethylaminosulfonyl, Diethylaminosulfonyl, N-Methoxy-N-methylaminosulfonyl, Phenoxy, $C_1$–$C_3$-Alkoxy-carbonyl oder $C_1$–$C_3$-Alkyl-aminocarbonyl steht und

$R^6$ für Wasserstoff steht;

$R^2$ für den Rest 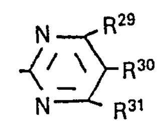 steht, worin

$R^{29}$ für Wasserstoff, Fluor, Chlor, Brom, Hydroxy, $C_1$–$C_3$-Alkyl, $C_1$–$C_3$-Alkoxy oder Difluormethoxy steht,

$R^{30}$ für Wasserstoff, Chlor, Brom oder Methyl steht und

$R^{31}$ für $C_1$–$C_3$-Alkyl, Hydroxy, Fluor, Chlor, Brom oder $C_1$–$C_3$-Alkoxy steht;

$R^3$ für einen Phenylrest steht, welcher gegebenenfalls substituiert ist durch einen oder zwei Reste aus der Reihe Fluor, Chlor, Brom, Jod, Cyano, Nitro, Hydroxy, Carboxy, $C_1$–$C_3$-Alkoxy-carbonyl, $C_1$–$C_4$-Alkyl, Trifluormethyl, Hydroxymethyl, Methoxycarbonylmethyl, Phenyl-$C_1$–$C_3$-alkyl, Cyclohexyl, $C_1$–$C_3$-Alkoxy, Trifluormethoxy, $C_1$–$C_3$-Alkylthio, Trifluormethylthio, Dimethylamino, Amino, Acetylamino, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pyridoxy [welches gegebenenfalls durch Chlor und/oder Trifluormethyl substituiert ist], oder welcher gegebenenfalls benzanelliert ist;

X für Sauerstoff oder Schwefel steht und

M für Wasserstoff, ein Natrium-, Kalium-, oder Calcium-äquivalent steht;

oder dass

(B) $R^1$, $R^3$, X und M die oben unter (A) angegebene Bedeutung haben und

$R^2$ für den Rest 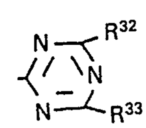 steht, worin

$R^{32}$ für Fluor, Chlor, Cyclopropyl, $C_1$–$C_2$-Alkyl, $C_1$–$C_2$-Alkoxy oder $C_1$–$C_2$-Alkylthio steht und

$R^{33}$ für Fluor, Chlor, Cyclopropyl, $C_1$–$C_2$-Alkyl,

$C_1$–$C_2$-Alkoxy, $C_1$–$C_2$-Alkylamino oder Di-($C_1$–$C_2$-alkyl)-amino steht, sowie Addukte von Verbindungen der Formel (I) mit Halogenwasserstoffsäuren, mit Schwefelsäure, mit gegebenenfalls durch Fluor und/oder Chlor substituierten Alkansulfonsäuren mit 1 bis 4 Kohlenstoffatomen oder mit Benzol- oder Naphthalinsulfonsäuren, welche gegebenenfalls durch Fluor, Chlor, Brom oder Methyl substituiert sind.

3. Verfahren zur Herstellung von Sulfonyliso-(thio)harnstoff-Derivaten der allgemeinen Formel (I) gemäss Anspruch 1, dadurch gekennzeichnet, dass man Sulfonylguanidin-Derivate der Formel (II)

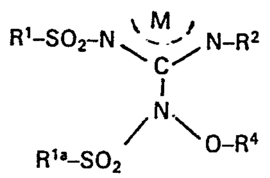

(II)

in welcher

$R^1$, $R^2$ und M die in Anspruch 1 angegebenen Bedeutungen haben,

$R^{1a}$ die gleiche Bedeutung wie $R^1$ hat, aber nicht in jedem Einzelfall mit $R^1$ identisch sein muss, und $R^4$ für $C_1$–$C_4$-Alkyl oder Benzyl steht, mit Verbindungen der Formel (III),

$$M^1\text{–}X\text{–}R^3 \qquad \text{(III)}$$

in welcher

X und $R^3$ die in Anspruch 1 angegebenen Bedeutungen haben und

$M^1$ für Wasserstoff, Natrium oder Kalium steht, gegebenenfalls in Gegenwart von Basen und in Gegenwart von Verdünnungsmitteln umsetzt und gegebenenfalls die hierbei erhaltenen Produkte der Formel (I) mit Säuren behandelt.

4. Herbizide Mittel, gekennzeichnet durch einen Gehalt an mindestens einem Sulfonyl(iso)-thioharnstoff-Derivat der allgemeinen Formel (I) gemäss Anspruch 1.

5. Verwendung von Sulfonyliso(thio)harnstoff-Derivaten der allgemeinen Formel (I) gemäss Anspruch 1 zur Bekämpfung von unerwünschtem Pflanzenwachstum.

6. Verfahren zur Herstellung von herbiziden Mitteln, dadurch gekennzeichnet, dass man Sulfonyliso(thio)harnstoff-Derivate der allgemeinen Formel (I) gemäss Anspruch 1 mit Streckmitteln und/oder oberflächenaktiven Mitteln vermischt.

## Revendications

1. Dérivés de sulfonyliso(thio)urées de formule générale (I)

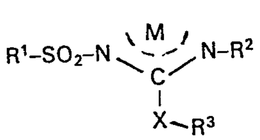

(I)

dans laquelle

$R^1$ représente le groupe 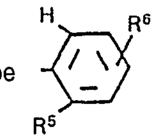 dans lequel

$R^5$ et $R^6$, ayant des significations identiques ou différentes, représentent chacun un hydrogène, un halogène, un groupe cyano, nitro, alkyle en $C_1$–$C_6$ [éventuellement substitué par le fluor, le chlore, le brome, des groupes cyano, carboxy, (alcoxy en $C_1$–$C_4$)-carbonyle), (alkylamino en $C_1$–$C_4$)-carbonyle, di(alkyle en $C_1$–$C_4$)-aminocarbonyle, hydroxy, alcoxy en $C_1$–$C_4$, formyloxy, (alkyle en $C_1$–$C_4$)-carbonyloxy, (alcoxy en $C_1$–$C_4$)-carbonyloxy, (alkylamino en $C_1$–$C_4$)-carbonyloxy, alkylthio en $C_1$–$C_4$, alkylsulfinyle en $C_1$–$C_4$, alkylsulfonyle en $C_1$–$C_4$, di(alkyle en $C_1$–$C_4$)-aminosulfonyle, cycloalkyle en $C_3$–$C_6$ ou phényle], un groupe alcényle en $C_2$–$C_6$ [éventuellement substitué par le fluor, le chlore, le brome, des groupes cyano, (alcoxy en $C_1$–$C_4$)-carbonyle, carboxy ou phényle], un groupe alcynyle en $C_2$–$C_6$ [éventuellement substitué par le fluor, le chlore, le brome, des groupes cyano, (alcoxy en $C_1$–$C_4$)-carbonyle, carboxy ou phényle], un groupe alcoxy en $C_1$–$C_4$ [éventuellement substitué par le fluor, le chlore, le brome, des groupes cyano, carboxy, (alcoxy en $C_1$–$C_4$)-carbonyle, alcoxy en $C_1$–$C_4$, alkylthio en $C_1$–$C_4$, alkylsulfinyle en $C_1$–$C_4$ ou alkylsulfonyle en $C_1$–$C_4$], un groupe alkylthio en $C_1$–$C_4$ [éventuellement substitué par le fluor, le chlore, le brome, des groupes cyano, carboxy, (alcoxy en $C_1$–$C_4$)-carbonyle, alkylthio en $C_1$–$C_4$, alkylsulfinyle en $C_1$–$C_4$ ou alkylsulfonyle en $C_1$–$C_4$], un groupe alcényloxy en $C_3$–$C_6$ [éventuellement substitué par le fluor, le chlore, le brome, des groupes cyano ou (alcoxy en $C_1$–$C_4$)-carbonyle], un groupe alcénylthio en $C_2$–$C_6$ [éventuellement substitué par le fluor, le chlore, le brome, des groupes cyano, nitro, alkylthio en $C_1$–$C_3$ ou (alcoxy en $C_1$–$C_4$)-carbonyle], un groupe alcynyloxy en $C_3$–$C_6$, alcynylthio en $C_3$–$C_6$ ou le groupe $-S(O)_p-R^7$, dans lequel

p est égal à 1 ou 2 et

$R^7$ représente un groupe alkyle en $C_1$–$C_4$ [éventuellement substitué par le fluor, le chlore, le brome, des groupes cyano ou (alcoxy en $C_1$–$C_4$)-carbonyle], un groupe alcényle en $C_3$–$C_6$, alcynyle en $C_3$–$C_6$, alcoxy en $C_1$–$C_4$, alcoxyamino en $C_1$–$C_4$, (alcoxy en $C_1$–$C_4$)-alkylamino en $C_1$–$C_4$, alkylamino en $C_1$–$C_4$ ou di(alkyle en $C_1$–$C_4$)-amino, $R^5$ et $R^6$ peuvent en outre représenter un groupe phénoxy, (alkyle en $C_1$–$C_4$)-carbonylamino, (alcoxy en $C_1$–$C_4$)-carbonylamino, (alkylamino en $C_1$–$C_4$)-carbonylamino, di(alkyle en $C_1$–$C_4$)-amino-carbonylamino ou le groupe $-CO-R^8$, dans lequel

$R^8$ représente un groupe alkyle en $C_1$–$C_6$, alcoxy en $C_1$–$C_6$, cycloalcoxy en $C_3$–$C_6$, alcényloxy en $C_3$–$C_6$, alkylthio en $C_1$–$C_4$, alkylamino en $C_1$–$C_4$, alcoxyamino en $C_1$–$C_4$, (alcoxy en $C_1$–$C_4$)-alkylamino en $C_1$–$C_4$ ou di(alkyle en $C_1$–$C_4$)-amino [éventuellement substitués par le fluor et/ou le chlore],

$R^5$ et $R^6$ peuvent en outre représenter un groupe alkylsulfonyloxy en $C_1$–$C_4$, di(alkyle en $C_1$–$C_4$)-aminosulfonylamino ou le groupe $-CH=N-R^9$, dans lequel

$R^9$ représente un groupe alkyle en $C_1$–$C_6$ éventuellement substitué par le fluor, le chlore, des groupes cyano, carboxy, alcoxy en $C_1$–$C_4$, carbonyle, alkylthio en $C_1$–$C_4$, alkylsulfinyle en $C_1$–$C_4$ ou alkylsulfonyle en $C_1$–$C_4$, un groupe benzyle éventuellement substitué par le fluor ou le chlore, un groupe alcényle en $C_3$–$C_6$ ou alcynyle en $C_3$–$C_6$ éventuellement substitué par le fluor ou le chlore, un groupe phényle éventuellement substitué par le fluor, le chlore, le brome, des groupes alkyles en $C_1$–$C_4$, alcoxy en $C_1$–$C_4$, trifluorométhyle, trifluorométhoxy ou trifluorométhylthio, un groupe alcoxy en $C_1$–$C_6$, alcényloxy en $C_3$–$C_6$, alcynyloxy en $C_3$–$C_6$ ou benzyloxy éventuellement substitué par le fluor et/ou le chlore, un groupe amino, alkylamino en $C_1$–$C_4$, di(alkyle en $C_1$–$C_4$)-amino, phénylamino, (alkyle en $C_1$–$C_4$)-carbonylamino, (alcoxy en $C_1$–$C_4$)-carbonylamino, (alkyle en $C_1$–$C_4$)-sulfonylamino ou un groupe phénylsulfonylamino éventuellement substitué par le fluor, le chlore, le brome ou un groupe méthyle, et en outre

$R^1$ peut représenter le groupe 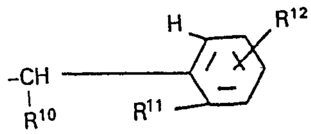 dans lequel

$R^{10}$ représente l'hydrogène ou un groupe alkyle en $C_1$–$C_4$,

$R^{11}$ et $R^{12}$ ayant des significations identiques ou différentes, représentent chacun l'hydrogène, le fluor, le chlore, le brome, un groupe nitro, cyano, alkyle en $C_1$–$C_4$ [éventuellement substitué par le fluor et/ou le chlore], un groupe alcoxy en $C_1$–$C_4$ [éventuellement substitué par le fluor et/ou le chlore], un groupe carboxy, (alcoxy en $C_1$–$C_4$)-carbonyle, alkylsulfonyle en $C_1$–$C_4$ ou di(alkyle en $C_1$–$C_4$)-aminosulfonyle; et en outre

$R^1$ peut représenter le groupe 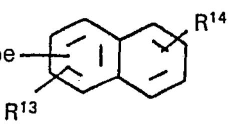

dans lequel

$R^{13}$ et $R^{14}$, ayant des significations identiques ou différentes, représentent chacun l'hydrogène, le fluor, le chlore, le brome, un groupe nitro, cyano, alkyle en $C_1$–$C_4$ [éventuellement substitué par le fluor et/ou le chlore] ou alcoxy en $C_1$–$C_4$ [éventuellement substitué par le fluor et/ou le chlore]; et en outre

$R^1$ peut représenter le groupe 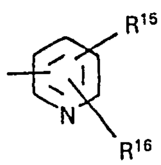

dans lequel

$R^{15}$ et $R^{16}$, ayant des significations identiques ou différentes, représentent chacun l'hydrogène, le fluor, le chlore, le brome, un groupe nitro, cyano, alkyle en $C_1$–$C_4$ [éventuellement substitué par le fluor et/ou le chlore], alcoxy en $C_1$–$C_4$ [éventuellement substitué par le fluor et/ou le chlore], un groupe alkylthio en $C_1$–$C_4$, alkylsulfinyle en $C_1$–$C_4$ ou alkylsulfonyle en $C_1$–$C_4$ [éventuellement substitués par le fluor et/ou le chlore] ou un groupe di(alkyle en $C_1$–$C_4$)-aminosulfonyle ou (alcoxy en $C_1$–$C_4$)-carbonyle; et en outre

$R^1$ peut représenter le groupe 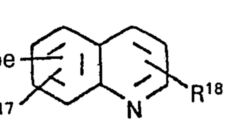

dans lequel

$R^{17}$ et $R^{18}$, ayant des significations identiques ou différentes, représentent chacun l'hydrogène, le fluor, le chlore, le brome, un groupe alkyle en $C_1$–$C_4$ [éventuellement substitué par le fluor et/ou le brome], un groupe alcoxy en $C_1$–$C_4$ [éventuellement substitué par le fluor et/ou le chlore], un groupe alkylthio en $C_1$–$C_4$, alkylsulfinyle en $C_1$–$C_4$ ou alkylsulfonyle en $C_1$–$C_4$ [éventuellement substitués par le fluor et/ou le chlore] ou un groupe di(alkyle en $C_1$–$C_4$)-aminosulfonyle; et en outre

$R^1$ peut représenter le groupe 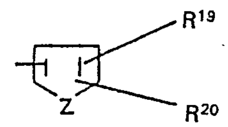

dans lequel

$R^{19}$ et $R^{20}$, ayant des significations identiques ou différentes, représentent chacun l'hydrogène, le fluor, le chlore, le brome, un groupe cyano, nitro, alkyle en $C_1$–$C_4$ [éventuellement substitué par le fluor et/ou le chlore], alcoxy en $C_1$–$C_4$ [éventuellement substitué par le fluor et/ou le chlore], un groupe alkylthio en $C_1$–$C_4$, alkylsulfinyle en $C_1$–$C_4$ ou alkylsulfonyle en $C_1$–$C_4$ [éventuellement substitué par le fluor et/ou le chlore], un groupe di(alkyle en $C_1$–$C_4$)-aminosulfonyle ou (alcoxy en $C_1$–$C_4$)-carbonyle et

Z représente l'oxygène, le soufre ou le groupe $N-Z^1$, dans lequel

$Z^1$ représente l'hydrogène, un groupe alkyle en $C_1$–$C_4$ [éventuellement substitué par le fluor, le chlore, le brome ou un groupe cyano], cycloalkyle en $C_3$–$C_6$, benzyle, phényle, [éventuellement substitué par le fluor, le chlore, le brome ou un groupe nitro], (alkyle en $C_1$–$C_4$)-carbonyle, (alcoxy en $C_1$–$C_4$)-carbonyle ou di(alkyle en $C_1$–$C_4$)-aminocarbonyle, et en outre

$R^1$ peut représenter le groupe 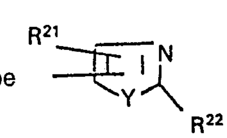

dans lequel

$R^{21}$ représente l'hydrogène, un groupe alkyle en $C_1$–$C_5$ ou un halogène,

$R^{22}$ représente l'hydrogène ou un groupe alkyle en $C_1$–$C_5$ et

Y représente le soufre ou le groupement N–$R^{23}$, dans lequel

$R^{23}$ représente l'hydrogène ou un groupe alkyle en $C_1$–$C_5$; et en outre

$R^2$ peut représenter le groupe

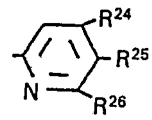

dans lequel

$R^{24}$ et $R^{26}$, ayant des significations identiques ou différentes, représentent chacun l'hydrogène, le fluor, le chlore, le brome, un groupe alkyle en $C_1$–$C_4$ [éventuellement substitué par le fluor et/ou le chlore] ou alcoxy en $C_1$–$C_4$ [éventuellement substitué par le fluor et/ou le chlore], sous réserve que l'un au moins des symboles $R^{24}$ et $R^{26}$ a une signification autre que l'hydrogène, et

$R^{25}$ représente l'hydrogène, le fluor, le chlore, le brome, un groupe cyano ou alkyle en $C_1$–$C_4$ [éventuellement substitué par le fluor et/ou le chlore]; et en outre

$R^2$ peut représenter le groupe

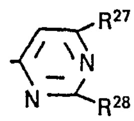

dans lequel

$R^{27}$ et $R^{28}$, ayant des significations identiques ou différentes, représentent chacun l'hydrogène, le fluor, le chlore, le brome, un groupe alkyle en $C_1$–$C_4$ [éventuellement substitué par le fluor et/ou le chlore], alcoxy en $C_1$–$C_4$ [éventuellement substitué par le fluor et/ou le chlore], alkylamino en $C_1$–$C_4$ ou di(alkyle en $C_1$–$C_4$)-amino, sous réserve que l'un au moins des symboles $R^{27}$ et $R^{28}$ a une signification autre que l'hydrogène; et en outre

$R^2$ peut représenter le groupe

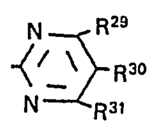

dans lequel

$R^{29}$ représente l'hydrogène, le fluor, le chlore, le brome, un groupe hydroxy, alkyle en $C_1$–$C_4$ [éventuellement substitué par le fluor et/ou le chlore] ou alcoxy en $C_1$–$C_4$ [éventuellement substitué par le fluor et/ou le chlore],

$R^{30}$ représente l'hydrogène, le fluor, le chlore, le brome, un groupe alkyle en $C_1$–$C_4$ [éventuellement substitué par le fluor et/ou le chlore], cyano, formyle, (alkyle en $C_1$–$C_4$)-carbonyle ou (alcoxy en $C_1$–$C_4$)-carbonyle et

$R^{31}$ représente l'hydrogène, le fluor, le chlore, le brome, un groupe hydroxy, alkyle en $C_1$–$C_4$ [éventuellement substitué par le fluor et/ou le chlore], alcoxy en $C_1$–$C_4$ [éventuellement substitué par le fluor et/ou le chlore], amino, alkylamino en $C_1$–$C_4$ ou di(alkyle en $C_1$–$C_4$)-amino, ou bien

$R^{30}$ et $R^{31}$ forment ensemble un groupe (alcane en $C_3$–$C_4$)-diyle; et en outre

$R^2$ peut représenter le groupe

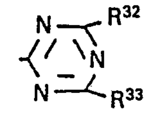

dans lequel

$R^{32}$ et $R^{33}$, ayant des significations identiques ou différentes, représentent chacun le fluor, le chlore, le brome, un groupe hydroxy, alkyle en $C_1$–$C_4$ [éventuellement substitué par le fluor et/ou le chlore], cycloalkyle en $C_3$–$C_5$, alcoxy en $C_1$–$C_4$ [éventuellement substitué par le fluor et/ou le chlore], alkylthio en $C_1$–$C_4$ ou alkylamino en $C_1$–$C_4$ ou di(alkyle en $C_1$–$C_4$)-amino; et en outre $R^2$ peut représenter le groupe

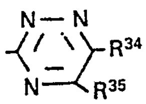

dans lequel

$R^{34}$ et $R^{35}$, ayant des significations identiques ou différentes, représentent chacun l'hydrogène, un groupe méthyle ou méthoxy; et en outre

$R^3$ peut représenter un groupe phényle portant éventuellement un ou plusieurs substituants choisis parmi les suivants:

les halogènes, les groupes cyano, nitro, hydroxy, carboxy, alkyle en $C_1$–$C_6$ [éventuellement substitué par le fluor, le chlore, le brome, des groupes nitro, cyano, hydroxy, carboxy, (alcoxy en $C_1$–$C_4$)-carbonyle, alcoxy en $C_1$–$C_4$, alkylthio en $C_1$–$C_4$ ou phényle], cycloalkyle en $C_3$–$C_6$, alcoxy en $C_1$–$C_4$ [éventuellement substitué par le fluor, le chlore, le brome, des groupes cyano, carboxy, alcoxy en $C_1$–$C_4$, alkylthio en $C_1$–$C_4$ ou (alcoxy en $C_1$–$C_4$)-carbonyle], alkylthio en $C_1$–$C_4$ [éventuellement substitué par le fluor, le chlore, le brome, des groupes cyano, carboxy, (alcoxy en $C_1$–$C_4$)-carbonyle], amino, alkylamino en $C_1$–$C_4$ ou di(alkyle en $C_1$–$C_4$)-amino [éventuellement substitués par le fluor, le chlore, le brome, des groupes cyano, carboxy, alcoxy en $C_1$–$C_4$ ou (alcoxy en $C_1$–$C_4$)-carbonyle], (alkyle en $C_1$–$C_4$)-carbonylamino, (alcoxy en $C_1$–$C_4$)-carbonylamino, di(alkyle en $C_1$–$C_4$)-amino-carbonyl-amino, formyle, (alkyle en $C_1$–$C_4$)-carbonyle, benzoyle, (alcoxy en $C_1$–$C_4$)-carbonyle, phénoxycarbonyle, benzyloxycarbonyle, phényle, [éventuellement substitué par le fluor, le chlore, le brome, des groupes cyano, nitro, hydroxy ou méthyle], phénoxy, phénylthio, phénylsulfonyle, phénylamino ou phénylazo [éventuellement substitués par le fluor, le chlore, le brome, des groupes cyano, nitro, méthyle et/ou trifluorométhyle], pyridoxy ou pyrimidoxy [éventuellement substitués par le fluor, le chlore, le brome, des groupes cyano, nitro, méthyle et/ou trifluorométhyle], (alkyle en $C_1$–$C_4$)-carbonyloxy, (alcoxy en ($C_1$–$C_4$)-carbonyloxy, (alkyle en $C_1$–$C_4$)-aminocarbonyloxy et di(alkyle en $C_1$–$C_4$)-amino-carbonyloxy ou qui est éventuellement condensé avec un groupe benzénique [lequel est éventuellement substitué par le fluor, le chlore, le brome, des groupes cyano, nitro, méthyle et/ou trifluorométhyle]; et

en outre $R^3$ peut représenter un cycle hétéro-aromatique à cinq ou six chaînons contenant 1 à 3 atomes d'azote et/ou un atome d'oxygène ou de soufre et qui est éventuellement condensé avec un noyau benzénique et/ou substitué par le fluor, le chlore, le brome, des groupes cyano, nitro, alkyle en $C_1$–$C_3$ ou alcoxy en $C_1$–$C_3$ [ces derniers pouvant eux-mêmes être éventuellement substitués par le fluor et/ou le chlore]; et en outre

X représente l'oxygène ou le soufre, et

M représente l'hydrogène, un équivalent de sodium, de potassium, de magnésium, de calcium, d'aluminium, de manganèse, de fer, de cobalt ou de nickel, ainsi que les adducts des composés de formule (I) et des hydracides halogénés, de l'acide sulfurique, d'acides alcane-sulfoniques en $C_1$–$C_4$ éventuellement substitués par le fluor et/ou le chlore ou d'acides benzène- ou naphtalène-sulfoniques éventuellement substitués par le fluor, le chlore, le brome ou des groupes méthyles.

2. Dérivés de sulfonyliso(thio)urées de formule générale (I) selon la revendication 1, caractérisés en ce que:

(A) $R^1$ représente le groupe 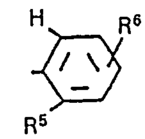

dans lequel

$R^5$ représente le fluor, le chlore, le brome, un groupe méthyle, trifluorométhyle, méthoxy, difluorométhoxy, trifluorométhoxy, alkylthio en $C_1$–$C_3$, difluorométhylthio, trifluorométhylthio, alkylsulfinyle en $C_1$–$C_3$, alkylsulfonyle en $C_1$–$C_3$, diméthylaminosulfonyle, diéthylaminosulfonyle, N-méthoxy-N-méthylaminosulfonyle, phénoxy, (alcoxy en $C_1$–$C_3$)-carbonyle, ou (alkyle en $C_1$–$C_3$)-aminocarbonyle et

$R^6$ représente l'hydrogène;

$R^2$ représente le groupe 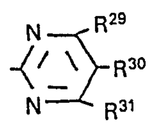

dans lequel

$R^{29}$ représente l'hydrogène, le fluor, le chlore, le brome, un groupe hydroxy, alkyle en $C_1$–$C_3$, alcoxy en $C_1$–$C_3$ ou difluorométhoxy,

$R^{30}$ représente l'hydrogène, le chlore, le brome ou un groupe méthyle et

$R^{31}$ représente un groupe alkyle en $C_1$–$C_3$, hydroxy, le fluor, le chlore, le brome ou un groupe alcoxy en $C_1$–$C_3$;

$R^3$ représente un groupe phényle portant éventuellement un ou deux substituants des classes suivantes:

le fluor, le chlore, le brome, l'iode, les groupes cyano, nitro, hydroxy, carboxy, (alcoxy en $C_1$–$C_3$)-carbonyle, alkyle en $C_1$–$C_4$, trifluorométhyle, hydroxyméthyle, méthoxycarbonylméthyle, phénylalkyle en $C_1$–$C_3$, cyclohexyle, alcoxy en $C_1$–$C_3$, trifluorométhoxy, alkylthio en $C_1$–$C_3$, trifluorométhylthio, diméthylamino, amino, acétylamino, formyle, acétyle, benzoyle, phényle, hydroxyphényle, phénoxy [éventuellement substitué par le chlore et/ou un groupe trifluorométhyle], phénylamino, phénylazo, pyridoxy [éventuellement substitué par le chlore et/ou un groupe trifluorométhyle] ou qui est éventuellement condensé avec un noyau benzénique;

X représente l'oxygène ou le soufre et

M représente l'hydrogène, un équivalent de sodium, de potassium ou de calcium; ou bien

(B) $R^1$, $R^3$, X et M ont les significations indiquées ci-dessus sous (A) et

$R^2$ représente le groupe 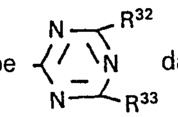 dans lequel

$R^{32}$ représente le fluor, le chlore, un groupe cyclopropyle, alkyle en $C_1$–$C_2$, alcoxy en $C_1$–$C_2$ ou alkylthio en $C_1$–$C_2$ et

$R^{33}$ représente le fluor, le chlore, un groupe cyclopropyle, alkyle en $C_1$–$C_2$, alcoxy en $C_1$–$C_2$, alkylamino en $C_1$–$C_2$ ou di(alkyle en $C_1$–$C_2$)-amino, ainsi que les adducts des composés de formule (I) et d'hydracides halogénés, de l'acide sulfurique, d'acides alcane-sulfoniques en $C_1$–$C_4$ évenutellement substitués par le fluor et/ou le chlore ou d'acides benzène- ou naphtalène-sulfoniques éventuellement substitués par le fluor, le chlore, le brome ou des groupes méthyles.

3. Procédé de préparation des dérivés de sulfonyliso(thio)urées de formule générale (I) selon la revendication 1, caractérisé en ce que l'on fait réagir des dérivés de sulfonylguanidines de formule (II)

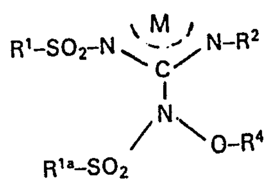

(II)

dans laquelle

$R^1$, $R^2$ et M ont les significations indiquées dans la revendication 1,

$R^{1a}$ a les mêmes significations que $R^1$ mais n'est pas nécessairement identique dans chaque cas à $R^1$, et

$R^4$ représente un groupe alkyle en $C_1$–$C_4$ ou benzyle, avec des composés de formule (III),

$$M^1\text{–}X\text{–}R^3 \qquad (III)$$

dans laquelle

X et $R^3$ ont les significations indiquées dans la revendication 1, et

$M^1$ représente l'hydrogène, le sodium ou le potassium, éventuellement en présence de bases et en présence de diluants puis, le cas échéant, on traite les produits obtenus répondant à la formule (I) par des acides.

4. Produits herbicides, caractérisés en ce qu'ils contiennent au moins un dérivé de sulfonyl(iso)-thiourée de formule générale (I) selon la revendication 1.

5. Utilisation des dérivés de sulfonyliso(thio) urées de formule générale(I) selon la revendication 1, dans la lutte contre les croissances de végétaux indésirables.

6. Procédé de préparation de produits herbicides, caractérisé en ce que l'on mélange des dérivés de sulfonyliso(thio)urées de formule générale (I) selon la revendication 1 avec des diluants et/ou des agents tensioactifs.

## Claims

1. Sulphonyliso(thio)urea derivatives of the general formula (I)

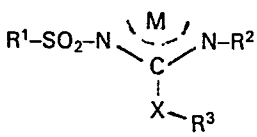

(I)

in which

$R^1$, represents the radical

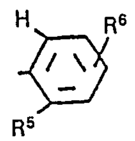

wherein

$R^5$ and $R^6$ are identical or different and represent hydrogen, halogen, cyano, nitro, $C_1$–$C_6$-alkyl [which is optionally substituted by fluorine, chlorine, bromine, cyano, carboxyl, $C_1$–$C_4$-alkoxycarbonyl, $C_1$–$C_4$-alkylaminocarbonyl, di-($C_1$–$C_4$-alkyl)-amino-carbonyl, hydroxyl, $C_1$–$C_4$-alkoxy, formyloxy, $C_1$–$C_4$-alkyl-carbonyloxy, $C_1$–$C_4$-alkoxy-carbonyloxy, $C_1$–$C_4$-alkylamino-carbonyloxy, $C_1$–$C_4$-alkylthio, $C_1$–$C_4$-alkyl-sulphinyl, $C_1$–$C_4$-alkylsulphonyl, di-($C_1$–$C_4$-alkyl)-aminosulphonyl, $C_3$–$C_6$-cycloalkyl or phenyl], or represent $C_2$–$C_6$-alkenyl [which is optionally substituted by fluorine, chlorine, bromine, cyano, $C_1$–$C_4$-alkoxy-carbonyl, carboxyl or phenyl], or represent $C_2$–$C_6$-alkinyl [which is optionally substituted by fluorine, chlorine, bromine, cyano, $C_1$–$C_4$-alkoxy-carbonyl, carboxyl or phenyl], or represent $C_1$–$C_4$-alkoxy [which is optionally substituted by fluorine, chlorine, bromine, cyano, carboxyl, $C_1$–$C_4$-alkoxy-carbonyl, $C_1$–$C_4$-alkoxy, $C_1$–$C_4$-alkylthio, $C_1$–$C_4$-alkylsulphinyl or $C_1$–$C_4$-alkylsulphonyl], or represent $C_1$–$C_4$-alkylthio [which is optionally substituted by fluorine, chlorine, bromine, cyano, carboxyl, $C_1$–$C_4$-alkoxycarbonyl, $C_1$–$C_4$-alkylthio, $C_1$–$C_4$-alkylsulphinyl or $C_1$–$C_4$-alkylsulphonyl], or represent $C_3$–$C_6$-alkenyloxy [which is optionally substituted by fluorine, chlorine, bromine, cyano or $C_1$–$C_4$-alkoxy-carbonyl], or represent $C_2$–$C_6$-alkenylthio [which is optionally substituted by fluorine, chlorine, bromine, cyano, nitro, $C_1$–$C_3$-alkylthio or $C_1$–$C_4$-alkoxycarbonyl], $C_3$–$C_6$-alkinyloxy, $C_3$–$C_6$-alkinylthio, or represent the radical $-S(O)_p-R^7$ wherein

p represents the numbers 1 or 2 and

$R^7$ represents $C_1$–$C_4$-alkyl [which is optionally substituted by fluorine, chlorine, bromine, cyano or $C_1$–$C_4$-alkoxy-carbonyl], $C_5$–$C_6$-alkenyl, $C_3$–$C_6$-alkinyl, $C_1$–$C_4$-alkoxy, $C_1$–$C_4$-alkoxy-amino, $C_1$–$C_4$-alkoxy-$C_1$–$C_4$-alkyl-amino, $C_1$–$C_4$-alkyl-amino or di-($C_1$–$C_4$-alkyl)-amino, or

$R^5$ and $R^6$ furthermore represent phenoxy, or represent $C_1$–$C_4$-alkylcarbonyl-amino, $C_1$–$C_4$-alkoxy-carbonylamino, $C_1$–$C_4$-alkylamino-carbonyl-amino, di-($C_1$–$C_4$-alkyl)-amino-carbonylamino or represent the radical $-CO-R^8$, wherein

$R^8$ represents $C_1$–$C_6$-alkyl, $C_1$–$C_6$-alkoxy, $C_3$–$C_6$-cycloalkoxy, $C_3$–$C_6$-alkenyloxy, $C_1$–$C_4$-alkylthio, $C_1$–$C_4$-alkylamino, $C_1$–$C_4$-alkoxyamino, $C_1$–$C_4$-alkoxy-$C_1$–$C_4$-alkyl-amino or di-($C_1$–$C_4$-alkyl)-amino [which are optionally substituted by fluorine and/or chlorine], or

$R^5$ and $R^6$ further represent $C_1$–$C_4$-alkylsulphonyloxy, di-($C_1$–$C_4$-alkyl)-aminosulphonylamino or represent the radical $-CH=N-R^9$, wherein

$R^9$ represents $C_1$–$C_6$-alkyl which is optionally substituted by fluorine, chlorine, cyano, carboxyl, $C_1$–$C_4$-alkoxy, carbonyl, $C_1$–$C_4$-alkylthio, $C_1$–$C_4$-alkylsulphinyl or $C_1$–$C_4$-alkylsulphonyl, or represents benzyl which is optionally substituted by fluorine or chlorine, or represents $C_5$–$C_6$-alkenyl or $C_3$–$C_6$-alkinyl, optionally substituted by fluorine or chlorine, or represents phenyl which is optionally substituted by fluorine, chlorine, bromine, $C_1$–$C_4$-alkyl, $C_1$–$C_4$-alkoxy, trifluoromethyl, trifluoromethoxy or trifluoromethylthio, or represents $C_1$–$C_6$-alkoxy, $C_3$–$C_6$-alkenoxy, $C_3$–$C_6$-alkinoxy or benzyloxy, optionally substituted by fluorine and/or chlorine, or represents amino, $C_1$–$C_4$-alkylamino, di-($C_1$–$C_4$-alkyl)-amino, phenylamino, $C_1$–$C_4$-alkyl-carbonyl-amino, $C_1$–$C_4$-alkoxy-carbonylamino or $C_1$–$C_4$-alkyl-sulphonylamino, or represents phenylsulphonylamino which is optionally substituted by fluorine, chlorine, bromine or methyl, or wherein, furthermore,

$R^1$ represents the radical

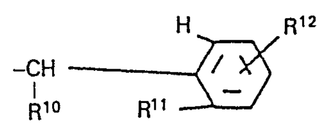

wherein

$R^{10}$ represents hydrogen or $C_1$–$C_4$-alkyl and

$R^{11}$ and $R^{12}$ are identical or different and represent hydrogen, fluorine, chlorine, bromine, nitro, cyano, $C_1$–$C_4$-alkyl [which is optionally substituted by fluorine and/or chlorine], $C_1$–$C_4$-alkoxy [which is optionally substituted by fluorine and/or chlorine], carboxyl, $C_1$–$C_4$-alkoxy-carbonyl, $C_1$–$C_4$-alkylsulphonyl or di-($C_1$–$C_4$-alkyl)-aminosulphonyl; or wherein, furthermore,

$R^1$ represents the radical

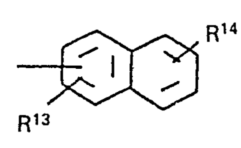

wherein

$R^{13}$ and $R^{14}$ are identical or different and represent hydrogen, fluorine, chlorine, bromine, nitro, cyano, $C_1$–$C_4$-alkyl [which is optionally substituted by fluorine and/or chlorine] or $C_1$–$C_4$-alkoxy [which is optionally substituted by fluorine and/or chlorine]; or wherein, furthermore,

$R^1$ represents the radical

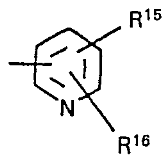

wherein

$R^{15}$ and $R^{16}$ are identical or different and represent hydrogen, fluorine, chlorine, bromine, nitro, cyano, $C_1$–$C_4$-alkyl [which is optionally substituted by fluorine and/or chlorine] or $C_1$–$C_4$-alkoxy [which is optionally substituted by fluorine and/or chlorine], or represent $C_1$–$C_4$-alkylthio, $C_1$–$C_4$-alkylsulphinyl or $C_1$–$C_4$-alkylsulphonyl [which are optionally substituted by fluorine and/or chlorine], or represent di-($C_1$–$C_4$-alkyl)-aminosulphonyl or $C_1$–$C_4$-alkoxy-carbonyl; or wherein, furthermore

$R^1$ represents the radical

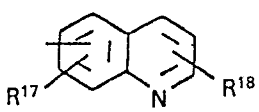

wherein

$R^{17}$ and $R^{18}$ are identical or different and represent hydrogen, fluorine, chlorine, bromine, $C_1$–$C_4$-alkyl [which is optionally substituted by fluorine and/or bromine], $C_1$–$C_4$-alkoxy [which is optionally substituted by fluorine and/or chlorine], or represent $C_1$–$C_4$-alkylthio, $C_1$–$C_4$-alkylsulphinyl or $C_1$–$C_4$-alkylsulphonyl [which are optionally substituted by fluorine and/or chlorine], or represent di($C_1$–$C_4$-alkyl)-aminosulphonyl; or wherein, furthermore,

$R^1$ represents the radical

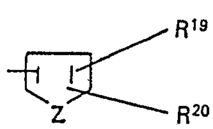

wherein

$R^{19}$ and $R^{20}$ are identical or different and represent hydrogen, fluorine, chlorine, bromine, cyano, nitro, $C_1$–$C_4$-alkyl [which is optionally substituted by fluorine and/or chlorine], $C_1$–$C_4$-alkoxy [which is optionally substituted by fluorine and/or chlorine], $C_1$–$C_4$-alkylthio, $C_1$–$C_4$-alkylsulphinyl or $C_1$–$C_4$-alkylsulphonyl [which is optionally substituted by fluorine and/or chlorine], di-($C_1$–$C_4$-alkyl)-amino-sulphonyl or $C_1$–$C_4$-alkoxy-carbonyl, and

Z represents oxygen, sulphur or the grouping N–$Z^1$, wherein

$Z^1$ represents hydrogen, $C_1$–$C_4$-alkyl [which is optionally substituted by fluorine, chlorine, bromine or cyano], $C_3$–$C_6$-cycloalkyl, benzyl, phenyl [which is optionally substituted by fluorine, chlorine, bromine or nitro], $C_1$–$C_4$-alkyl-carbonyl, $C_1$–$C_4$-alkoxy-carbonyl or di-($C_1$–$C_4$-alkyl)-amino-carbonyl; or wherein, furthermore,

$R^1$ represents the radical

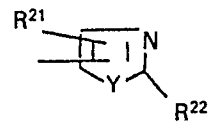

wherein

$R^{21}$ represents hydrogen, $C_1$–$C_4$-alkyl or halogen,

$R^{22}$ represents hydrogen or $C_1$–$C_5$-alkyl and

Y represents sulphur or the grouping N–$R^{23}$

wherein

$R^{23}$ represents hydrogen or $C_1$–$C_5$-alkyl; and wherein, furthermore,

$R^2$ represents the radical

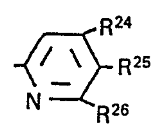

wherein

$R^{24}$ and $R^{26}$ are identical or different and represent hydrogen, fluorine, chlorine, bromine, $C_1$–$C_4$-alkyl [which is optionally substituted by fluorine and/or chlorine] or $C_1$–$C_4$-alkoxy [which is optionally substituted by fluorine and/or chlorine], with the proviso that at least one of the radicals $R^{24}$ and $R^{26}$ are other than hydrogen, and

$R^{25}$ represents hydrogen, fluorine, chlorine, bromine, cyano or $C_1$–$C_4$-alkyl [which is optionally substituted by fluorine and/or chlorine]; or wherein furthermore,

$R^2$ represents the radical

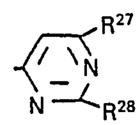

wherein

$R^{27}$ and $R^{28}$ identical or different and represent hydrogen, fluorine, chlorine, bromine, $C_1$–$C_4$-alkyl [which is optionally substituted by fluorine and/or chlorine], $C_1$–$C_4$-alkoxy [which is optionally substituted by fluorine and/or chlorine], $C_1$–$C_4$-alkylamino or di-($C_1$–$C_4$-alkyl)-amino, with the proviso that at least one of the radicals $R^{27}$ and $R^{28}$ is other than hydrogen; or wherein, furthermore,

$R^2$ represents the radical

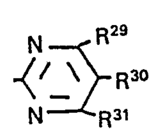

wherein

$R^{29}$ represents hydrogen, fluorine, chlorine, bromine, hydroxyl, $C_1$–$C_4$-alkyl [which is optio-

nally substituted by fluorine and/or chlorine] or $C_1$–$C_4$-alkoxy [which is optionally substituted by fluorine and/or chlorine],

$R^{30}$ represents hydrogen, fluorine, chlorine, bromine, $C_1$–$C_4$-alkyl [which is optionally substituted by fluorine and/or chlorine], cyano, formyl, $C_1$–$C_4$-alkyl-carbonyl or $C_1$–$C_4$-alkoxy-carbonyl and

$R^{31}$ represents hydrogen, fluorine, chlorine, bromine, hydroxyl, $C_1$–$C_4$-alkyl [which is optionally substituted by fluorine and/or chlorine], $C_1$–$C_4$-alkoxy [which is optionally substituted by fluorine and/or chlorine], amino, $C_1$–$C_4$-alkylamino or di-($C_1$–$C_4$-alkyl)-amino, or

$R^{30}$ and $R^{31}$ together represent $C_3$–$C_4$-alkanediyl; or wherein, furthermore,

$R^2$ represents the radical

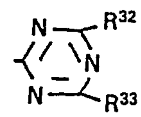

wherein

$R^{32}$ and $R^{33}$ are identical or different and represent fluorine, chlorine, bromine, hydroxyl, $C_1$–$C_4$-alkyl [which is optionally substituted by fluorine and/or chlorine], $C_3$–$C_5$-cycloalkyl, $C_1$–$C_4$-alkoxy [which is optionally substituted by fluorine and/or chlorine] or $C_1$–$C_4$-alkylthio, or represent $C_1$–$C_4$-alkyl-amino or di-($C_1$–$C_4$-alkyl)-amino; or wherein, furthermore,

$R^2$ represents the radical

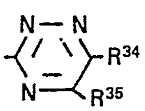

wherein

$R^{34}$ and $R^{35}$ are identical or different and represent hydrogen, methyl or methoxy; and wherein, furthermore,

$R^3$ represents a phenyl radical which is optionally substituted by one or more of the radicals from the series comprising halogen, cyano, nitro, hydroxyl, carboxyl, $C_1$–$C_6$-alkyl [which is optionally substituted by fluorine, chlorine, bromine, nitro, cyano, hydroxyl, carboxyl, $C_1$–$C_4$-alkoxy-carbonyl, $C_1$–$C_4$-alkoxy, $C_1$–$C_4$-alkylthio or phenyl], $C_3$–$C_6$-cycloalkyl, $C_1$–$C_4$-alkoxy [which is optionally substituted by fluorine, chlorine, bromine, cyano, carboxyl, $C_1$–$C_4$-alkoxy, $C_1$–$C_4$-alkylthio or $C_1$–$C_4$-alkoxycarbonyl] $C_1$–$C_4$-alkylthio [which is optionally substituted by fluorine, chlorine, bromine, cyano, carboxyl, $C_1$–$C_4$-alkoxy-carbonyl], amino, $C_1$–$C_4$-alkyl-amino or di-($C_1$–$C_4$-alkyl)-amino [which are optionally substituted by fluorine, chlorine, bromine, cyano, carboxyl, $C_1$–$C_4$-alkoxy or $C_1$–$C_4$-alkoxy-carbonyl], $C_1$–$C_4$-alkyl-carbonyl-amino, $C_1$–$C_4$-alkoxy-carbonyl-amino, (di)-$C_1$–$C_4$-alkylaminocarbonylamino, formyl, $C_1$–$C_4$-alkyl-carbonyl, benzoyl, $C_1$–$C_4$-alkoxy-carbonyl, phenoxy-carbonyl, benzyloxycarbonyl, phenyl [which is optionally substituted by fluorine, chlorine, bromine, cyano, nitro, hydroxyl or methyl], phenoxy, phenylthio, phenylsulphonyl, phenylamino or phenylazo [which are optionally substituted by fluorine, chlorine, bromine, cyano, nitro, methyl and/or trifluoromethyl], pyridoxy or pyrimidoxy [which are optionally substituted by fluorine, chlorine, bromine, cyano, nitro, methyl and/or trifluoromethyl], $C_1$–$C_4$-alkyl-carbonyloxy, $C_1$–$C_4$-alkoxy-carbonyloxy, $C_1$–$C_4$-alkyl-amino-carbonyloxy and di-($C_1$–$C_4$-alkyl)-amino-carbonyloxy, or which is optionally fused by a benzo radical [which is optionally substituted by fluorine, chlorine, bromine, cyano, nitro, methyl and/or trifluoromethyl]; or wherein, furthermore,

$R^3$ represents a five-membered or six-membered heteroaromatic ring which contains 1 to 3 nitrogen atoms and/or one oxygen or sulphur atom and which is optionally benzo-fused and/or substituted by fluorine, chlorine, bromine, cyano, nitro, $C_1$–$C_3$-alkyl or $C_1$–$C_3$-alkoxy [the latter optionally being substituted by fluorine and/or chlorine]; and wherein, furthermore,

X represents oxygen or sulphur and

M represents hydrogen or one equivalent of sodium, potassium, magnesium, calcium, aluminium, manganese, iron, cobalt or nickel, and also adducts of compounds of the formula (I) with hydrogen halide acids, with sulphuric acid, with alkanesulphonic acids which have 1 to 4 carbon atoms and which are optionally substituted by fluorine and/or chlorine, or with benzene- or naphthalenesulphonic acids which are optionally substituted by fluorine, chlorine, bromine or methyl.

2. Sulphonyliso(thio)urea derivatives of the general formula (I) according to Claim 1, characterized in that, in this formula, (A) $R^1$ represents the radical

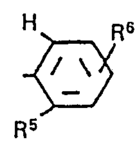

wherein

$R^5$ represents fluorine, chlorine, bromine, methyl, trifluoromethyl, methoxy, difluoromethoxy, trifluoromethoxy, $C_1$–$C_3$-alkylthio, difluoromethylthio, trifluoromethylthio, $C_1$–$C_3$-alkylsulphinyl, $C_1$–$C_3$-alkylsulphonyl, dimethylaminosulphonyl, diethylaminosulphonyl, N-methoxy-N-methylaminosulphonyl, phenoxy, $C_1$–$C_3$-alkoxy-carbonyl or $C_1$–$C_3$-alkylaminocarbonyl and

$R^6$ represents hydrogen; wherein, furthermore,

$R^2$ represents the radical

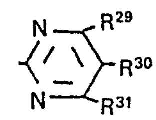

wherein

$R^{29}$ represents hydrogen, fluorine, chlorine,

bromine, hydroxyl, $C_1$–$C_3$-alkyl, $C_1$–$C_3$-alkoxy or difluoromethoxy,

$R^{30}$ represents hydrogen, chlorine, bromine or methyl and

$R^{31}$ represents $C_1$–$C_3$-alkyl, hydroxyl, fluorine, chlorine, bromine or $C_1$–$C_3$-alkoxy;

$R^3$ represents a phenyl radical which is optionally substituted by one or two radicals from the series comprising fluorine, chlorine, bromine, iodine, cyano, nitro, hydroxyl, carboxyl, $C_1$–$C_3$-alkoxycarbonyl, $C_1$–$C_4$-alkyl, trifluoromethyl, hydroxymethyl, methoxycarbonylmethyl, phenyl-$C_1$–$C_3$-alkyl, cyclohexyl, $C_1$–$C_3$-alkoxy, trifluoromethoxy, $C_1$–$C_3$-alkylthio, trifluoromethylthio, dimethylamino, amino, acetylamino, formyl, acetyl, benzoyl, phenyl, hydroxyphenyl, phenoxy [which is optionally substituted by chlorine and/or trifluoromethyl], phenylamino- phenylazo and pyridoxy [which is optionally substituted by chlorine and/or trifluoromethyl], or which is optionally benzo-fused;

X represents oxygen or sulphur and

M represents hydrogen or one equivalent of sodium, potassium or calcium; or wherein, furthermore,

(B) $R^1$, $R^3$, X and M have the meaning given above under (A) and

$R^2$ represents the radical

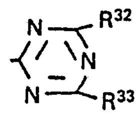

wherein

$R^{32}$ represents fluorine, chlorine, cyclopropyl, $C_1$–$C_2$-alkyl, $C_1$–$C_2$-alkoxy or $C_1$–$C_2$-alkylthio and

$R^{33}$ represents fluorine, chlorine, cyclopropyl, $C_1$–$C_2$-alkyl, $C_1$–$C_2$-alkoxy, $C_1$–$C_2$-alkylamino di-($C_1$–$C_2$-alkyl)-amino, and also adducts of compounds of the formula (I) with hydrogen halide acids, with sulphuric acid, with alkane-sulphonic acids which have 1 to 4 carbon atoms and which are optionally substituted by fluorine and/or chlorine, or with benzene- or naphthalenesulphonic acids which are optionally substituted by fluorine, chlorine, bromine or methyl.

3. Process for the preparation of sulphonyliso-(thio)urea derivatives of the general formula (I) according to Claim 1, characterized in that sulphonylguanidine derivatives of the formula (II)

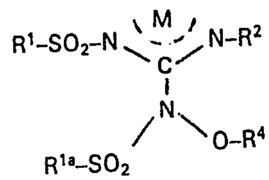

(II)

in which

$R^1$, $R^2$ and M have the meanings given in Claim 1,

$R^{1a}$ has the same meaning as $R^1$, but does not have to be identical to $R^1$ in each individual case and

$R^4$ represents $C_1$–$C_4$-alkyl or benzyl, are reacted with compounds of the formula (III)

$$M^1\text{–}X\text{–}R^3 \qquad \text{(III)}$$

in which

X and $R^3$ have the meanings given in Claim 1 and $M^1$ represents hydrogen, sodium or potassium, if appropriate in the presence of bases and if appropriate in the presence of diluents, and, if appropriate, the products of the formula (I) thereby obtained are treated with acids.

4. Herbicidal agents, characterized in that they contain at least one sulphonyliso(thio)urea derivative of the general formula (I) according to Claim 1.

5. Use of sulphonyliso(thio)urea derivatives of the general formula (I) according to Claim 1 for combating undesired plant growth.

6. Process for the preparation of herbicidal agents, characterized in that sulphonyliso(thio) urea derivatives of the general formula (I) according to Claim 1 are mixed with extenders and/or surface-active agents.